(19) Europäisches Patentamt
European Patent Office
Office européen des brevets

(11) EP 1 921 074 A1

(12) **EUROPEAN PATENT APPLICATION**
published in accordance with Art. 153(4) EPC

(43) Date of publication:
**14.05.2008 Bulletin 2008/20**

(21) Application number: **06797075.6**

(22) Date of filing: **30.08.2006**

(51) Int Cl.:
***C07D 277/20*** *(2006.01)* ***A61K 31/426*** *(2006.01)*
***A61K 31/4709*** *(2006.01)* ***A61K 31/517*** *(2006.01)*
***A61P 3/10*** *(2006.01)* ***A61P 43/00*** *(2006.01)*
***C07D 277/44*** *(2006.01)* ***C07D 277/56*** *(2006.01)*
***C07D 417/04*** *(2006.01)* ***C07D 417/12*** *(2006.01)*

(86) International application number:
**PCT/JP2006/317102**

(87) International publication number:
**WO 2007/026761 (08.03.2007 Gazette 2007/10)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **31.08.2005 JP 2005252464**
**28.06.2006 JP 2006177436**

(71) Applicant: **Astellas Pharma Inc.**
**Tokyo 103-8411 (JP)**

(72) Inventors:
- **HAYAKAWA, Masahiko**
**Chuo-ku Tokyo 103-8411 (JP)**
- **NIGAWARA, Takahiro**
**Chuo-ku Tokyo 103-8411 (JP)**
- **TSUCHIYA, Kazuyuki**
**Chuo-ku Tokyo 103-8411 (JP)**
- **ISHIBASHI, Naoki**
**Chuo-ku Tokyo 103-8411 (JP)**
- **OKUMURA, Mitsuaki**
**Chuo-ku Tokyo 103-8411 (JP)**
- **KAWAMOTO, Yuichiro**
**Chuo-ku Tokyo 103-8411 (JP)**
- **NAGAYOSHI, Akira**
**Chuo-ku Tokyo 103-8411 (JP)**

(74) Representative: **Gille Hrabal Struck Neidlein Prop Roos**
**Patentanwälte**
**Brucknerstrasse 20**
**40593 Düsseldorf (DE)**

# (54) THIAZOLE DERIVATIVE

(57) [Problem] To provide a compound which is useful as a GK activator.

[Means for Resolution] As a result of an extensive study on thiazole derivatives, the present inventors have found that a compound having an oxamoyl group, a glycol group or the like on a thiazole ring and a compound having an acetamide group substituted by a bicyclic heteroaryl group such as a quinolyl have a good GK activation effect, and thereby have accomplished the present invention.

Since the compounds of the present invention have a good GK activation effect, these are useful as therapeutic agents for diabetes, particularly type II diabetes.

EP 1 921 074 A1

**Description**

TECHNICAL FIELD

The present invention relates to a novel thiazole derivative which is useful as a pharmaceutical, particularly an agent for treating diabetes.

BACKGROUND OF THE INVENTION

GK (glucokinase (ATP:D-hexose 6-phosphotransferase, EC2.7.1.1)) is an enzyme which is expressed in the pancreas and the liver and phosphorylates hexose, and its presence in the brain has also been revealed in recent years. This enzyme belongs to the hexokinase family and is also called an alias hexokinase IV. In comparison with other hexokinases, GK has characteristics such as 1) it has low affinity for glucose as its substrate and shows a Km value close to the blood glucose concentration, 2) it is not inhibited by glucose 6-phosphate which is its enzyme reaction product, 3) it has about half molecular weight of 50 kDa, and the like.
The human glucokinase gene is positioned at the $7^{th}$ chromosome 7p13 as a single gene and controlled by 30 kb or more distant tissue-specific different promoters in pancreatic β cells and hepatic cells and uses a different first exon but the other exons 2 to 10 are common. Accordingly, in the pancreatic and hepatic GK proteins, only the N-terminal 15 residues are different.
Accompanied by the increase of blood glucose level, glucose concentration in the pancreatic β cells quickly reaches its equilibrium via a glucose transporting carrier GLUT 2, and GK detects a change in the intracellular glucose concentration and activates the glycolytic pathway. As a result of this, ATP/ADP ratio in the pancreatic β cells increases and the $K_{ATP}$ channel is closed, and a voltage-dependent Ca channel detects this and the intracellular calcium concentration is thereby increased and release of insulin occurs. That is, GK acts as a glucose sensor in the pancreatic β cells and carries an important role in the control of insulin secretion. GK also acts as a glucose sensor in the liver, responds to the increase of blood glucose level and converts glucose into glucose 6-phosphate. As a result of this, production of glycogen increases, and the glycolytic pathway is also activated and the gluconeogenesis in the liver is thereby inhibited. In patients whose glucose phosphorylation ability was reduced due to gene mutation of GK, hyperglycemia occurs frequently and juvenile diabetes is generated (MODY 2). On the other hand, in patients who show a low value of the Km value of GK activity due to a gene mutation, hypoglycemia is recognized after meal and at the time of fasting. That is, GK acts as a glucose sensor in human too and thereby playes an important role in maintaining normal blood glucose level. From these facts, it is expected that an agent capable of activating GK becomes an excellent therapeutic agent for type II diabetes, which corrects hyperglycemia after meal by accelerating glucose-dependent insulin secretion from the pancreatic β cells and, at the same time, inhibits release of glucose from the liver. Further, there also is a possibility that excess acceleration of insulin secretion does not occur due to acceleration of glucose uptake into the liver under hyperglycemic state after meal and therefore that the pancreatic secondary failure as a conventional problem with sulfonylurea (SU) agents can be avoided. In addition, it has been reported in recent years that apoptosis is induced when a mouse cultured pancreatic cell (MIN6N8) is cultured under high glucose. In addition, since apoptosis of the MIN6N8 was inhibited when glucokinase was over-expressed in this cell *(*Diabetes 54:2) 2602 - 2611 (2005), it is expected that a GK activating agent shows a pancreas protective action.
The GK which exists in the brain is a pancreas type and frequently expressed in the nerve of feeding center VMH (Ventromedial hypothalamus). Glucose-sensitive nerves are classified into a glucose excitatory GE(Glucose Excited)-neuron and a glucose suppressive GI(Glucose Inhibited)-neuron. The presence of mRNA and protein of GK is found in about 70% of the GE-neuron and about 40% of the GI-neuron.
In these glucose-sensitive nerves, GK detects increase of the intracellular glucose and activates the glycolytic pathway, and the intracellular ATP/ADP ratio thereby increases. As a result of this, the $K_{ATP}$ channel is closed in the GE-neuron, frequency of action potential of the neuron is increased and a neurotransmitter is released. On the other hand, it is considered that a Cl- channel is concerned in the GI-neuron. In a rat in which expression of GK mRNA is increased in the VMH, compensatory action for the glucose-deficient state is reduced.
Receptors for leptin and insulin concerning in the feeding behavior are also present in the glucose-sensitive nerves. In the GE-neuron under a high glucose condition, leptin and insulin open the $K_{ATP}$ channel and reduce the frequency of action potential. In addition, the NPY (Neuropeptide Y)-neuron which functions for the appetite promotion at ARC (arcuate nucleus) is suppressive for glucose and the POMC (Proopiomelanocortin)-neuron which functions for the appetite suppression is excitatory for glucose (Diabetes 53:2521 - 2528 (2004)). From these facts, it is expected that feeding behavior is suppressed by activating GK of the central, which is effective for the treatment of obesity and metabolic syndrome.
Though a large number of compounds having the GK activation action have been reported, there are no reports so far on compounds whose clinical efficacy was confirmed. In addition, a novel GK activator having a good profile regarding various side effects(actions for hERG and CYP) and its solubility is in great demand.

Several thiazole derivatives having GK activation action have been reported (e.g., Patent References 1 to 11), but there are no reports on a compound having an oxamoyl group or a glycol group on a thiazole ring and there are no reports too on a compound having an acetamide group substituted by a bicyclic heteroaryl group such as a quinolyl.

Patent Reference 1: International Publication WO 00/58293
Patent Reference 2: International Publication WO 01/83465
Patent Reference 3: International Publication WO 01/83465
Patent Reference 4: International Publication WO 01/85706
Patent Reference 5: International Publication WO 01/85707
Patent Reference 6: International Publication WO 02/08209
Patent Reference 7: International Publication WO 02/14312
Patent Reference 8: International Publication WO 03/95438
Patent Reference 9: International Publication WO 2004/72066
Patent Reference 10: International Publication WO 2004/50645
Patent Reference 11: International Publication WO 2006/58923

DISCLOSURE OF THE INVENTION

PROBLEMS THAT THE INVENTION IS TO SOLVE

An object of the present invention is to provide a pharmaceutical having GK activation action, particularly a novel compound which is useful as an agent for treating diabetes.

MEANS FOR SOLVING THE PROBLEMS

The present inventors have made extensive studies on thiazole derivatives and, as a result, confirmed that a compound having an oxamoyl group, a glycol group or the like on a thiazole ring and a compound having an acetamide group substituted by a bicyclic heteroaryl group such as a quinolyl have good GK activation action and finding that a compound in which various side effects(actions for hERG and CYP) and/or its solubility was improved is also present, resulting in accomplishment of the present invention.
That is, the present invention relates to a thiazole derivative represented by a general formula (I) or a salt thereof.

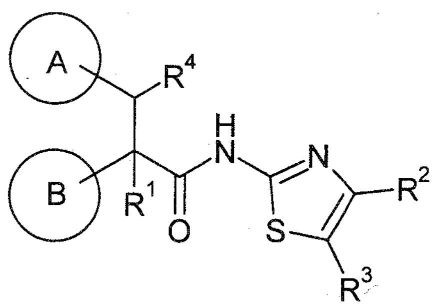

(I)

(Symbols in the formula have the following meanings;

A: cycloalkyl or cycloalkenyl which may respectively be substituted,
B: a group selected from phenyl, pyridyl, quinolyl, isoquinolyl, quinoxalinyl, quinazolinyl and cinnolinyl, which may be substituted with 1 or 2 substituent groups,
$R^1$:-H, halogen or $-R^0$,
$R^4$: -H, -OH or halogen,
or $R^1$ and $R^4$ together form a bond,
$R^2$ and $R^3$: the same or different from each other, and each is a group selected from the following (i) or (ii),

(i): $-CH(OR^A)-R^B$, $-CO-CO-NR^CR^D$, $-CO-CO-NR^C-OR^D$, -CO-lower alkylene-$OR^E$, $-C(OR^E)(OR^F)-R^B$, $-C(OR^E)(OR^F)-R^0$, $-C(R^G)(OR^E)-CH(OR^F)-R^C$, $-C(R^G)(OR^E)-C(R^0)(OR^F)-R^C$, $-CH(OR^E)-CH(OR^F)-R^B$, $-C(R^G)(OR^E)$-lower alkylene-$OR^F$, $-CH(CH_2OR^E)-CH_2OR^F$, $-C(R^G)(CH_2OR^E)-CH_2OR^F$, -lower alkylene-$C(R^G)$

$(OR^E)-CH(OR^F)-R^C$, -lower alkylene-$C(R^G)(OR^E)-C(R^0)(OR^F)-R^C$, -lower alkylene-$CH(CH_2OR^E)-CH_2OR^F$ and/or lower alkylene-$C(R^G)(CH_2OR^E)-CH_2OR^F$,

(ii):-H,-halogen, $-NO_2$, -CN, $-R^0$, $-CO-CO_2H$, $-CO-CO-OR^0$, -halogeno lower alkyl, -lower alkylene-$OR^A$ and/or -lower alkylene-$NR^CR^D$,

$R^A$: the same or different from each other and each represents -H, $-R^0$, -halogeno lower alkyl or -lower alkylene-aryl,

$R^B$: $-CO_2H$, $-CO_2R^0$, $-CO-NR^CR^D$, $-CO-NR^C-OR^D$, -lower alkylene-$NR^CR^D$, -lower alkylene-$OR^A$, -lower alkylene-$CO_2R^0$, -lower alkylene-CO-$NR^CR^D$ or -lower alkylene-CO-$NR^C$-$OR^D$,

$R^C$ and $R^D$: the same or different from each other and each represents -H, $-R^0$, -lower alkylene-$N(R^A)_2$, -lower alkylene-$OR^A$, -lower alkylene-$CO_2H$, -lower alkylene-$CO_2R^0$ or- lower alkylene-CO-$N(R^A)_2$,

$R^E$ and $R^F$: the same or different from each other and each represents a group described in $R^A$, -C(O)-$R^0$ or -C (O)-aryl, or $R^E$ and $R^F$ together form lower alkylene or -C(O)-,

$R^G$: H, $-R^0$ or cycloalkyl, and

$R^0$: the same or different from each other and each represents lower alkyl.

However, when 1) B is phenyl or pyridyl which may be substituted and also 2) $R^1$ is H or $R^1$ and $R^4$ together form a bond, at least one of $R^2$ and $R^3$ is a group selected from (i). The same shall apply hereinafter.)

Further, the present invention also relates to a pharmaceutical composition which comprises the aforementioned thiazole derivative or a pharmaceutically acceptable salt thereof and a pharmaceutically acceptable carrier, particularly a pharmaceutical composition which is a GK activator or a preventive or therapeutic agent for diabetes, obesity or metabolic syndrome.

That is, (1) a pharmaceutical composition which comprises the compound described in the formula (I) or a pharmaceutically acceptable salt thereof and a pharmaceutically acceptable carrier,

(2) the pharmaceutical composition described in(1), which is a GK activator,

(3) the pharmaceutical composition described in(1), which is an agent for preventing and/or treating diabetes,

(4) the pharmaceutical composition described in(3), which is an agent for preventing and/or treating type II diabetes,

(5) the pharmaceutical composition described in(1), which is an agent for preventing and/or treating obesity,

(6) the pharmaceutical composition described in(1), which is an agent for preventing and/or treating metabolic syndrome,

(7) use of the compound described in the formula (I) or a pharmaceutically acceptable salt thereof, for the manufacture of a GK activator or an agent for preventing and/or treating diabetes, obesity or metabolic syndrome, and

(8) a method for preventing and/or treating diabetes, obesity or metabolic syndrome, which comprises administering a therapeutically effective amount of the compound described in the formula (I) or a salt thereof to a patient.

In addition, this application also relates to a pharmaceutical, particularly a GK activator which uses the thiazole derivative represented by the formula (I) or a salt thereof as the active ingredient.

## ADVANTAGE OF THE INVENTION

Since the compound of the present invention has a GK activation action, it is useful as a therapeutic and preventive agent for diabetes, particularly type II diabetes. It is also useful as a therapeutic and preventive agent for complications of diabetes including nephropathy, retinopathy, neuropathy, disturbance of peripheral circulation, cerebrovascular accidents, ischemic heat disease and arteriosclerosis. In addition, it is also useful as a therapeutic and preventive agent for obesity and metabolic syndrome by suppressing overeating.

## BEST MODE FOR CARRYING OUT THE INVENTION

The following describes the present invention in detail.

In this description, the "alkyl" and "alkylene" mean straight or branched saturated hydrocarbon chains. The "lower alkyl" is an alkyl group having from 1 to 6 carbon atoms, preferably methyl, ethyl, n-propyl, 2-propyl, hexyl or the like. The "lower alkylene" means a divalent group as a result of eliminating one optional hydrogen atom from the aforementioned "lower alkyl" and is preferably an alkylene having from 1 to 4 carbon atoms, more preferably methylene, ethylene, methylmethylene or propylene.

The "halogen" is F, Cl, Br or I. The "halogeno lower alkyl" means an alkyl having from 1 to 6 carbon atoms which is substituted with one or more of halogen and is preferably a $C_{1-6}$ alkyl substituted with one or more of F, more preferably a $C_{1-6}$ alkyl substituted with 1 to 3 of F, more further preferably fluoromethyl, difluoromethyl, trifluoromethyl or trifluoroethyl.

The "cycloalkyl" is a cycloalkyl having from 3 to 10 carbon atoms, and it may form a bridged ring (e.g., adamantyl or the like). Preferred is a cycloalkyl having from 3 to 7 carbon atoms, more preferably cyclopropyl, cyclobutyl, cyclopentyl, cyclohexyl or cycloheptyl. The "cycloalkenyl" is a cyclic group having from 3 to 7 carbon atoms and having 1 or 2 of double bond, preferably cyclopentenyl, cyclohexenyl or cycloheptenyl. The "aryl" means an aromatic hydrocarbon group

having from 6 to 14 carbon atoms, and it includes a phenyl group ring-condensed with a "cycloalkenyl" such as indenyl, tetrahydronaphthyl and fluorenyl. Preferred are phenyl and naphthyl and more preferred is phenyl.
The "hydrocarbon ring" includes the aforementioned "cycloalkyl", "cycloalkenyl" and aryl

The "heterocyclic group" is a 3- to 7-membered monocyclic or bicyclic heterocyclic group which contains 1 to 4 hetero atoms selected from O, S and N, and it includes a saturated ring, an aromatic ring (heteroaryl) and a partially hydrogenated ring group thereof. In addition, it may form an oxide or dioxide in which the ring atom S or N is oxidized and may also form a bridged ring or spiro ring. For example, pyridyl, pyrazinyl, pyrimidinyl, pyridazinyl, imidazolyl, benzimidazolyl, benzofuranyl, benzothienyl, benzothiadiazolyl, benzothiazolyl, benzisothiazolyl, benzoxazolyl, benzisoxazolyl, pyrrolyl, pyrrolidinyl, thienyl, furyl, dioxanyl, dioxolanyl, triazinyl, triazolyl, thiazolyl, thiadiazolyl, oxadiazolyl, pyrazolyl, pyrazolidinyl, isothiazolyl, oxazolyl, isoxazolyl, quinolyl, isoquinolyl, tetrahydroquinolyl, tetrahydroisoquinolyl, quinazolinyl, quinoxalinyl, phthalazinyl, piperidyl, piperazinyl, azepanyl, diazepanyl, tetrahydrofuranyl, morpholinyl, methylenedioxyphenyl, ethylenedioxyphenyl, trithianyl, indolyl, isoindolyl, indolinyl, indazolyl, tetrahydrobenzimidazolyl, chromanyl, chromonyl(4-oxo-4H-1-benzopyranyl), and benzimidazolonyl(2,3-dihydro-2-oxobenzimidazolyl) can be cited. Preferred is a 5- or 6-membered monocyclic heteroaryl and further preferred is furyl, thienyl, imidazolyl, thiazolyl or pyridyl.

The "$R^1$ and $R^4$ together form a bond" means that the bond between carbon atoms to which $R^1$ and $R^4$ are respectively bonded is double bond as shown in the following formula (Ia). In this connection, the groups A and B in the following formula (Ia) are described by a configuration of Z against the double bond, but the compound of the present invention may be either E form or Z form. Preferred is Z form.

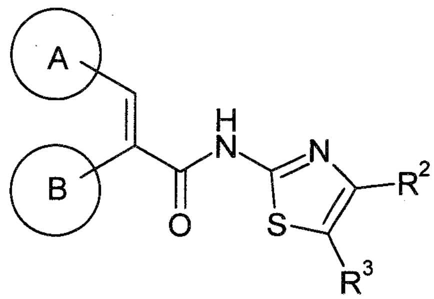

(Ia)

The "may be substituted" means "no substitution" or "has 1 to 5 same or different substituent groups". In this connection, when two or more substituent groups are possessed, for example like the case of $R^0$ of $-CON(R^0)_2$, these substituent groups may be the same or different from each other.
The substituent group in the "phenyl, pyridyl, quinolyl, isoquinolyl, quinoxalinyl, quinazolinyl and cinnolinyl, which may be substituted" is preferably $-R^0$, halogeno lower alkyl, halogen, -OH, -lower alkylene-OH, $-N_3$, -OR°, -O-halogeno lower alkyl, -lower alkylene-$OR^0$, -O-hydrocarbon ring, -O-hetero ring, -CN, $-NO_2$, -CHO, $-CO_2H$, $-CO_2R^0$, -lower alkylene-$CO_2H$, -lower alkylene-$CO_2R^0$, -CO-$R^0$, -CO-halogeno lower alkyl, -CO-hydrocarbon ring, -CO-hetero ring, $-CONH_2$, -CONH-$R^0$, $-CON(R^0)_2$, -CONH-hydrocarbon ring, -CONH-hetero ring, -NHCO-$R^0$, $-N(R^0)$-CO-$R^0$, -NH-$CO_2R^0$, -N $(R^0)$-$CO_2R^0$, -NHCO-hydrocarbon ring, -NHCO-hetero ring, -SH, $-SR^0$, -S-halogeno lower alkyl, -S-hydrocarbon ring, -S-lower alkylene-hydrocarbon ring, -S-hetero ring, -SO-$R^0$, -SO-halogeno lower alkyl, -SO-hydrocarbon ring, -SO-lower alkylene-hydrocarbon ring, -SO-hetero ring, $-SO_2R^0$, $-SO_2$-halogeno lower alkyl, $-SO_2$-hydrocarbon ring, $-SO_2$-lower alkylene-hydrocarbon ring, $-SO_2$-hetero ring, $-SO_3H$ $-SO_2NH$ $-SO_2NH$-$R^0$, $-SO_2N(R^0)_2$, $-SO_2NH$-hydrocarbon ring, $-SO_2NH$-hetero ring, -O-$SO_2$-$R^0$, -O-$SO_2$-halogeno lower alkyl, $-NHSO_2$-R°, $-N(R^0)$-$SO_2$-$R^0$, $-NHSO_2$-hydrocarbon ring or $-NHSO_2$-hetero ring, wherein the aforementioned "hydrocarbon ring" and "hetero ring" may be substituted with 1 to 5 groups selected from R°, halogeno lower alkyl, halogen, -OH and $-OR^0$.
The substituent group in the "cycloalkyl or cycloalkenyl which may respectively be substituted" is preferably $-R^0$, halogeno lower alkyl, halogen or $-OR^0$, more preferably halogen.

A preferred embodiment of the present invention is described in the following.

(1) As A, preferred is a $C_{3-8}$ cycloalkyl, more preferred is a $C_{3-7}$ cycloalkyl, further preferred is a $C_{5-6}$ cycloalkyl, further more preferred is cyclopentyl.
(2) As B, preferred is phenyl, pyridyl or quinolyl which may be substituted with 1 or 2 substituent groups, more preferred is phenyl or pyridyl which is substituted with 1 or 2 substituent groups, further preferred is phenyl which is substituted with 1 or 2 substituent groups, and further more preferred is phenyl which is substituted with one substituent group selected from the following groups preferred as the substituent group on B and which may be further substituted with one substituent group selected from the class consisting of lower alkyl and halogen. In this case, as the substituent group on B, preferred is $-R^0$, halogeno lower alkyl, halogen, $-OR^0$, -CN, $-NO_2$, -CHO, $-CO_2H$,

-$CO_2R^0$, -CO-$R^0$, -CO-hydrocarbon ring, -CO-hetero ring, -$SO_2R^0$, -$SO_2$-halogeno lower alkyl, -$SO_2$-hydrocarbon ring or -$SO_2$-hetero ring, more preferred is -$R^0$, halogeno lower alkyl, halogen, -$NO_2$, -CO-$R^0$, -CO-hydrocarbon ring, -CO-hetero ring, -$SO_2R^0$, -$SO_2$-halogeno lower alkyl, or -$SO_2$-cycloalkyl, further preferred is -$SO_2R^0$, -$SO_2$-halogeno lower alkyl, or -$SO_2$-cycloalkyl, further more preferred is -$SO_2$-methyl, -$SO_2$-ethyl, -$SO_2$-trifluoromethyl, -$SO_2$-cyclopropyl or -$SO_2$-cyclobutyl, and particularly preferred is -$SO_2$-methyl, -$SO_2$-trifluoromethyl, -$SO_2$-cyclopropyl or -$SO_2$-cyclobutyl.

(3) As $R^1$ and $R^4$, preferred is both H or a bond formed from $R^1$ and $R^4$ as one body, more preferred is a bond formed from $R^1$ and $R^4$ as one body.

(4) As $R^2$ and $R^3$, preferably one is H, -$R^0$ or halogen and the other is a group selected from (i), more preferably one is H and the other is a group selected from (i), further preferably $R^3$ is H and $R^2$ is a group selected from (i). As the group selected from (i), preferred is -CH($OR^A$)-$R^B$, -C($OR^E$)($OR^F$)-$R^B$, -C($OR^E$)($OR^F$)-$R^0$, -C($R^G$)($OR^E$)-CH($OR^F$)-$R^C$ or -lower alkylene-C($R^G$)($CH_2OR^E$)-$CH_2OR^F$, more preferred is -CH(OH)-$CH_2OH$, -C($R^0$)(OH)-$CH_2OH$, -CH($OR^0$)-$CH_2OH$, -CH($OR^0$)-$CH_2OR^0$, -CH(OH)-$CO_2H$, -CH($OR^0$)-$CO_2H$, -CH(OH)-$CO_2R^0$, -CH($OR^0$)-$CO_2R^0$, -$CH_2$-CH($CH_2OH$)-$CH_2OH$ or -$CH_2$-C($R^0$)($CH_2OH$)-$CH_2OH$, further preferred is -CH(OH)-$CH_2OH$, -C($R^0$)(OH)-$CH_2OH$, -CH($OR^0$)-$CH_2OH$, -CH($OR^0$)-$CH_2OR^0$, -CH(OH)-$CO_2R^0$, -CH($OR^0$)-$CO_2R^0$ or -$CH_2$-CH($CH_2OH$)-$CH_2OH$, further more preferred is -CH(OH)-$CH_2OH$, -C($R^0$)(OH)-$CH_2OH$, -CH($OR^0$)-$CH_2OH$, -CH($OR^0$)-$CH_2OR^0$ or -$CH_2$-CH($CH_2OH$)-$CH_2OH$ and particularly preferred is -CH(OH)-$CH_2OH$ or -C($CH_3$)(OH)-$CH_2OH$. As another preferred embodiment of the group selected from (i), preferred is -CO-CO-$NR^CR^D$, and more preferred is -CO-CO-$NH_2$, -CO-CO-NH-$R^0$, -CO-CO-N($R^0$)$_2$, -CO-CO-NH-lower alkylene-O-$R^0$ or -CO-CO-NH-lower alkylene-OH. As still another preferred embodiment of the group selected from (i), preferred is -CO-lower alkylene-$OR^{*E}$, more preferred is -CO-lower alkylene-OH and further preferred is -CO-$CH_2OH$.

As a further preferred embodiment, a compound consisting of a combination of respective preferred groups described in the aforementioned (1) to (4) is desirable.

In addition, still further preferred embodiment of the compound of the present invention represented by the general formula (I) is shown in the following.

(1) A compound described in (I), wherein $R^1$ and $R^4$ are both H, or $R^1$ and $R^4$ together form a bond.

(2) The compound described in (1), wherein A is a $C_{5-6}$ cycloalkyl.

(3) The compound described in (2), wherein B is phenyl substituted with 1 or 2 substituent groups selected from the group consisting of -$R^0$, halogeno lower alkyl, halogen, -OR°, -CN, -$NO_2$, -CHO, -$CO_2H$, -$CO_2R^0$, -CO-$R^0$, -CO-hydrocarbon ring, -CO-hetero ring, -$SO_2R^0$, -$SO_2$-halogeno lower alkyl, -$SO_2$-hydrocarbon ring and -$SO_2$-hetero ring.

(4) The compound described in (3), wherein one of $R^2$ and $R^3$ is H, lower alkyl or halogen and the other is -CH($OR^A$)-$R^B$, -C($OR^E$)($OR^F$)-$R^B$, -C($OR^E$)($OR^F$)-$R^0$, -C($R^G$)($OR^E$)-CH($OR^F$)-$R^C$, -lower alkylene-C($R^G$)($CH_2OR^E$)-$CH_2O^F$ or -CO-lower alkylene-$OR^E$.

(5) The compound described in (4), wherein B is phenyl which is substituted with one substituent group selected from the class consisting of -$SO_2R^0$, -$SO_2$-halogeno lower alkyl and -$SO_2$-cycloalkyl and which may be further substituted with one substituent group selected from the class consisting of -$R^0$ and halogen.

(6) The compound described in (5), wherein one of $R^2$ and $R^3$ is H and the other is -CH(OH)-$CH_2OH$, -C($R^0$)(OH)-$CH_2OH$, -CH($OR^0$)-$CH_2OH$, -CH($OR^0$)-$CH_2OR^0$, -$CH_2$-CH($CH_2OH$)-$CH_2OH$ or -CO-$CH_2OH$.

(7) The compound or a pharmaceutically acceptable salt thereof described in claim 1, which is selected from the group consisting of
(2E)-3-cyclopentyl-N-[4-(1,2-dihydroxyethyl)-1,3-thiazol-2-yl]-2-[4-(methylsulfonyl)phenylacrylamide,
(2E)-3-cyclopentyl-2-[4-(cyclopropylsulfonyl)phenyl]-N-[4-(1,2-dihydroxyethyl)-1,3-thiazol-2-yl]acrylamide,
(2R)-3-cyclopentyl-2-[4-(cyclopropylsulfonyl)phenyl]-N-[4-(1,2-dihydroxyethyl)-1,3-thiazol-2-yl]propanamide,
(2E)-3-cyclopentyl-2-[4-(cyclopropylsulfonyl)phenyl]-N-[4-(1,2-dihydroxy-1-methylethyl)-1,3-thiazol-2-yl]acrylamide,
(2E)-2-[4-(cyclobutylsulfonyl)phenyl]-3-cyclopentyl-N-[4-(1,2-dihydroxyethyl)-1,3-thiazol-2-yl]acrylamide,
(2E)-3-cyclopentyl-2-[4-(cyclopropylsulfonyl)phenyl]-N-{4-[3-hydroxy-2-(hydroxymethyl)propyl]-1,3-thiazol-2-yl}acrylamide,
(2E)-3-cyclopentyl-2-[4-(cyclopropylsulfonyl)phenyl]-N-[4-(1,2-dihydroxyethyl)-5-methyl-1,3-thiazol-2-yl]acrylamide,
(2E)-2-[4-(cyclobutylsulfonyl)phenyl]-3-cydopentyl-N-[4-(1,2-dihydroxy-1-methylethyl)-1,3-thiazol-2-yl]acrylamide,
(2R)-3-cyclopentyl-2-[4-(cyclopropylsulfonyl)phenyl]-N-[4-(1,2-dihydroxy-1-methylethyl)-1,3-thiazol-2-yl]propanamide,
(2E)-3-cyclopentyl-2-[4-(cyclopropylsulfonyl)phenyl]-N-{4-[(1S)-1,2-dihydroxyethyl]-1,3-thiazol-2-yl}acrylamide, and
(2E)-3-cyclopentyl-2-[4-(cyclopropylsulfonyl)phenyl]-N-(4-glycoloyl-1,3-thiazol-2-yl)acrylamide.

There are cases in which the compound of the present invention exists also in the form of other tautomers and geometrical isomers depending on the kind of substituent groups. Though sometimes described in this description only as an embodiment of these isomers, these isomers are also included in the present invention and isolated isomers or mixtures thereof are also included therein.

Also, the compound (I) sometimes has an asymmetric carbon atom or axial asymmetry, and optical isomers based on this (e.g., (R)-form, (S)-form and the like) can be present. The present invention includes all of the mixtures and isolated forms of these optical isomers.

Further, pharmacologically acceptable prodrugs of the compound (I) are also included in the present invention. The pharmacologically acceptable prodrug is a compound which has a group that can be converted into amino group, OH, $CO_2H$ or the like of the present invention by solvolysis or under a physiological condition. As the groups which form prodrugs, for example, the groups described in Prog. Med., 5,2157 - 2161 (1985) and "Iyakuhin no Kaihatsu (Development of Medicines)" (Hirokawa Shoten, 1990) Vol. 7 Bunshi Sekkei (Molecular Design) 163 - 198 can be cited.

In addition, there are cases in which the compound of the present invention forms acid addition salts or salts with bases depending on the kind of substituent groups, and such salts are included in the present invention with the proviso that they are pharmaceutically acceptable salts. Illustratively, acid addition salts with inorganic acids (e.g., hydrochloric acid, hydrobromic acid, hydriodic acid, sulfuric acid, nitric acid, phosphoric acid and the like) or organic acids (e.g., formic acid, acetic acid, propionic acid, oxalic acid, malonic acid, succinic acid, fumaric acid, maleic acid, lactic acid, malic acid, tartaric acid, citric acid, methanesulfonic acid, ethanesulfonic acid, p-toluenesulfonic acid, aspartic acid, glutamic acid and the like), salts with inorganic bases (e.g., sodium, potassium, magnesium, calcium, aluminum and the like) or with organic bases (e.g., methylamine, ethylamine, ethanolamine, lysine, omithine and the like), ammonium salts and the like may be exemplified.

The present invention also includes various hydrates and solvates of the compounds of the present invention and pharmaceutically acceptable salts thereof, and substances having polymorphism thereof.

(Production Methods)

The compounds of the present invention and pharmaceutically acceptable salts thereof can be produced by various conventionally known synthetic methods making use of their basal backbones or the characteristics based on the kinds of substituent groups. In that case, depending on the kinds of functional group, there is a case in which replacement of said functional group by an appropriate protecting group (a group which can be easily converted into said functional group), at a stage of the starting materials to intermediates, is effective in view of the production techniques. As such a functional group, it includes amino group, hydroxyl group, carboxyl group and the like, as their protecting groups, the protecting groups described for example in "Protective Groups in Organic Synthesis, edited by Greene and Wuts, (3rd edition, 1999)" can be cited, and these may be optionally selected and used in response to the reaction conditions. By such a method, a desired compound can be obtained by carrying out the reaction by introducing said protecting group and then removing the protecting group as occasion demands.

In addition, a prodrug of the compound (I) can be produced by introducing a specific group at a stage of the starting materials to intermediates similar to the case of the aforementioned protecting group or by carrying out the reaction using the obtained compound (I). The reaction can be carried out by employing the general methods which are conventionally known by those skilled in the art, such as esterification, amidation, dehydration and the like.

The following describes typical production methods of the compounds of the present invention. In this connection, the production methods of the present invention are not limited to the Examples shown below.

(Production method 1)

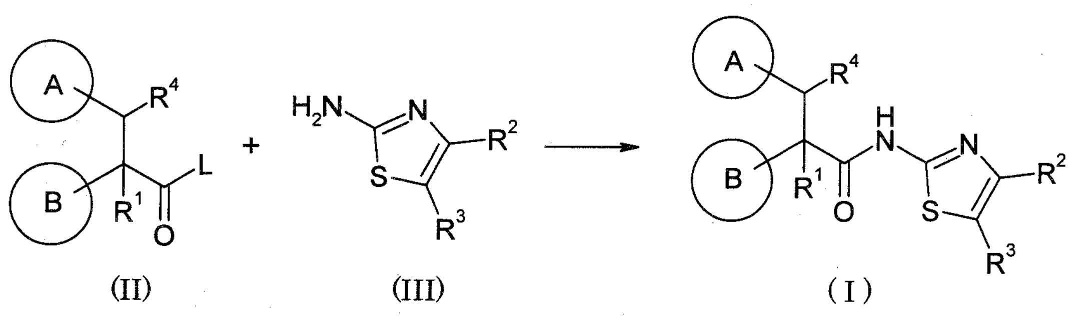

(II) (III) (I)

(In the formulae, L represents a leaving group or OH. The same shall apply hereinafter.)
This production method is a method in which the compound of the present invention represented by the formula (I) is obtained by subjecting a 2-aminothiazole compound (III) and a compound (II) to amidation reaction. As the leaving group of L, an organic sulfonate group (e.g., methanesulfonyloxy, p-toluenesulfonyloxy or the like), halogen and the like may be exemplified. Alternatively, various acid anhydrides can be used as the (II).
When L is hydroxyl group, the reaction can be carried out in the presence of a condensing agent such as N,N'-dicyclohexylcarbodiimide (DCC), 1-ethyl-3-(3'-dimethylaminopropyl)carbodiimide (WSC), 1,1'-carbonyldiimidazole (CDI), diphenylphosphorylazide (DPPA), phosphorus oxychloride/pyridine, triphenylphosphine/N-bromosuccinimide and the like, and in some cases, it can be carried out further in the presence of an additive agent (e.g., N-hydroxysuccinimide (HONSu), 1-hydroxybenzotriazole (HOBt) or the like). When L is a leaving group, it is desirable in some cases to carry out the reaction in the presence of an inorganic base (e.g., sodium carbonate, potassium carbonate, sodium bicarbonate, potassium bicarbonate or the like) or an organic base (e.g., triethylamine, diisopropylethylamine, pyridine or the like).
Regarding the solvent, reaction inert solvents such as aromatic hydrocarbons (e.g., benzene, toluene, xylene and the like), ethers (e.g., diethyl ether, tetrahydrofuran (THF), dioxane, diglyme, 1,2-dimethoxyethane, 2-methoxy diethyl ether and the like), halogenated hydrocarbons (e.g., dichloromethane, 1,2-dichloroethane, chloroform and the like), acetonitrile, ethyl acetate and the like can be used alone or as a mixture of two or more. In addition, the compound (II) and compound (III) are optionally used in equivalent molar to excess amounts in response to the reaction and compounds.

(Production method 2)

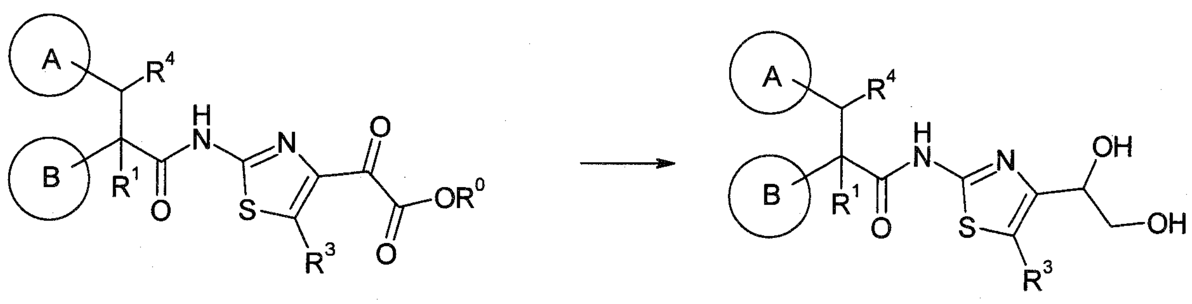

(IV) (I b)

This production method is a method in which a compound of the present invention represented by a formula (Ib) is obtained by subjecting a compound (IV) to a reduction reaction.
The reduction reaction can be carried out under cooling, under room temperature or under heating in a solvent such as the aforementioned ethers, alcohols (e.g., methanol, ethanol and the like), and the like, or a mixed solvent thereof, in the presence of a reducing agent (e.g., sodium borohydride or the like). The reducing agent can be used in an equivalent amount or an excess amount based on the compound (IV).

The groups $R^2$ and $R^3$ or various substituent groups on B in the formula (I) can be converted easily into other functional groups using the compound (I) of the present invention as the starting material and employing the methods which are obvious for those skilled in the art or modified methods thereof. For example, it can be carried out by optionally combining alkylation, acylation, oxidation, reduction, hydrolysis, amidation and the like steps which can be generally employed by those skilled in the art.

(Production of starting material compounds)

The starting material compounds in the aforementioned production methods can be produced for example by using the following methods, conventionally known methods or modified methods thereof.

(Starting material synthesis 1)

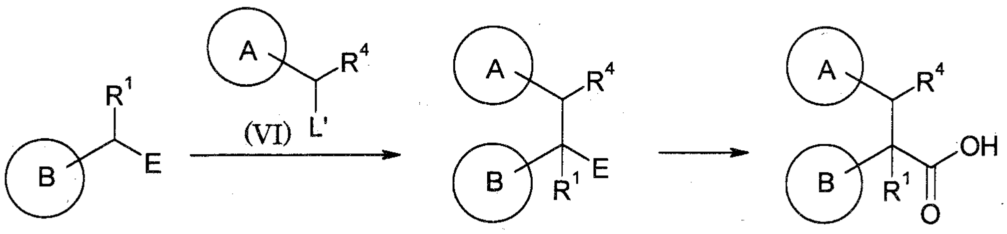

(V) (VII) (IIa)

(In the formulae, E means and carboxylic acid equivalent (e.g., an ester, nitrile or the like), and L' a leaving group (e.g., halogen or the like). The same shall apply hereinafter.)

The starting material compound (IIa) can be produced carrying out hydrolysis of a compound (VII) as its corresponding ester compound or nitrile compound under acidic or basic condition. As the acid, hydrochloric acid, hydrobromic acid or the like can be used, and lithium hydroxide, sodium hydroxide, potassium hydroxide or the like as the base, respectively.
The compound (VII) can be produced by subjecting the compound (V) to an alkylation reaction by the compound (VI). The reaction can be carried out by a general alkylation reaction and can be carried out under cooling to under heating in a reaction inert solvent such as ethers, 1,3-dirnethyltetrahydropyrimidine (DMPU) or the like in the presence of a base such as lithium diisopropylamide (LDA), sodium hydride, potassium hexamethyldisilazide, t-butoxy potassium, butyl lithium or the like.
In addition, when there is an asymmetric carbon in the starting material compound (IIa), an optically active starting material compound (IIa) can be obtained, for example, by isolating a racemic compound (IIa) as a diastereomer through its amidation with an asymmetry auxiliary group such as (4R)-4-benzyl-1,3-oxazolidin-2-one or the like and then hydrolyzing it.

(Starting material synthesis 2)

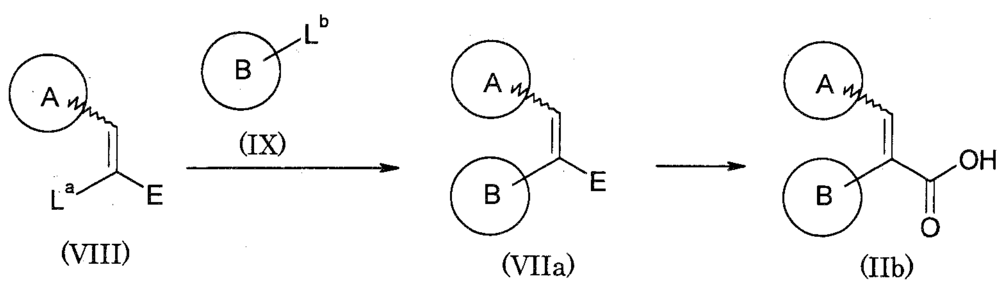

(VIII) (VIIa) (IIb)

(In the formulae, one of $L^a$ and $L^b$ represents halogen or trifluoromethylsulfonyloxy group, and the other $-B(OR^Z)_2$ or $-SnR^0_3$, $R^z$ represents H or lower alkyl, or two $R^z$ together form lower alkylene. The same shall apply hereinafter.)
The starting material compound (IIb) in which $R^1$ and $R^4$ together form a bond can be produced by hydrolyzing a compound (VIIa) as its corresponding ester compound or nitrile compound, in the same manner as the case of the hydrolysis of starting material synthesis 1.
The compound (VIIa) can be produced by a coupling reaction of compound (VIII) and compound (IX).
The coupling reaction can be carried out under cooling, under room temperature or under heating using the compound (VIII) and compound (IX) in an equivalent amount, or one of them in an excess amount, in a solvent such as ethers, alcohols, halogenated hydrocarbons, aromatic hydrocarbons, water or the like, or in a mixed solvent thereof, using a palladium complex (e.g., tetrakistriphenylphosphine palladium, palladium acetate, 1,1'-bis(diphenylphosphino)ferrocene-palladium(II) dichloride or the like) as the catalyst. In addition, it is advantageous in some cases in smoothly advancing the reaction to carry out the reaction in the presence of a base (e.g., sodium carbonate, cesium carbonate, sodium tert-butoxide or the like) or a lithium salt (e.g., lithium chloride, lithium bromide or the like).

(Starting material synthesis 3)

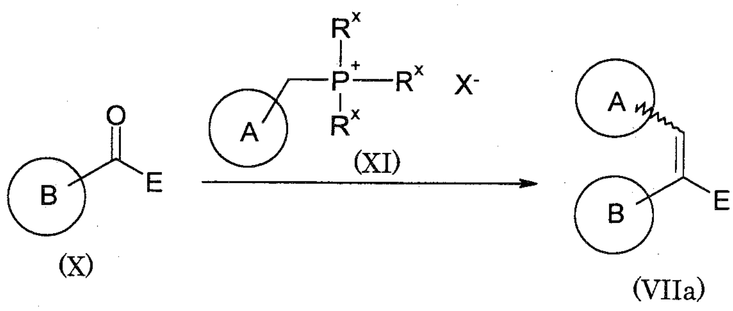

(In the formulae, $R^X$ represents a residual part of Wittig reagent, and X- represents a counter anion (e.g., halogen anion or the like). The same shall apply hereinafter.)
The compound (VIIa) can be produced by a Wittig reaction of compound (X) and compound (XI).
The Wittig reaction can be carried out under cooling to under heating in a solvent such as the aforementioned aromatic hydrocarbons, ethers, halogenated hydrocarbons, N,N-dimethylformamide (DMF), N,N-dimethylacetamide (DMA), N-methylpyrrolidone (NMP), dimethyl sulfoxide (DMSO), acetonitrile or the like, using potassium carbonate, tert-butoxy potassium, sodium hydride, n-butyl lithium, lithium hexadisilazide or the like as a base.

(Starting material synthesis 4)

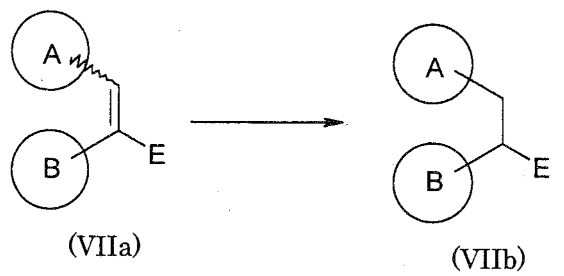

The compound (VIIb) can be produced by reducing the double bond of compound (VIIa).
The reduction reaction can be carried out at room temperature or under heating in a reaction inert solvent such as the aforementioned aromatic hydrocarbons, ethers, halogenated hydrocarbons, esters (e.g., ethyl acetate and the like), DMF, DMA, NMP, acetic acid or the like, in an atmosphere of hydrogen under ordinary pressure or pressurization, using palladium-carbon, palladium hydroxide-carbon, Raney nickel, platinum or the like as the catalyst. Depending on the compound, it is advantageous in some cases in smoothly advancing the reaction to carry out the reaction in the presence of an acid (preferably hydrochloric acid, acetic acid or the like).

(Starting material synthesis 5)

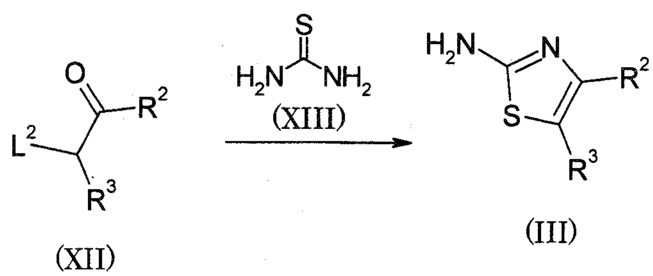

(XII)

(XIII)

(III)

(In the formulae, $L^2$ represents a leaving group (halogen or the like). The same shall apply hereinafter.)
The compound (III) can be produced by cyclization of compound (XII) and thiourea (XIII).
The cyclization reaction can be carried out at room temperature or under heating in a reaction inert solvent such as the aforementioned aromatic hydrocarbons, ethers, DMF, DMA, NMP, pyridine, alcohols, water or the like. Depending on the compound, it is advantageous in some cases in smoothly advancing the reaction to carry out the reaction in the presence of a base (preferably potassium carbonate, sodium bicarbonate, sodium methoxide or the like).

The compounds of the present invention are isolated and purified as free compounds or their pharmaceutically acceptable salts, hydrates, solvates or polymorphic substances. A pharmaceutically acceptable salt of the compound (I) of the present invention can also be produced by subjecting to a general salt formation reaction.
The isolation and purification are carried out by employing general chemical operations such as extraction, fractional crystallization, various types of fractional chromatography and the like.
Various isomers can be separated by selecting an appropriate starting material compound or making use of a difference in a physicochemical property between isomers. For example, an optically active isomer can be introduced into a stereochemically pure isomer by a general optical resolution method (e.g., a fractional crystallization for introducing into a diastereomer salt with optically active base or acid, a chiral column-aided chromatography or the like). In addition, it is also able to produce from an appropriate optically active starting material compound.

Pharmacological activities of the compounds of the present invention were confirmed by the following tests.

Test Example 1: Measurement of GK activation

Measurement of the GK activation by test agents was carried out in accordance with the method described in Science 301: 370 - 373, 2003, and by partially modifying this. The GK activity was measured as a change in absorbance based on the amount of NADPH which is converted from NADP (nicotinamide adenine dinucleotide phosphate) when glucose 6-phosphate produced by GK using glucose as the substrate is dehydrogenated to glucose-6-phosphate dehydrogenase.
Regarding the recombinant human liver GK (GST-hGK2) to be used in this assay, it was used by expressing as GST (glutathione S transferase)-fusion protein in *E. coli* and purifying by a Glutathione Sepharose column.
Regarding the sequence of glucokinase isoform 2, cloning of ORF (open reading frame) was carried out by the following procedure based on AK122876.1 (accession number). Using pME18S-FL3-Glucokinase isoform 2 as the template, PCR (polymerase chain reaction) was carried out using 5'-TAGAATTCATGGCGATGGATGTCACAAG-3'(SEQ ID NO:1) as the 5' primer and 5'-ATCTCGAGTCACTGGCCCAGCATACAG-3' (SEQ ID NO:2) as the 3' primer, and the PCR product was TA-cloned into pGEM-T easy vector. The sequence of this clone was confirmed by carrying out its sequencing. Thereafter, a fragment digested with *Eco*RI and *Xho*I was ligated to a vector pGEX-5X-1 digested in the same manner to prepare pGEX-human Glucokinase 2.
Regarding the enzyme reaction, the measurement was carried out at 27˚C using a 96 well flat bottom plate. As the enzyme mixed liquid, 25 mM HEPES pH 7.4; 25 mM KCl; 2 mM $MgCl_2$; 1 mM ATP; 0.1 % BSA; 1 mM DTT; 0.8 mM NADP; 2.5 U/ml glucose-6-phosphate dehydrogenase; GST-hGK2 (all in final concentration, however, the amount of GST-hGK2 was adjusted in such a manner that increase of absorbance of the DMSO control in 10 minutes (ΔOD) becomes about 0.12) was prepared. The enzyme mixed liquid was dispensed in 89 μl portions into the aforementioned plate, and a test agent dissolved in DMSO or the DMSO control was added thereto in 1 μl portions.
Subsequently, glucose (5 mM in final concentration) was added as the substrate solution in 10 μl portions and the reaction was started at 27˚C.
After commencement of the reaction, the absorbance was measured at a wavelength of 340 nm for 15 minutes at intervals of about 30 seconds, and the GK activation of each compound was calculated from the increase of absorbance during the first 10 minutes (ΔOD). Index of the GK activation of each test agent was calculated from the following formula as the GK activation (%).

$$\text{GK activation (\%)} = [(\Delta OD_{Test}) - (\Delta OD_{Cont})]/(\Delta OD_{Cont}) \times 100$$

$\Delta OD_{Test}$: $\Delta$OD at 10 $\mu$M test agent
$\Delta OD_{Cont}$: $\Delta$OD of DMSO control
Results of the measurement described in the above are shown in Table 1. In this connection, Ex indicates Example number.

[Table 1]

| Ex | GK activation (%) |
|---|---|
| 5 | 263 |
| 6 | 299 |
| 7 | 214 |
| 10 | 294 |
| 13 | 293 |
| 24 | 229 |
| 38 | 239 |
| 43 | 326 |
| 44 | 283 |
| 45 | 249 |
| 49 | 273 |
| 62 | 227 |
| 63 | 241 |
| 66 | 267 |
| 70 | 244 |
| 71 | 240 |
| 73 | 235 |
| 74 | 260 |
| 77 | 217 |

Test Example 2: Hypoglycemic action in C57BL6 mice

Body weights of freely ingesting C57BL6 mice (N = 5) were measured. Each test compound was dissolved in Cremophor (registered trademark) solvent (Cremophor:DMSO:Water 5:5:90, v/v/v) to a concentration of 1 mg/ml. To each mouse was orally administered 10 ml/kg of the agent liquid (corresponds to the test compound of 10 mg/kg) or 10 ml/kg of the solvent control. Just before the administration of the test compound, about 60 $\mu$l of blood was collected from the venous plexus of the fundus of the eye using a capillary. Blood was collected in the same manner 1 or 4 hours after the administration of the test compound. Blood plasma was separated from the thus collected blood to measure the blood glucose level. The blood glucose level after 1 or 4 hours of the administration of the test compound was compared with the blood glucose level of the solvent control group at the same period of time.
As a result, the blood glucose level after 1 hour of the administration of 10 mg/kg of the compound Ex 6 of the present invention was lowered by a factor of 22% in comparison with the blood glucose level of the solvent control group.

Test Example 3: Action upon high blood glucose level after oral glucose loading in ICR mice

After overnight fasting, body weights of ICR mice (N = 5) were measured. Each test compound was dissolved

in Cremophor solvent (Cremophor:DMSO:Water 5:5:90, v/v/v) to a concentration of 0.3 mg/ml. To each mouse was orally administered 10 ml/kg of the agent liquid (corresponds to the test compound of 3 mg/kg) or 10 ml/kg of the solvent control. Just before the administration of the test compound, about 60 μl of blood was collected from the venous plexus of the fundus of the eye using a capillary. After 30 minutes of the administration of the test compound, 200 mg/ml of glucose aqueous solution was orally administered at a dose of 10 ml/kg (corresponds to 2 g/kg). Just before the administration of glucose, about 60 μl of blood was collected from the venous plexus of the fundus of the eye using a capillary. Blood was collected in the same manner after 0.5, 1 and 2 hours of the glucose administration. Blood plasma was separated from the thus collected blood to measure the blood glucose level. AUC of the blood glucose level after the administration of the test compound until 2 hours after the glucose loading was compared with AUC of the solvent control group within the same period of time.
Test results of the administration of 3 mg/kg of respective compounds of the present invention are shown in the following Table 2.
[Table 2]

| Ex | Blood glucose lowering ratio (%) |
|---|---|
| 13 | 19 |
| 45 | 23 |
| 62 | 19 |
| 63 | 35 |
| 71 | 22 |
| 73 | 27 |
| 74 | 23 |
| 77 | 18 |

Test Example 4: Action upon high blood glucose level after oral glucose loading in db/db mice

After overnight fasting, body weights of db/db (C57BL/KsJ-db/db) mice (N = 5) were measured. Each test compound was dissolved in Cremophor solvent (Cremophor:DMSO:Water 5:5:90, v/v/v) to a concentration of 1 mg/ml. To each db/db mouse was orally administered 10 ml/kg of the agent liquid (corresponds to the test compound of 10 mg/kg) or 10 ml/kg of the solvent control. Just before the administration of the test compound, about 60 μl of blood was collected from the venous plexus of the fundus of the eye using a capillary. After 30 minutes of the administration of the GK activator, 200 mg/ml of glucose aqueous solution was orally administered at a dose of 10 ml/kg (corresponds to 2 g/kg). Just before the administration of glucose, about 60 μl of blood was collected from the venous plexus of the fundus of the eye using a capillary. Blood was collected in the same manner after 0.5, 1 and 2 hours of the glucose administration. Blood plasma was separated from the thus collected blood to measure the blood glucose level. AUC of the blood glucose level after the administration of the test compound until 2 hours after the glucose loading was compared with AUC of the solvent control group within the same period of time.
As a result, AUC of the blood glucose level from the administration of 10 mg/kg of the compound Ex 45 of the present invention until 2 hours after the glucose loading was lowered by a factor of 39% in comparison with AUC of the solvent control group.
From the above test results, it was confirmed that the compounds of the present invention have good GK activation action. In addition, since compounds in which various side effects (actions upon hERG and CYP) and/or solubility were improved were also found, it is evident that the compounds of the present invention are useful as agents for preventing and treating diabetes and the like.
The pharmaceutical preparations which comprise one or two or more of the compounds (I) of the present invention or salts thereof as the active ingredient can be prepared by generally used methods using carriers, excipients and the like for pharmaceutical preparations use which are generally used in this field.
The administration may be either oral administration by tablets, pills, capsules, granules, powders, solutions and the like or parenteral administration by injections for intraarticular injection, intravenous injection, intramuscular injection and the like, suppositories, eye drops, eye ointments, transdermal solutions, ointments, transdermal patches, transmucosal solutions, transmucosal patches, inhalations and the like.
As the solid composition for oral administration by the present invention, tablets, powders, granules and the like are

used. In such a solid composition, one or more active substances are mixed with at least one inert filler such as lactose, mannitol, glucose, hydroxypropylcellulose, microcrystalline cellulose, starch, polyvinyl pyrrolidone and/or magnesium alminometasilicate or the like. In accordance with the usual way, the composition may contain inert additives such as lubricants (e.g., magnesium stearate and the like), disintegrators (e.g., carboxymethylstarch sodium and the like), stabilizers, and solubilizing agents. As occasion demands, the tablets or pills may be coated with a sugar coating or a film of a gastric or enteric substance.
As the liquid composition for oral administration, pharmaceutically acceptable emulsions, solutions, suspensions, syrups, elixirs and the like are included, and a generally used inert diluent such as purified water or ethanol is used. In addition to the inert diluent, said liquid composition may contain auxiliary agents such as solubilizing agents, moistening agents, suspending agents and the like, sweeteners, correctives, aromatics and antiseptics.
As the injections for parenteral administration, sterile aqueous or non-aqueous solutions, suspensions and emulsions are included. As the aqueous solvent, for example, distilled water for injection and physiological saline are included. Examples of the non-aqueous solvent include propylene glycol, polyethylene glycol, plant oil (e.g., olive oil or the like), alcohols (e.g., ethanol or the like), polysorbate 80 (the name in Pharmacopeia) and the like. Such a composition may further contain tonicity agents, antiseptics, moistening agents, emulsifying agents, dispersing agents, stabilizing agents or solubilizing agents. These are sterilized by, for example, filtration through a bacteria retaining filter, formulation of bactericides or irradiation. In addition, these can also be used by producing sterile solid compositions and dissolving or suspending them in sterile water or a sterile solvent for injection prior to use.
Transmucosal preparations such as inhalations, transnasal preparations and the like are used in the form of solid, liquid or semisolid, and can be produced in accordance with the conventionally known methods. For example, conventionally known fillers and also pH adjusting agents, antiseptics, surfactants, lubricants, stabilizers, thickeners and the like may be optionally added. An appropriate device for inhalation or blowing can be used for the administration. For example, a compound can be administered as such or as a powder of formulated mixture, or as a solution or suspension in combination with a medically acceptable carrier, by using a conventionally known device (e.g., a measured administration inhalation device or the like) or a sprayer. The dry powder inhaler or the like may be for single or multiple administration use, and a dry powder or powder-containing capsule can be used. Alternatively, it may be in the form of a pressurized aerosol spray or the like which uses an appropriate propellant such as chlorofluoroalkane, hydrofluoroalkane, or a suitable gas such as carbon dioxide or the like.

Generally, in the case of oral administration, the daily dose is approximately from 0.001 to 100 mg/kg, preferably from 0.1 to 30 mg/kg, further preferably from 0.1 to 10 mg/kg, per body weight, and this is administered once or by dividing into 2 to 4 doses. When intravenously administered, it is suitable that the daily dose is approximately from 0.0001 to 10 mg/kg body weight, and this is administered once a day or dividing it into two or more times per day. In addition, in the case of a transmucosal preparation, approximately from 0.001 to 100 mg/kg body weight is administered once a day or dividing into two or more doses. The dose is optionally decided in response to individual case by taking symptom, age, sex and the like into consideration.

## EXAMPLES

The following describes the production methods of the compounds (I) of the present invention further in detail based on examples. The compounds of the present invention are not limited to the compounds described in the following Examples. Also, production methods of the starting material compounds are shown in Reference Examples.
In addition, the following abbreviations are used in Reference Examples, Examples and the tables which are described later. Ex: Example number, Rf: Reference Example number, No: compound number, Dat: physicochemical data (MS: m/z value in mass spectrometry (+:cation,-: anion), NMR 1: δ (ppm) of $^1H$ NMR in DMSO-$d_6$, NMR 2: δ (ppm) of $^1H$ NMR in $CDCl_3$), $[\alpha]^t_D$: specific rotation (chloroform, t (˚C)), Str: structural formula (HCl in the structural formula indicates that it is hydrochloride), Syn: production method (the numeral shows that it is produced using a corresponding starting material, similar to an Example compound having the number as the Example number), RSyn: production method (the numeral shows that it is produced using a corresponding starting material, similar to Reference Example Compound having the number as Reference Example number), Me: methyl, Et: ethyl, Pr: n-propyl, iPr: isopropyl, Ac: acetyl. In addition, the numeral before a substituent group indicates the substituting position, for example, 4-$MeSO_2$ represents 4-methanesulfonyl.

### Reference Example 1

THF (8 ml) and DMPU (2 ml) were added to 1.5 M LDA/cyclohexane solution, and 6-quinolinylacetonitrile (1.75 g) was added dropwise thereto together with THF (8 ml) and DMPU (2 ml) at -60˚C. After 1 hour of stirring of the reaction liquid, iodomethylcyclopentane (2.62 g) was added thereto at -65˚C or below, followed by stirring under cooling for 1 hour and then at room temperature for a whole day and night. The reaction liquid was concentrated, saturated brine

was added, followed by extraction with ethyl acetate. This was dried over anhydrous magnesium sulfate, concentrated and then purified by silica gel column chromatography (hexane/ethyl acetate) to obtain 3-cyclopentyl-2-quinolin-6-ylpropanenitrile (2.61 g) as a pale yellow oily substance.

Reference Example 2

In concentrated hydrochloric acid (30 ml), 3-cyclopentyl-2-quinolin-6-ylpropanenitrile (1.72 g) was stirred with heating at a bath temperature of 100˚C for 4 days. The reaction liquid was concentrated, and 1 M sodium hydroxide aqueous solution (15 ml) was added thereto. After separation of the insoluble matter, the filtrate was washed with diethyl ether and adjusted to a pH of about 2 with 1M hydrochloride acid, and the thus precipitated crystals were collected by filtration to obtain 3-cyclopentyl-2-quinolin-6-ylpropanoic acid as colorless crystals (1.15 g).

Reference Example 3

Under ice-cooling, oxalyl chloride (3.00 ml) was added dropwise to a mixture of 3-cyclopentyl-2-[4-(methylsulfonyl)phenyl]propionic acid (produced in accordance with the method described in WO 00/58293) (1.00 g), DMF (0.130 ml) and dichloromethane (17 ml). The reaction mixture was stirred under ice-cooling for 30 minutes and at room temperature overnight, and then the solvent was evaporated under a reduced pressure. Toluene was added to the resulting residue, and the solvent was again evaporated under a reduced pressure. The resulting residue was dissolved in dichloromethane (17 ml), and diisopropylethylamine (1.18 ml) and ethyl (2-amino-1,3-thiazol-4-yl)oxoacetate (1.35 g) were subsequently added thereto, followed by stirring overnight at room temperature. The reaction solution was washed with 1 M hydrochloric acid, a saturated sodium bicarbonate aqueous solution and saturated brine in that order and then dried over anhydrous magnesium sulfate. The solvent was evaporated under a reduced pressure and the resulting residue was purified by silica gel chromatography (chloroform/methanol) to obtain ethyl [2-({3-cyclopentyl-2-[4-(methylsulfonyl)phenyl]propanoyl}amino)-1,3-thiazol-4-yl]oxoacetate (0.660 g) as an orange amorphous.

Reference Example 4

A 1 M sodium hydroxide aqueous solution (2.21 ml) was added dropwise to a 1,4-dioxane (7.5 ml) solution of methyl 3-(3-cyclopenten-1-yl)-2-[4-(methylsulfonyl)phenyl]propanoate (455 mg) at room temperature, followed by stirring as such for 2 hours. The reaction mixture was concentrated under a reduced pressure, water was added, and then it was acidified with 1 M hydrochloric acid. The white suspension thus formed was collected by filtration to obtain 3-(3-cyclopenten-1-yl)-2-[4-(methylsulfonyl)phenyl]propanoic acid (374 mg) as a white powder.

Reference Example 5

Under ice-cooling, 0.99 M diethylzinc solution (3.67 ml) was added dropwise to a dichloromethane (7.3 ml) solution of methyl 3-(3-cyclopenten-1-yl)-2-[4-(methylsulfonyl)phenyl]propanoate (224 mg), followed by stirring as such for 30 minutes. Then, diiodomethane (1.95 g) was added thereto, followed by stirring at 40˚C for 5 hours. Chloroform and an ammonium chloride aqueous solution were added to the reaction mixture to carry out separation of layers, and the organic layer was dried with magnesium sulfate and evaporated under a reduced pressure. A 1 M sodium hydroxide aqueous solution (1.09 ml) was added dropwise to a 1,4-dioxane (3 ml) solution of the resulting product at room temperature, followed by stirring as such for 2 hours. The reaction mixture was concentrated under a reduced pressure, and water was added, followed by acidification with 1 M hydrochloric acid. Then, the white suspension thus formed was collected by filtration to obtain 3-bicyclo[3.1.0]hexan-3-yl-2-[4-(methylsulfonyl)phenyl]propanoic acid (185 mg) as a pale yellow powder.

Reference Example 6

Pyridine (16 ml) was added to a dichloromethane (250 ml) suspension of ethyl (2-amino-1,3-thiazol-4-yl)(oxo) acetate (26.5 g), and then, under cooling, allyl chloroformate (17 ml) was added dropwise thereto at 0˚C or below. Pyridine (5 ml) was again added thereto under ice-cooling and 5 ml of allyl chloroformate was added dropwise thereto. The reaction liquid was concentrated and the residue was dissolved in ethyl acetate, washed with 1 M hydrochloric acid, a saturated sodium bicarbonate aqueous solution and saturated brine in that order, dried over anhydrous magnesium sulfate and then concentrated to obtain ethyl (2-{[(allyloxy)carbonyl]amino}-1,3-thiazol-4-yl)(oxo)acetate (37.5 g) as a dark brown solid.

Reference Example 7

Under ice-cooling, sodium borohydride (850 mg) was gradually added to a dioxane (15 ml) solution of ethyl (2-{[(allyloxy)carbonyl]amino}-1,3-thiazol-4-yl)(oxo)acetate (1.68 g). After removing the bath, water (1.5 ml) was added dropwise thereto, and after the dropwise addition, the reaction liquid was stirred for 0.5 hour. Thereafter, 12 M hydrochloric acid (10 ml) was further added slowly dropwise thereto, followed by stirring for 0.5 hour. The reaction liquid was concentrated and then suspended in methanol, the insoluble matter was separated by filtration, and the filtrate was concentrated. The concentrate was again suspended in methanol, the insoluble matter was separated by filtration, and the filtrate was concentrated. By adding dichloromethane to the resulting residue, followed by concentration, 1.8 g of a pale yellow amorphous was obtained. Acetone (50 ml) and tosylic acid hydrate (0.12 g) were added to this compound, followed by heating under reflux for 15 hours while carrying out dehydration. The reaction liquid was concentrated and diluted with dichloromethane under ice-cooling. Then, 1 M sodium hydroxide (5 ml) was added thereto and the water layer was diluted to carry out separation of layers. The organic layer was dried over anhydrous magnesium sulfate and then concentrated. The resulting pale yellow crystals were dissolved in THF (20 ml), and diethylamine (2 ml), triphenylphosphine (25 mg) and tetrakistriphenylphosphine palladium (73 mg) were added thereto in that order. After 0.5 hour, tetrakistriphenylphosphine palladium (120 mg) was supplemented, followed by stirring for 3 hours. The reaction liquid was concentrated, diluted with chloroform and then washed with saturated brine. The resulting organic layer was dried over anhydrous magnesium sulfate and then concentrated. By purifying the residue by silica gel column chromatography (chloroform → chloroform/methanol = 98:2), 4-(2,2-dimethyl-1,3-dioxolan-4-yl)-1,3-thiazole-2-amine (754 mg) was obtained as a pale yellow solid.

Reference Example 8

1-Bromo-2,5-pyrrolidinedione was added to an acetic acid (10 ml) solution of ethyl (2-amino-1,3-thiazol-4-yl) oxoacetate under ice-cooling, followed by stirring overnight at room temperature. The solvent was evaporated under a reduced pressure, the resulting residue was adjusted to pH 8 by adding a saturated sodium bicarbonate aqueous solution, followed by extraction with chloroform (30 ml). The organic layer was washed with saturated brine (20 ml) and then dried over anhydrous magnesium sulfate. The solvent was evaporated under a reduced pressure, chloroform (3 ml) was added to the resulting residue, and the precipitate was collected by filtration to obtain ethyl (2-amino-5-bromo-1,3-thiazol-4-yl)oxoacetate (735 mg) as a light brown solid.

Reference Example 9

1-Chloro-2,5-pyrrolidinedione (8.67 g) was added to an acetic acid (100 ml) solution of ethyl (2-amino-1,3-thiazol-4-yl)oxoacetate, followed by stirring overnight at room temperature. The reaction solvent was evaporated under a reduced pressure, ethyl acetate (100 ml) was added to the resulting residue, and the precipitate thus formed was collected by filtration and dried to obtain ethyl (2-amino-5-chloro-1,3-thiazol-4-yl)oxoacetate (3.06 g) as a pale yellow solid.

Reference Example 10

Di-tert-butyl dicarbonate (8.50g) was added to a THF (100 ml) solution of ethyl (2-amino-5-ethyl-1,3-thiazol-4-yl)acetate (4.12 g), followed by stirring at room temperature for 3 hours and then at 70°C for 3 days. After spontaneous cooling to room temperature, the solvent was evaporated under a reduced pressure, and the resulting residue was dissolved in ethyl acetate (50 ml), washed with 1 M hydrochloric acid (50 ml) and saturated brine (50 ml), and then dried over anhydrous magnesium sulfate. The solvent was evaporated under a reduced pressure, and the resulting residue was purified by silica gel column chromatography (hexane/ethyl acetate = 100/0 → 80/20 → 60/40) to obtain ethyl {2-[(tert-butoxycarbonyl)amino]-5-ethyl-1,3-thiazol-4-yl}acetate (4.48 g) as a colorless solid.

Reference Example 11

Selenium dioxide (1.90 g) was added to a 1,4-dioxane (45 ml) solution of ethyl {2-[(tert-butoxycarbonyl)amino]-5-ethyl-1,3-thiazol-4-yl} acetate (4.48 g), followed by stirring overnight at 70°C. After filtering through a celite pad while hot and subsequent washing with dioxane (45 ml), the filtrate was evaporated under a reduced pressure and the resulting residue was purified by silica gel column chromatography (hexane/ethyl acetate = 3/1 → 2/1) to obtain ethyl {2-[(tert-butoxycarbonyl)amino]-5-ethyl-1,3-thiazol-4-yl}hydroxyacetate (2.68 g) as an orange amorphous.

Reference Example 12

A 4 M hydrogen chloride/ethyl acetate (15 ml) solution was added to an ethyl acetate (15 ml) solution of ethyl {2-[(tert-butoxycarbonyl)amino]-5-ethyl-1,3-thiazol-4-yl}hydroxyacetate (2.67 g), followed by stirring at room temperature for 7 hours. After evaporation of the reaction solution under a reduced pressure, ethyl acetate (30 ml) was added and the solvent was again evaporated under a reduced pressure. Ethyl acetate (20 ml) was added to the resulting residue and the precipitate was collected by filtration and dried to obtain ethyl (2-amino-5-ethyl-1,3-thiazol-4-yl)hydroxyacetate hydrochloride (1.38 g) as a brown solid.

Reference Example 13

Under ice-cooling, methylmagnesium bromide 0.82 M THF solution (18 ml) was added to a THF solution (40 ml) of ethyl (2-{[(allyloxy)carbonyl]amino}-1,3-thiazol-4-yl)(oxo)acetate (4.07 g), followed by stirring as such for 2 hours. Under ice-cooling, methylmagnesium bromide 0.82 M THF solution (17 ml) was further added thereto in two portions. Under ice-cooling, a saturated ammonium chloride aqueous solution was added thereto. After extraction with ethyl acetate, the organic layer was washed with saturated brine. This was dried over anhydrous magnesium sulfate, followed by filtration. The crude product obtained by concentration was purified by silica gel chromatography (hexane/ethyl acetate = 5/1 → 2/1) to obtain ethyl 2-(2-{[(allyloxy)carbonyl]amino}-1,3-thiazol-4-yl)-2-hydroxypropanoate (2.59 g) as a pale yellow oily substance.

Reference Example 14

Under ice-cooling, (bromomethyl)cyclopropane (12.6 g) and potassium carbonate (21.4 g) were respectively added to a DMF (100 ml) solution of 4-bromobenzenethiol (14.7 g), followed by stirring at room temperature for 2 hours. Diethyl ether and water were added to the reaction mixture to carry out separation of layers, and the organic layer was washed with 1 M sodium hydroxide aqueous solution and dried over anhydrous magnesium sulfate, followed by evaporation under a reduced pressure, thereby obtaining 1-bromo-4-[(cyclopropylmethyl)sulfanyl]benzene (18.9 g).

Reference Example 15

1) A mixture of 1-bromo-4-[(cyclopropylmethyl)sulfanyl]benzene (7.50 g), 1,1'-bis(diphenylphosphino)ferrocene-palladium(II) dichloride-dichloromethane complex (755 mg), potassium acetate (9.08 g), bis(pinacolato)diboron (8.62 g) and DMF (70 ml) was stirred at 120˚C for 1 hour. Ethyl acetate and water were added to the reaction mixture to carry out separation of layers, the organic layer was extracted twice with ethyl acetate, the combined organic layer was evaporated under a reduced pressure, and the resulting residue was purified by silica gel column chromatography (hexane/ethyl acetate = 4/1). 1,1'-Bis(diphenylphosphino)ferrocene-palladium(II) dichloride-dichloromethane complex (755 mg), a DMF (2 ml) solution of ethyl (2Z)-2-bromo-3-cyclopentylacrylate and a 2 M sodium carbonate aqueous solution (70 ml) were respectively added to a DMF (70 ml) solution of the resulting product, followed by stirring at 80˚C for 4 hours. Ethyl acetate and water were added to the reaction mixture to carry out separation of layers, the organic layer was washed with water and saturated brine and dried over anhydrous magnesium sulfate. Then, the residue obtained by evaporation under a reduced pressure was purified by silica gel column chromatography (hexane/ethyl acetate = 4/1) to obtain ethyl (2E)-3-cyclopentyl-2-{4-[(cyclopropylmethyl)sulfanyl]phenyl}acrylate (7.60 g) as an oily substance.
2) 3-Chloroperbenzoic acid (11.9 g) was added under ice-cooling to a dichloromethane solution (100 ml) of the resulting oily substance, followed by stirring at room temperature for 2 hours. The reaction mixture was treated with sodium thiosulfate under ice-cooling and then extracted three times with dichloromethane. The combined organic layer was washed with a sodium bicarbonate aqueous solution and dried over anhydrous magnesium sulfate. Then, the residue obtained by evaporation under a reduced pressure was purified by silica gel column chromatography (hexane/ethyl acetate = 9/1 to 1/1) to obtain ethyl (2E)-3-cyclopentyl-2-{4-[(cyclopropylmethyl)sulfonyl]phenyl}acrylate (5.80 g) as an oily substance.

Reference Example 16

A mixture of ethyl (2E)-3-cyclopentyl-2-{4-[(cyclopropylmethyl)sulfonyl]phenyl}acrylate (5.50 g), ethanol (40 ml) and a 3 M potassium hydroxide aqueous solution (10 ml) was stirred at 60˚C for 1 hour. The reaction mixture was neutralized with concentrated hydrochloric acid, followed by extraction twice with ethyl acetate. The combined organic

layer was dried over anhydrous sodium sulfate and then the residue obtained by evaporation under a reduced pressure was crystallized from diethyl ether/hexane and washed with ethyl acetate to obtain (2E)-3-cyclopentyl-2-{4-[(cyclopropylmethyl)sulfonyl]phenyl}acrylic acid (4.50 g) as white crystals.

Reference Example 17

Under ice-cooling, ethyl chloroglyoxylate (2.25 g) was added dropwise to a dichloromethane (10 ml) suspension of aluminum(III), chloride (2.60 g). After 30 minutes of stirring, a dichloromethane (5 ml) solution of 1-(cyclopropylsulfanyl)-2-methylbenzene (2.46 g) was added dropwise thereto, followed by stirring at the same temperature for 1 hour and then at room temperature for 6 hours. After adding water to the reaction mixture under ice-cooling, the layers were separated and the organic layer was dried over anhydrous magnesium sulfate and evaporated under a reduced pressure. The resulting residue was purified by silica gel column chromatography (hexane/ethyl acetate = 5/1) to obtain ethyl [4-(cyclopropylsulfanyl)-3-methylphenyl](oxo)acetate (4.0 g) as a pale yellow oily substance.

Reference Example 18

At -5˚C, 1 M lithium hexamethyldisilazide/THF (77 ml) was added dropwise to a THF (100 ml) suspension of (cyclopentylmethyl)(triphenyl)phosphonium iodide (36 g). After the dropwise addition and subsequent 1 hour of stirring on an ice bath, a THF (10 ml) solution of ethyl [4-(cyclopropylsulfanyl)phenyl](oxo)acetate (15 g) was added dropwise thereto at 0˚C or below. The reaction liquid was stirred on an ice bath for 0.5 hour, followed by stirring overnight at room temperature. Under ice-cooling, 1 M hydrochloric acid (75 ml) was added dropwise to the reaction mixture, followed by concentration. Diethyl ether was added thereto and the thus formed solid was separated by filtration. The filtrate was subjected to the separation of layers, and the water layer was extracted with diethyl ether. The combined organic layer was dried over anhydrous magnesium sulfate and then concentrated. By purifying the residue by silica gel column chromatography (hexane → hexane/ethyl acetate = 20/1), a pale yellow oily substance ethyl 3-cyclopentyl-2-[4-(cyclopropylsulfanyl)phenyl]acrylate (7.67 g) was obtained as an E/Z mixture.

Reference Example 19

Under ice-cooling, formic acid (70 ml) and a 30% hydrogen peroxide aqueous solution (20 ml) were added to an E/Z mixture of ethyl (2E)-3-cyclopentyl-2-[4-(cyclopropylthio)phenyl]acrylate (7.1 g), followed by stirring at room temperature for 4 hours. Under ice-cooling, a 10% sodium sulfite aqueous solution was added dropwise to the reaction solution, followed by extraction with ethyl acetate. The organic layer was washed with saturated brine and dried over anhydrous magnesium sulfate. After concentration, ethanol (100 ml), water (20 ml) and 8 M potassium hydroxide (30 ml) were added to the resulting residue, followed by stirring at room temperature. After stirring for a whole day and night, the reaction liquid was concentrated and 12 M hydrochloric acid (20 ml) was added thereto under ice-cooling. The crystals thus formed were collected by filtration and washed with ethyl acetate (5 ml) and diethyl ether (5 ml) to obtain (2E)-3-cyclopentyl-2-[4-(cyclopropylsulfonyl)phenyl]acrylic acid (3.1 g) as colorless crystals.

Reference Example 20

Under ice-cooling, a 30% hydrogen peroxide aqueous solution (151 ml) was added dropwise to a formic acid (481 ml) suspension of an E/Z mixture of ethyl 3-cyclopentyl-2-[4-(cyclopropylsulfanyl)phenyl]acrylate (110 g). After 2 hours at room temperature, a saturated sodium sulfite aqueous solution (900 ml) was added thereto under ice-cooling, followed by stirring at room temperature for 30 minutes. After carrying out separation operation of layers by adding ethyl acetate (1 liter), the resulting organic layer was washed with saturated brine and dried over anhydrous magnesium sulfate. By evaporating the solvent under a reduced pressure, ethyl 3-cyclopentyl-2-[4-(cyclopropylsulfonyl)phenyl]acrylate (121 g) was obtained as a pale yellow oil. A methanol (350 ml) solution of the resulting ethyl 3-cyclopentyl-2-[4-(cyclopropylsulfonyl)phenyl]acrylate (45 g) was added to a methanol (100 ml) suspension of 20% palladium hydroxide/carbon powder (9 g), followed by stirring for 28 hours under a hydrogen pressure of $3 \times 10^5$ Pa. After filtration using celite, the solvent of the filtrate was evaporated under a reduced pressure to obtain ethyl 3-cyclopentyl-2-[4-(cyclopropylsulfonyl)phenyl]propanoate (40 g) as a pale yellow oil.

Reference Example 21

A 1 M sodium hydroxide aqueous solution (260 ml) was added to a THF (130 ml) and ethanol (130 ml) mixed solution of ethyl 3-cyclopentyl-2-[4-(cyclopropylsulfonyl)phenyl]propanoate (39 g), followed by stirring for 2 hours. The solvent was evaporated under a reduced pressure, and diethyl ether and water were added to the resulting residue to

carry out separation operation of layers. After adjusting pH of the resulting water layer to about 3 using 1 M hydrochloric acid, ethyl acetate was added, and separation operation of layers was carried out. The resulting organic layer was washed with saturated brine and then dried over anhydrous magnesium sulfate. By evaporating the solvent under a reduced pressure, 3-cyclopentyl-2-[4-(cyclopropylsulfonyl)phenyl]propanoic acid (33.1 g) was obtained as a colorless solid.
Under ice-cooling, triethylamine (17 ml) was added to a THF (210 ml) suspension of 3-cyclopentyl-2-[4-(cyclopropylsulfonyl)phenyl]propanoic acid (33 g, 102.4 mmol), followed by stirring for 10 minutes. Then, 2,2-dimethylpropanoyl chloride (16 ml) was added dropwise thereto, followed by stirring at 2˚C for 1 hour.
At the same time, an n-butyl lithium hexane solution (1.58 M, 76 ml) was added dropwise at -60˚C to a THF (210 ml) solution of (4R)-4-benzyl-1,3-oxazolidin-2-one (21.8 g), followed by stirring at room temperature and cooled to -70˚C.
An mixture of oxazolidine anion was added dropwise at -60˚C to the previously prepared acid anhydride solution. After completion of the dropwise addition, this was stirred at -60˚C for 1 hour and then stirred at room temperature for 12 hours. After addition of water followed by stirring for 30 minutes, the solvent was evaporated under a reduced pressure. The resulting residue was extracted with ethyl acetate (500 ml) and then the resulting organic layer was washed with saturated brine (400 ml). After drying with anhydrous magnesium sulfate, the solvent was evaporated under a reduced pressure. The resulting residue was purified by silica gel column chromatography (hexane/ethyl acetate = 4/1) to obtain (4R)-4-benzyl-3-{(2R)-3-cyclopentyl-2-[4-(cyclopropylsulfonyl)phenyl]propanoyl-1,3-oxazolidin-2-one (3.2 g) and (4R)-4-benzyl-3-{(2S)-3-cyclopentyl-2-[4-(cyclopropylsulfonyl)phenyl]propanoyl-1,3-oxazolidin-2-one (7.2 g), respectively as colorless amorphous.

Reference Example 22

An aqueous (4 ml) solution of lithium hydroxide monohydrate (563 mg) was added to a 30% hydrogen peroxide aqueous solution (3 ml), and a THF (27 ml) and an aqueous (6 ml) solution of (4R)-4-benzyl-3-{(2R)-3-cyclopentyl-2-[4-(cyclopropylsulfonyl)phenyl]propanoyl-1,3-oxazolidin-2-one were added dropwise thereto under ice-cooling, followed by stirring under ice-cooling for 1 hour. A 10% sodium thiosulfate solution (100 ml) was added to the reaction solution under ice-cooling, followed by extraction with diethyl ether (100 ml x 2). The pH of the resulting water layer was adjusted to about 3 by adding 1 M hydrochloric acid. After extraction with ethyl acetate (200 ml x 2), the resulting organic layer was washed with saturated brine and then dried over anhydrous magnesium sulfate. By evaporating the solvent under a reduced pressure, (2R)-3-cyclopentyl-2-[4-(cyclopropylsulfonyl)phenyl]propanoic acid (2.0 g) was obtained as a colorless solid.

Reference Example 23

Thiourea (1.02 g) was added to an ethanol (30 ml) solution of(2R)-4-bromo-3-oxobutane-1,2-diyl dibenzoate (2.6 g), followed by stirring at 70˚C for 1 hour. After spontaneous cooling at room temperature, the solvent was evaporated under a reduced pressure. A saturated sodium bicarbonate aqueous solution (10 ml), water (30 ml) and ethyl acetate (40 ml) were added to the resulting residue, and the organic layer was washed with water (30 ml) and saturated brine (40 ml) in that order, followed by drying over anhydrous magnesium sulfate. By evaporating the solvent under a reduced pressure, (1S)-1-(2-amino-1,3-thiazol-4-yl)-2-(benzyloxy)ethyl benzoate(2.4g) was obtained as a pale yellow solid.

Reference Example 24

A THF (250 ml) solution of ethyl (2-{[(allyloxy)carbonyl]amino}-1,3-thiazol-4-yl)acetate (50 g) and ethyl formate (20 ml) was added dropwise to a THF (250 ml) suspension of 60% sodium hydride (9.6 g), followed by stirring at room temperature for 12 hours. After adjusting the pH to about 6 by adding 1 M hydrochloric acid, the solvent was evaporated under a reduced pressure. Water (300 ml) and chloroform (400 ml) were added, and separation operation of layers was carried out, followed by washing with saturated sodium bicarbonate aqueous solution (300 ml) and saturated brine(300 ml) in that order and subsequent drying with anhydrous magnesium sulfate. By purifying the resulting residue by silica gel column chromatography (hexane/ethyl acetate = 70/30), ethyl 2-(2-{[(allyloxy)carbonyl]amino}-1,3-thiazol-4-yl)-3-oxopropanoate (48.0 g) was obtained as a pale yellow solid.

Reference Example 25

Ethyl 2-(2-{[(allyloxy)carbonyl]amino}-1,3-thiazol-4-yl)-3-oxopropanoate (20 g) was dissolved in THF (200 ml), ethanol (200 ml) and water (200 ml), and under ice-cooling, sodium borohydride (5.0 g) was added thereto in portionwise. Sodium borohydride (5.0 g) was added thereto 2 hours later, followed by stirring. Further 2 hours later, sodium borohydride (5.0 g) was added thereto, followed by stirring for 12 hours. After evaporation of the solvent under a reduced pressure, the resulting residue was purified by silica gel column chromatography (chloroform/methanol = 100/0 → 95/5) to obtain

allyl {4-[2-hydroxy-1-(hydroxymethyl)ethyl]-1,3-thiazol-2-yl}carbamate (10.8 g) as a pale yellow solid.

Reference Example 26

Under ice-cooling, acetic anhydride (10.6 ml) and pyridine (9.0 ml) were added to a dichloromethane solution (72.5 ml) of allyl {4-[2-hydroxy-l-(hydroxymethyl)ethyl]-1,3-thiazol-2-yl}carbamate (2.9 g). After stirring at room temperature for 11 hours, the solvent was evaporated under a reduced pressure. Water (80 ml) and chloroform (80 ml) were added to the resulting residue, and separation operation of layers was carried out. The resulting organic layer was washed with 1 M hydrochloric acid (80 ml), a saturated sodium bicarbonate aqueous solution (80 ml) and saturated brine (80 ml) in that order, and then dried over anhydrous magnesium sulfate. The solvent was evaporated under a reduced pressure and the resulting residue was purified by silica gel column chromatography to obtain 2-(2-{[(allyloxy) carbonyl]amino}-1,3-thiazol-4-yl)propane-1,3-diyl diacetate (3.57 g) as a dark brown oil.

Reference Example 27

Diethylamine (3.4 ml) and tetrakis(triphenylphosphine palladium (760 mg) were added to a THF (30 ml) solution of 2-(2-{[(allyloxy)carbonyl]amino]-1,3-thiazol-4-yl)propane-1,3-diyl diacetate (3.0 g), followed by stirring at room temperature for 11 hours. The solvent was evaporated under a reduced pressure, water (30 ml) and chloroform (60 ml) were added, and then an extraction operation was carried out. The resulting organic layer was washed with a saturated sodium bicarbonate aqueous solution (30 ml) and saturated brine (30 ml) in that order and then dried over anhydrous magnesium sulfate. After evaporation of the solvent under a reduced pressure, the resulting residue was purified by silica gel column chromatography (chloroform/methanol = 100/0 → 94/6) to obtain 2-(2-amino-1,3-thiazol-4-yl)propane-1,3-diyl diacetate (2.07 g) as a dark brown oil.

Reference Example 28

Carbon tetrachloride (0.2 ml) was added to a mixed solution of magnesium (698 mg) and ethanol (5 ml), followed by stirring. This was stirred at room temperature for 30 minutes and then stirred at 85˚C for 1 hour. After spontaneous cooling to room temperature, diethyl methylmalonate (5.0 g) was added dropwise thereto. After 30 minutes of reflux by adding diethyl ether (7 ml) and subsequent ice-cooling, chloroacetyl chloride (2.3 ml) was added dropwise thereto, followed by stirring overnight at 100˚C. After addition of 3 M sulfuric acid (10 ml) and subsequent stirring for 15 minutes, diethyl ether (40 ml) was added, and an extraction operation was carried out. The resulting organic layer was dried over anhydrous magnesium sulfate. The solvent was evaporated under a reduced pressure, the resulting residue was dissolved in ethanol (100 ml), and thiourea (4.4 g) was added thereto, followed by stirring for 12 hours. After evaporation of the solvent, the residue was dissolved by adding water (20 ml). The solid precipitated by adding a saturated bicarbonate aqueous solution (30 ml) was collected by filtration and dried to obtain diethyl (2-amino-1,3-thiazol-4-yl)(methyl)malonate (2.3 g) as a colorless solid.

Reference Example 29

Acetic anhydride (12.8 ml) and pyridine (11.0 ml) were added to a dichloromethane solution (80 ml) of 2-(2-bromo-2-propen-1-yl)-1,3-propanediol (2.65 g), followed by stirring at room temperature for 20 hours. Chloroform and 1 M hydrochloric acid were added to the reaction mixture to carry out separation of layers, and the organic layer was washed with a saturated sodium bicarbonate aqueous solution and saturated brine, respectively. The organic layer was dried over anhydrous magnesium sulfate and the solvent was evaporated under a reduced pressure. By purifying the resulting residue by silica gel column chromatography (ethyl acetate/hexane = 10/90 → 20/80 → 30/70), 2-(acetoxymethyl)-4-bromo-4-penten-1-yl acetate (2.61 g) was obtained as a colorless oily substance.

Reference Example 30

N-bromosuccinimide (2.00 g) and 20% hydrogen bromide (ethanol solution, 92 μl) were respectively added to an acetonitrile (40 ml)/water (10 ml) mixed solution of 2-(acetoxymethyl)-4-bromo-4-penten-1-yl acetate (2.61 g), followed by stirring at room temperature for 5 hours. The reaction mixture was diluted with diethyl ether, and a sodium thiosulfate aqueous solution was added thereto. After 10 minutes of stirring and subsequent separation of layers, the organic layer was washed with water and saturated brine, respectively. The organic layer was dried over anhydrous magnesium sulfate and the solvent was evaporated under a reduced pressure. By purifying the resulting residue by silica gel column chromatography (ethyl acetate/hexane = 10/90 → 20/80 → 30/70), 2-(acetoxymethyl)-5-bromo-4-oxopentyl acetate (0.830 g) was obtained as a colorless oily substance.

Reference Example 31

Thiourea (214 mg) was added to an ethanol (20 ml) solution of 2-(acetoxymethyl)-5-bromo-4-oxopentyl acetate (830 mg), followed by stirring at 60˚C for 1 hour. By concentrating the reaction mixture under a reduced pressure, 2-[(2-amino-1,3-thiazol-4-yl)methyl]propane-1,3-diyl diacetate (760 mg) was obtained as a white solid.

Reference Example 32

Thionyl chloride (0.165 ml) was added to a dichloromethane (1.7 ml) solution of 2-{[(allyloxy)carbonyl]amino}-1,3-thiazole-4-carboxylic acid (43.0 mg), followed by stirring at 60˚C for 1 hour. Ethyl malonate potassium salt (67.0 mg), magnesium chloride (44.0 mg) and triethylamine (83 μl) were added to an acetonitrile (1.38 ml) solution of a residue which had been obtained by adding toluene to the reaction mixture and concentration under a reduced pressure, followed by stirring overnight at room temperature. Ethyl acetate and water were added to the residue obtained by adding toluene to the reaction mixture and concentration under a reduced pressure to carry out separation of layers. The organic layer was washed with saturated brine and dried over anhydrous sodium sulfate. The solvent was evaporated under a reduced pressure and the resulting residue was purified by silica gel column chromatography (methanol/chloroform = 0/100 → 2/98 → 4/96) to obtain ethyl 3-(2-{[(allyloxy)carbonyl]amino}-1,3-thiazol-4-yl)-3-oxopropanoate (13.0 mg) as a yellow oily substance.

Reference Example 33

Lithium borohydride (123 mg) was added to a THF (0.3 ml), ethanol (0.3 ml) and water (0.3 ml) mixed solution of ethyl 3-(2-{[(allyloxy)carbonyl]amino}-1,3-thiazol-4-yl)-3-oxopropanoate (56.0 mg), followed by stirring at 70˚C for 2 hours. Ethyl acetate and a saturated ammonium chloride aqueous solution were added to the reaction mixture to carry out separation of layers. The organic layer was washed with saturated brine and dried over anhydrous sodium sulfate, and then the solvent was evaporated under a reduced pressure. By purifying the residue by silica gel column chromatography (methanol/chloroform = 0/100 → 2/98 → 5/95), allyl [4-(1,3-dihydroxypropyl)-1,3-thiazol-2-yl)]carbamate (30 mg) was obtained as a colorless oily substance.

Reference Example 34

A mixture of allyl [4-(1,3-dihydroxypropyl)-1,3-thiazol-2-yl)]carbamate (5.5 g), acetic anhydride (12 ml) and pyridine (10.0 ml) was stirred at 70˚C for 2 hours. Ethyl acetate and 1 M hydrochloric acid were added to the reaction mixture to carry out separation of layers. The organic layer was washed with a saturated sodium bicarbonate aqueous solution and saturated brine, respectively, and dried over anhydrous magnesium sulfate. Then, the solvent was evaporated under a reduced pressure. By purifying the resulting residue by silica gel column chromatography (ethyl acetate/hexane = 10/90 → 30/70 → 50/50), 1-(2-{[(allyloxy)carbonyl]amino}-1,3-thiazol-4-yl)propane-1,3-diyl diacetate (1.52 g) was obtained as a pale yellow oily substance.

Reference Example 35

Tetrakis(triphenylphosphine)palladium (479 mg) and diethylamine (1.52 ml) were added to a THF (40 ml) solution of 1-(2-{[(allyloxy)carbonyl]amino}-1,3-thiazol-4-yl)propane-1,3-diyl diacetate (1.42 g), followed by stirring at room temperature for 1.5 hours. Ethyl acetate and water were added to the reaction mixture to carry out separation of layers. The organic layer was washed with a saturated sodium bicarbonate aqueous solution and saturated brine, respectively, and dried over anhydrous sodium sulfate. Then, the solvent was evaporated under a reduced pressure. By purifying the resulting residue by silica gel column chromatography (ethyl acetate/hexane = 20/80 → 50/59 → 70/30), 1-(2-amino-1,3-thiazol-4-yl)propane-1,3-diyl diacetate (1.06 g) was obtained as a pale yellow oily substance.

Reference Example 36

A mixture of 4-bromo-N-ethylbenzenesulfonamide (6.00 g), 1,1'-bis(diphenylphosphino)ferrocene-palladium (II) dichloride-dichloromethane complex (557 mg), potassium acetate (6.69 g), bis(pinacolato)diboron (6.35 g) and DMF (60 ml) was stirred at 120˚C for 1 hour. Ethyl acetate and water were added to the reaction mixture to carry out separation of layers. The organic layer was evaporated under a reduced pressure and the resulting residue was purified by silica gel column chromatography (hexane/ethyl acetate = 4/1). 1,1'-Bis(diphenylphosphino)ferrocene-palladium(II) dichloride-dichloromethane complex (557 mg), DMF (2 ml) solution of ethyl (2Z)-2-bromo-3-cyclopentylacrylate (3.37 g) and 2 M sodium carbonate aqueous solution (30 ml) were respectively added to a DMF (30 ml) solution of the resulting product,

followed by stirring at 80˚C for 2 hours. Ethyl acetate and water were added to the reaction mixture to carry out separation of layers. The organic layer was washed with water and saturated brine, respectively, and dried over anhydrous magnesium sulfate. The residue obtained by evaporation under a reduced pressure was purified by silica gel column chromatography (hexane/ethyl acetate = 4/1) to obtain ethyl (2E)-3-cyclopentyl-2-{4-[(ethylamino)sulfonyl]phenyl}acrylate (1.99 g) as an oily substance.

Reference Example 37

Under ice-cooling, sodium borohydride (427 mg) was added to a THF (100 ml) solution of ethyl (2-{[(allyloxy) carbonyl]amino}-1,3-thiazol-4-yl)(oxo)acetate (10.7 g), followed by stirring under ice-cooling for 1 hour. Ethyl acetate and 1 M hydrochloric acid were added to the reaction mixture to carry out separation of layers. The organic layer was washed with saturated brine, dried over anhydrous magnesium sulfate, and then the solvent was evaporated under a reduced pressure. By purifying the resulting residue by silica gel column chromatography (ethyl acetate/hexane = 30% to 50%), ethyl 2-{[(allyloxy)carbonyl]amino}-1,3-thiazol-4-yl)(hydroxy)acetate (9.70 g) was obtained as a pale yellow oily substance.

Reference Example 38

At -70˚C, a 3 M methylmagnesium bromide THF solution (50.8 ml) was added to a THF (100 ml) solution of ethyl (2-{[(allyloxy)carbonyl]amino}-1,3-thiazol-4-yl)(hydroxy)acetate (9.70 g). After 2 hours of stirring, the temperature was allowed to warm to 0˚C, followed by 1 hour of stirring. Under ice-cooling, saturated ammonium chloride aqueous solution was added thereto, followed by extraction with ethyl acetate. The organic layer was washed with saturated brine, dried over anhydrous magnesium sulfate, and then the solvent was evaporated under a reduced pressure. By purifying the resulting crude product by silica gel column chromatography (ethyl acetate/hexane = 10/90 →20/80 → 30/70 → 40/60), allyl [4-(1,2-dihydroxy-2-methylpropyl)-1,3-thiazol-2-yl]carbamate (1.73 g) as a pale yellow solid.

Reference Example 3 9

p-Toluenesulfonic acid (105 mg) was added to an acetone dimethyl acetal (50 ml) solution of allyl [4-(1,2-dihydroxy-2-methylpropyl)-1,3-thiazol-2-yl]carbamate (830 mg), followed by stirring overnight at room temperature. Ethyl acetate and a saturated sodium bicarbonate aqueous solution were added to the reaction mixture to carry out separation of layers. The organic layer was washed with saturated brine and dried over anhydrous magnesium sulfate. By evaporating the solvent under a reduced pressure, allyl [4-(2,2,5,5-tetramethyl-1,3-dioxolan-4-yl)-1,3-thiazol-2-yl]carbamate (952 mg) was obtained as a yellow oily substance.

Reference Example 40

Benzoyl chloride (2.8 ml) was added under ice-cooling to a pyridine (7 ml) solution of benzyl (2S)-2,3-dihydroxypropanoate (2.12 g), followed by stirring at room temperature for 2 hours. Water (30 ml) and ethyl acetate (50 ml) were added to the reaction solution. The organic layer was washed with a 1 M hydrochloric acid(30 ml x 2), water (30 ml), a saturated sodium bicarbonate aqueous solution (20 ml) and saturated brine (30 ml), and then dried over anhydrous magnesium sulfate. The desiccant was removed and the solvent was evaporated under a reduced pressure. The resulting colorless solid was dissolved in THF (20 ml), and 10% palladium/carbon was added thereto under an atmosphere of nitrogen, followed by stirring under an atmosphere at 3 atm of hydrogen at room temperature for 6 hours. After filtration of the reaction solution through celite, the filtrate was concentrated under a reduced pressure, and the resulting residue was purified by silica gel column chromatography (chloroform/methanol = 100/0 → 90/10) to obtain (2S)-2,3-bis(benzoyloxy)propionic acid (440 mg) as a colorless solid.

Reference Example 41

Under ice-cooling, oxalyl dichloride (1 ml) and a few drops of DMF were added to a dichloromethane (5 ml) solution of (2S)-2,3-bis(benzoyloxy)propionic acid (440 mg), followed by stirring at room temperature for 2 hours. A light brown oil obtained by evaporating the reaction solvent under a reduced pressure was dissolved in THF (5 ml), and a 2 M diazomethyltrimethylsilane/diethyl ether solution (2.4 ml) was added dropwise thereto at an inner temperature of -20˚C. After rising the temperature to 10˚C, a yellow syrup obtained by evaporating the reaction solvent under a reduced pressure was dissolved in THF (5 ml), and a 48% hydrobromic acid aqueous solution (1 ml) was added thereto at an inner temperature of -30˚C which was then allowed to rise to room temperature. THF was evaporated under a reduced pressure, dichloromethane (30 ml) and water (30 ml) were added to the resulting residue, and the organic layer was

dried over anhydrous magnesium sulfate. The solvent was evaporated under a reduced pressure and the resulting brown syrup was allowed to stand overnight at room temperature, thereby obtaining a light brown solid. By washing the resulting solid with diethyl ether, (2S)-4-bromo-3-oxobutane-1,2-diyl dibenzoate (242 mg) as a colorless solid.

Reference Example 42

Thiourea (90 mg) was added to an ethanol (5 ml) solution of (2S)-4-bromo-3-oxobutane-1,2-diyl dibenzoate (235 mg), followed by stirring at 70˚C for 30 minutes. After spontaneous cooling to room temperature, the solvent was evaporated under a reduced pressure, a saturated sodium bicarbonate aqueous solution (10 ml), water (30 ml) and ethyl acetate (40 ml) were added to the resulting residue. The organic layer was washed with water (30 ml) and saturated brine (40 ml) in that order and then dried over anhydrous magnesium sulfate. By evaporating the solvent under a reduced pressure, (1R)-1-(2-amino-1,3-thiazol-4-yl)-2-(benzoyloxy)ethyl benzoate (210 mg) was obtained as a pale yellow solid.

In the same manner as in Reference Examples 1 to 42, Reference Example Compounds 43 to 67 which are described later in Tables 3 to 11 were produced using corresponding starting materials. Structures and physicochemical data of Reference Example Compounds are shown in the Tables 3 to 11.

Example 1

At -10˚C, phosphorus oxychloride (70 μl) was added to a pyridine (2 ml) solution of 3-cyclopentyl-2-quinolin-6-ylpropanoic acid (202 mg) and 2-amino-5-chlorothiazole. After 30 minutes of stirring, the temperature was gradually risen, and when the inner temperature was risen to 10˚C, the reaction liquid was diluted with chloroform and water. After adjusting the pH to about 9 by adding a small amount of a sodium bicarbonate aqueous solution, separation of layers was carried out and the organic layer was washed with water and saturated brine. The organic layer was dried over anhydrous magnesium sulfate and concentrated, and then the residue was purified by silica gel column chromatography (chloroform/methanol). By washing the resulting solid with hot ethyl acetate/hexane mixed liquid, N-(5-chloro-1,3-thiazol-2-yl)-3-cyclopentyl-2-quinolin-6-ylpropanamide (99 mg) was obtained as a colorless solid.

Example 2

Under ice-cooling, oxalyl chloride (15.0 ml) was added dropwise to a mixture of 3-cyclopentyl-2-[4-(methylsulfonyl)phenyl]propionic acid (produced in accordance with the method described in WO 00/58293) (5.00 g), DMF (0.039 ml) and dichloromethane (45 ml). The reaction mixture was stirred under ice-cooling for 30 minutes and at room temperature for 2 days, and then the solvent was evaporated under a reduced pressure. Toluene was added to the resulting residue and the solvent was again evaporated under a reduced pressure to obtain a colorless solid (5.30 g). After dissolving a portion of the colorless solid (490 mg) in dichloromethane (7 ml), under ice-cooling, diisopropylethylamine (0.550 ml) was added thereto and then a THF (3 ml) solution of ethyl (2-amino-1,3-thiazol-5-yl)hydroxyacetate (630 mg) was added thereto, followed by 3 days of stirring at room temperature. Water (20 ml) was added to the reaction mixture, followed by extraction with chloroform (20 ml). The organic layer was washed with saturated brine and then dried over anhydrous magnesium sulfate. The solvent was evaporated under a reduced pressure and the resulting residue was purified by silica gel column chromatography (chloroform/methanol) to obtain ethyl [2-({3-cyclopentyl-2-[4-(methylsulfonyl)phenyl]propanoyl}amino)-1,3-thiazol-5-yl]hydroxyacetate (306 mg) as a yellow amorphous.

Example 3 and Example 4

Under ice-cooling, sodium borohydride (20 mg) was added to a THF (10 ml) solution of ethyl [2-({3-cyclopentyl-2-[4-(methylsulfonyl)phenyl]propanoyl}amino)-1,3-thiazol-5-yl]oxoacetate (345 mg), followed by stirring at room temperature for 1 hour. Water (40 ml) and saturated brine (20 ml) were added to the reaction mixture, followed by extraction with ethyl acetate (100 ml). The resulting organic layer was washed with saturated brine and then dried over anhydrous magnesium sulfate. The solvent was evaporated under a reduced pressure and the resulting residue was purified by silica gel column chromatography (chloroform/methanol) to obtain the firstly eluted ethyl [2-({3-cyclopentyl-2-[4-(methylsulfonyl)phenyl]propanoyl}amino)-1,3 -thiazol-4-yl]hydroxyacetate (Example 3: 130 mg) as a pale yellow amorphous, and the secondly eluted 3-cyclopentyl-N-[4-(1,2-dihydroxyethyl)-1,3-thiazol-2-yl]-2-[4-(methylsulfonyl)phenyl]propanamide (Example 4: 150 mg) as a colorless amorphous.

Example 5

Under ice-cooling, 1-bromo-2,5-pyrrolidinedione (5.62 g) was added in small portions to a dichloromethane (56 ml) solution of triphenylphosphine (8.30 g). After 20 minutes of stirring, a dichloromethane (28 ml) solution of (2R)-3-cyclopentyl-2-[4-(methylsulfonyl)phenyl]propionic acid (produced in accordance with the method described in WO 00/58293) (5.50 g) was added dropwise thereto, followed by further stirring for 20 minutes. Ethyl (2-amino-1,3-thiazol-4-yl)oxoacetate (9.35 g) was added to the reaction mixture and stirred overnight at room temperature. Chloroform (100 ml) was added to the reaction mixture, and the organic layer was washed with 1 M hydrochloric acid (150 ml, twice), water (100 ml), saturated sodium bicarbonate aqueous solution (150 ml, twice) and saturated brine (100 ml) in that order. After drying over anhydrous magnesium sulfate, the solvent was evaporated under a reduced pressure and the resulting residue was purified by silica gel column chromatography (chloroform). Sodium borohydride (3.51 g) was added under ice-cooling to a THF (90 ml) solution of the resulting product, followed by stirring at room temperature for 30 minutes. Then, ethanol (15 ml) was added thereto, followed by stirring at room temperature for 30 minutes. Water (100 ml) was added to the reaction mixture and THF was evaporated under a reduced pressure. After extractions with chloroform (50 ml, twice), the organic layer was washed with saturated brine and then dried over anhydrous magnesium sulfate. The solvent was evaporated under a reduced pressure and the resulting residue was purified by silica gel column chromatography (chloroform/methanol)to obtain (2R)-3-cyclopentyl-N-[4-(1,2-dihydroxyethyl)-1,3-thiazol-2-yl]-2-[4-(methylsulfonyl)phenyl]propanamide (4.05 g) as a colorless amorphous.

Example 6

THF (3 ml) and 1 M hydrochloric acid (3 ml) were added to (2E)-3-cyclopentyl-N-[4-(2,2-dimethyl-1,3-dioxolan-4-yl)-1,3-thiazol-2-yl]-2-[4-(methylsulfonyl)phenyl]acrylamide (150 mg), followed by stirring overnight at room temperature. The reaction solution was concentrated under a reduced pressure, dissolved in chloroform and then washed with 1 M hydrochloric acid, saturated sodium bicarbonate aqueous solution and saturated brine. The resulting organic layer was dried over anhydrous magnesium sulfate and then concentrated under a reduced pressure. The residue was crystallized with dichloromethane and then concentrated under a reduced pressure. The crystals were washed with ethyl acetate to obtain (2E)-3-cyclopentyl-N-[4-(1,2-dihydroxyethyl)-1,3-thiazol-2-yl]-2-[4-(methylsulfonyl)phenyl]acrylamide (85 mg) as colorless crystals.

Example 7

Sodium borohydride (150 mg) was added under ice-cooling to a THF (5 ml) solution of ethyl {2-[(3-cyclopentyl-2-{4-[4-(trifluoromethyl)sulfonyl]phenyl}propanoyl)amino]-1,3-thiazol-4-yl]oxoacetate (416 mg), followed by stirring at room temperature for 30 minutes. Then, ethanol (5 ml) was added thereto, followed by stirring at room temperature for 30 minutes. Water (20 ml) was added to the reaction solution, the solvent was evaporated under a reduced pressure, and water (30 ml) and chloroform (50 ml) were added to the resulting residue. The organic layer was washed with saturated brine (50 ml) and dried over anhydrous magnesium sulfate. The solvent was evaporated under a reduced pressure and the resulting residue was purified by silica gel column chromatography (chloroform/methanol = 100/0 → 97/3) to obtain 3-cyclopentyl-N-[4-(1,2-dihydroxyethyl)-1,3-thiazol-2-yl]-2-{4-[(trifluoromethyl)sulfonyl]phenyl}propanamide (230 mg) as a colorless amorphous.

Example 8

Under ice-cooling, pyridine (0.14 ml) and acetic anhydride (0.16 ml) were added to a dichloromethane solution (2 ml) of (2E)-3-cyclopentyl-2-[4-(cyclopropylsulfonyl)phenyl]-N-[4-(1,2-dihydroxyethyl)-1,3-thiazol-2-yl]acrylamide (79 mg). This was stirred overnight at room temperature, water was added, followed by extraction with ethyl acetate. The organic layer was washed with a 1 M hydrochloric acid and saturated brine and dried over anhydrous magnesium sulfate. The crude product obtained by concentration was purified by silica gel column chromatography (hexane/ethyl acetate = 10/1 → 3/1). The resulting oily substance was made into powder using hexane as the solvent and then collected by filtration to obtain 1-[2-({(2E)-3-cyclopentyl-2-[4-(cyclopropylsulfonyl)phenyl]-2-propenoyl}amino)-1,3-thiazol-4-yl]ethylene glycol diacetate (41 mg) as a white solid.

Example 9

Under ice-cooling, acetic anhydride (36 ml) and pyridine (0.26 ml) were added to a dichloromethane solution (2 ml) of (2E)-3-cyclopentyl-2-[4-(cyclopropylsulfonyl)phenyl]-N-[4-(1,2-dihydroxyethyl)-1,3-thiazol-2-yl]acrylamide (150 mg). This was stirred overnight at room temperature. Deionized water was added thereto, followed by extraction with ethyl acetate. The organic layer was washed with 1 M hydrochloric acid and saturated brine. The organic layer was dried over anhydrous magnesium sulfate, followed by concentration. The resulting crude product was purified by silica gel column chromatography (hexane/ethyl acetate = 1/1 → 1/5). The resulting white solid was made into powder using s solvent (hexane/diisopropyl ether = 10/1) and then collected by filtration to obtain 2-[2-({(2E)-3-cyclopentyl-2-[4-(cyclopropylsulfonyl)phenyl]-2-propenoyl}amino)-1,3-thiazol-4-yl]-2-hydroxyethyl acetate (77 mg) as a white solid.

Example 10

Under ice-cooling, N-bromosuccinimide (325 mg) was added to a dichloromethane (3 ml) solution of triphenylphosphine(479mg), followed by stirring for 30 minutes. Then, (2E)-2-[3-chloro-4-(methylsulfonyl)phenyl]-3-cyclopentylacrylic acid(300 mg) was added thereto. After further stirring under ice-cooling for 30 minutes, 4-(2,2-dimethyl-1,3-dioxolan-4-yl)-1,3-thiazole-2-amine (548 mg) was added thereto, followed by stirring at the same temperature for 1 hour and at room temperature for 1 hour. Ethyl acetate and water were added to the reaction mixture to carry out separation of layers, and the organic layer was washed with 1 M hydrochloric acid, a saturated sodium bicarbonate aqueous solution and saturated brine, respectively, and then dried over anhydrous magnesium sulfate. The solvent was evaporated under a reduced pressure and the resulting residue was purified by silica gel column chromatography (hexane/ethyl acetate = 7/3) to obtain (2E)-2-[3-chloro-4-(methylsulfonyl)phenyl]-3-cyclopentyl-N-[4-(2,2-dimethyl-1,3-dioxolan-4-yl)-1,3-thiazol-2-yl]acrylamide (132 mg) as a pale orange powder.

Example 11

Potassium carbonate (147 mg) was added to a methanol solution (3 ml) of 2-[2-({(2E)-3-cyclopentyl-2-[4-(cyclopropylsulfonyl)phenyl]prop-2-enoyl}amino)-1,3-thiazol-4-yl]propane-1,3-diyl diacetate (200 mg), followed by stirring at room temperature for 30 minutes. After carrying out separation operation of layers by adding water (30 ml) and chloroform thereto, the resulting organic layer was washed with saturated brine (30 ml) and dried over anhydrous magnesium sulfate. After evaporation of the solvent under a reduced pressure, the resulting residue was crystallized using a solvent (dichloromethane/diethyl ether = 2/1) and collected by filtration to obtain (2E)-3-cyclopentyl-2-[4-(cyclopropylsulfonyl)phenyl]-N-{4-[2-hydroxy-1-(hydroxymethyl)ethyl]-1,3-thiazol-2-yl}acrylamide (142 mg) as colorless crystals.

Example 12

(2E)-3-Cyclopentyl-2-[4-(cyclopropylsulfonyl) phenyl]-N-[4-(1,2-dihydroxyethyl)-1,3-thiazol-2-yl] acrylamide (270 mg) and 1,1'-carbonyldiimidazole (124 mg) were dissolved in THF (5.4 ml), followed by stirring at room temperature for 12 hours. The solvent was evaporated under a reduced pressure and the resulting residue was purified by silica gel column chromatography (hexane/ethyl acetate = 80/20 → 60/40). The solvent was evaporated under a reduced pressure and the resulting colorless amorphous was crystallized using a solvent (dichloromethane/diethyl ether = 3/1) and collected by filtration to obtain (2E)-3-cyclopentyl-2-[4-(cyclopropylsulfonyl)phenyl]-N-[4-(2-oxo-1,3-dioxolan-4-yl)-1,3-thiazol-2-yl]acrylamide (125 mg) as a colorless solid.

Example 13

A 1 M sodium hydroxide aqueous solution (13 ml) was added to a THF (5 ml) and ethanol (5 ml) mixed solution of (1S)-2-(benzoyloxy)-1-[2-({(2E)-3-cyclopentyl-2-[4-(cyclopropylsulfonyl)phenyl]-2-propenoyl}amino)-1,3-thiazol-4-yl] ethyl benzoate (338 mg), followed by stirring at room temperature for 14 hours. The reaction solvent was evaporated under a reduced pressure and then water (30 ml) and dichloromethane (30 ml) were added to the residue. The organic layer was washed with saturated sodium bicarbonate aqueous solution (30 ml) and saturated brine (40 ml) and then dried over anhydrous magnesium sulfate. By evaporating the solvent under a reduced pressure, (2E)-3-cyclopentyl-2-[4-(cyclopropylsulfonyl)phenyl]-N-{4-[(1S)-1,2-dihydroxyethyl]-1,3-thiazol-2-yl}acrylamide (40 mg) was obtained as a colorless amorphous.

Example 14

1-[Bis(dimethylamino)methylene]-1H-1,2,3-triazolo[4,5-b]pyridinium 3-oxido hexafluorophosphate (HATU) (890 mg) and 4-dimethylaminopyridine (DMAP) (286 mg) were added to a DMF (10 ml) solution of (2E)-3-cyclopentyl-2-[4-(cyclopropylsulfonyl)phenyl]acrylic acid (500 mg), followed by stirring at room temperature for 25 minutes. Then, a DMF (2 ml) solution of 4-(2,2,5,5-tetramethyl-1,3-dioxolan-4-yl)-1,3-thiazole-2-amine (356 mg) was added thereto at room temperature, followed by stirring at 70˚C for 4 hours. Ethyl acetate and water were added to the reaction mixture to carry out separation of layers. The organic layer was washed with 1 M hydrochloric acid, a saturated sodium bicarbonate aqueous solution and saturated brine, respectively, and then dried over anhydrous magnesium sulfate. Then, the solvent was evaporated under a reduced pressure. The resulting residue was purified by silica gel column chromatography (ethyl acetate/hexane = 10/90 → 30/70 → 50/50) to obtain (2E)-3-cyclopentyl-2-[4-(cyclopropylsulfonyl)phenyl]-N-[4-(2,2,5,5-tetramethyl-1,3-dioxolan-4-yl)-1,3-thiazol-2-yl]acrylamide (335 mg) as a colorless oily substance.

Example 15

Manganese dioxide (480 mg) was added to a dichloromethane (4 ml) solution of ethyl 2-[2-({(2E)-3-cyclopentyl-2-[4-(cyclopropylsulfonyl)phenyl]-2-propenoyl}amino)-1,3-thiazol-4-yl]-2-hydroxyacetate (94.0 mg), followed by stirring at room temperature for 40 hours. After separation of the insoluble matter by filtration and subsequent concentration under a reduced pressure, ethyl 2-[2-({(2E)-3-cyclopentyl-2-[4-(cyclopropylsulfonyl)phenyl]-2-propenoyl}amino)-1,3-thiazol-4-yl]-2-oxoacetate (82 mg) was obtained as a colorless oily substance.

In the same manner as in Examples 1 to 15, the Example compounds 16 to 89 shown in the following tables 12 to 27 were produced using corresponding starting materials. Structures and physicochemical data of the Example compounds are shown in the following tables 12 to 27.

In addition, structures of other compounds of the present invention are shown in Tables 28 to 31. These can be easily synthesized using the methods described in the aforementioned production methods and Examples and the methods obvious to those skilled in the art, or modified methods thereof.

[Table 3]

| Rf | RSyn | Str | Dat |
|---|---|---|---|
| 1 | 1 | 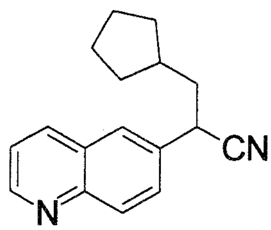 | MS(FAB+):251 |
| 2 | 2 | 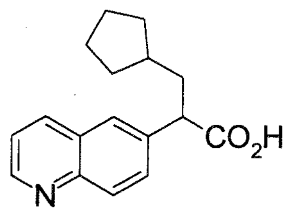 | MS(FAB+):270 |
| 3 | 3 | 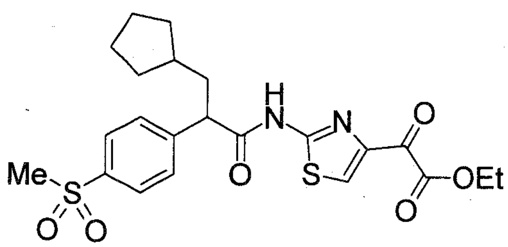 | MS(FAB+):479 |
| 43 | 1 | 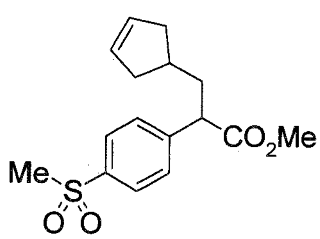 | MS(ESI-):307 |

(continued)

| Rf | RSyn | Str | Dat |
|---|---|---|---|
| 4 | 4 | 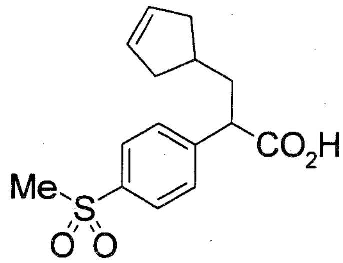 | MS (ESI+):295 |
| 5 | 5 | 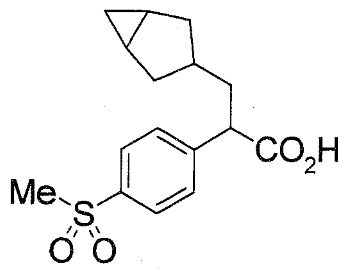 | MS (ESI+):309 |
| 6 | 6 | 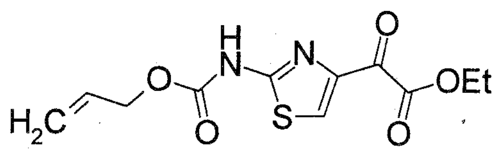 | MS (ESI+):285 |

[Table 4]

| | | | |
|---|---|---|---|
| 7 | 7 | 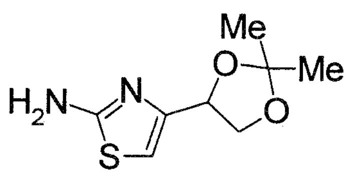 | MS(EI):200 |
| 8 | 8 | 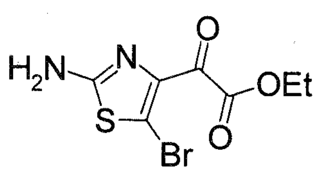 | MS (EI): 278, 280 |
| 9 | 9 | 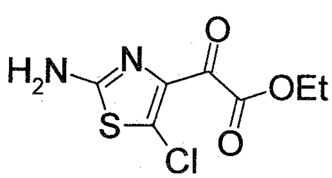 | MS(EI):234 |
| 10 | 10 | 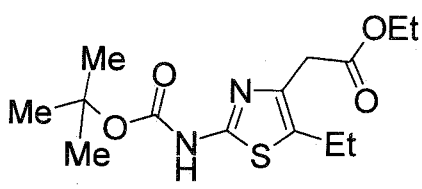 | MS(FAB+):315 |

(continued)

| | | | |
|---|---|---|---|
| 11 | 11 | 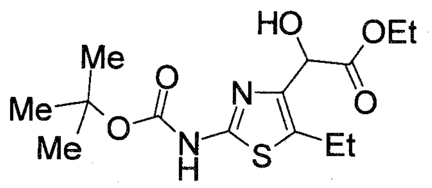 | MS(FAB+):331 |
| 12 | 12 | 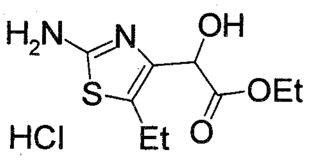 | MS(FAB+):231 |
| 13 | 13 | 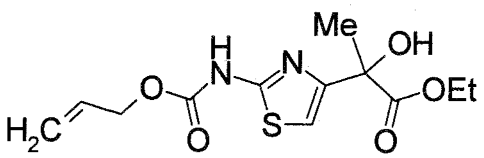 | MS(FAB+):301 |
| 44 | 7 | 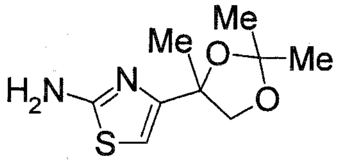 | MS(EI):214 |
| 14 | 14 | 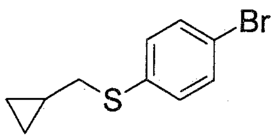 | NMR2:021-027 (2H, m), 0.55-0.61 (2H, m), 0.97-1.08 (1H, m), 2.84 (2H, d, J = 6.9 Hz), 7.22 (2H, d, J = 8.7 Hz), 7.39(2H,d,J=8.7Hz) |

[Table 5]

| | | | |
|---|---|---|---|
| 45 | 14 | 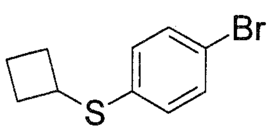 | NMR2:1.96-2.11 (4H, m), 2.40-2.50 (2H, m), 3.81-3.89 (1H, m), 7.10 (2H, d, J = 8.4 Hz), 7.38 (2H, d, J = 8.4 Hz) |
| 46 | 14 | 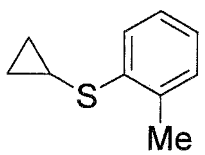 | NMR2:0.66-0.73 (2H, m),1.06-1.14 (2H,m), 2.08-2.17 (1H, m), 2.26 (3H, s), 7.02-7.24 (3H, m), 7.53 (1H, d, J = 8.1Hz) |
| 47 | 14 | 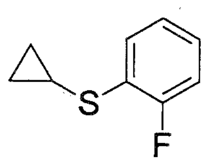 | NMR2:0.66-0.74(2H,m),1.02-1.10 (2H, m), 2.13-2.23 (1H, m), 6.98-7.17 (3H,m), 7.47-7.54 (1H,m) |
| 15 | 15 | 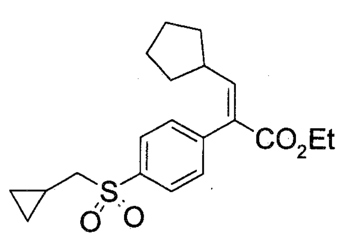 | MS (ESI+): 363 |

(continued)

| | | | |
|---|---|---|---|
| 48 | 15 | 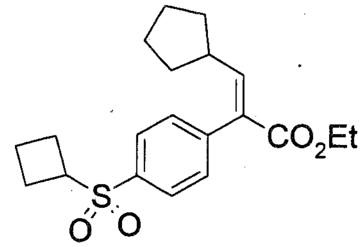 | MS (ESI+): 363 |
| 16 | 16 | 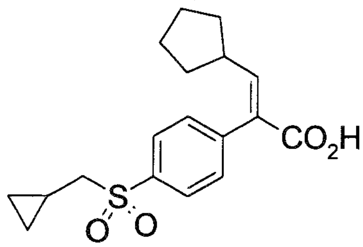 | NMR2:0.15-0.20 (2H,m), 0.55-0.63 (2H,m), 0.98-1.08 (1H, m), 1.40-2.46 (9H, m), 3.05 (2H, d, J = 6.9 Hz), 7.18 (1H, d, J = 10.5 Hz), 7.39 (2H, d, J = 8.4 Hz), 7.94 (2H, d, J = 8.4 Hz) |
| 49 | 16 | 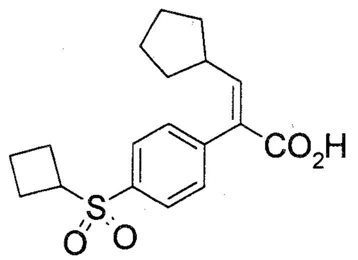 | MS (ESI+): 335 |

[Table 6]

| | | | |
|---|---|---|---|
| 17 | 17 | 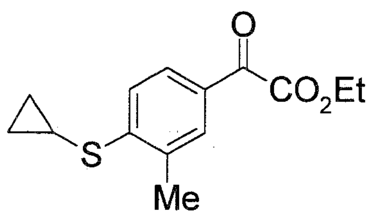 | NMR2:0.71-0.77 (2H,m),1.16-1.24 (2H,m),1.43 (3H,t,J = 7.0Hz),2.11-221 (1H, m), 228 (3H, s), 4.44 (2H, q, J = 7.0 Hz), 7.65 (1H, d, J = 8.4 Hz), 7.73 (1H, d, J = 1.8 Hz), 7.83 (1H, dd, J = 1.8,8.4 Hz) |
| 50 | 17 | 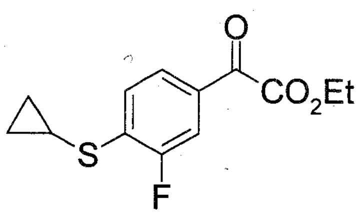 | NMR2:0.72-0.79(2H,m),1.16-1.23 (2H,m),1.43 (3H, t, J = 7.0 Hz), 2.13-2.22 (1H, m), 4.45 (2H, q, J = 7.0 Hz), 7.62-7.70 (2H, m), 7.82 (1H, dd, J = 1.8, 8.4 Hz) |
| 18 | 18 | 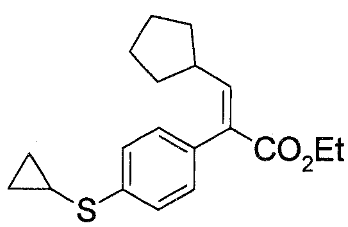 | MS(ESI+):317 |

(continued)

| | | | |
|---|---|---|---|
| 51 | 18 | 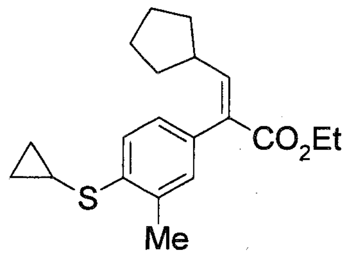 | MS(ESI+):331 |
| 52 | 18 | 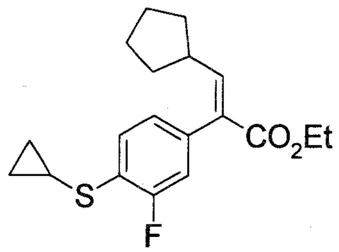 | MS (ESI+): 335 |
| 19 | 19 | 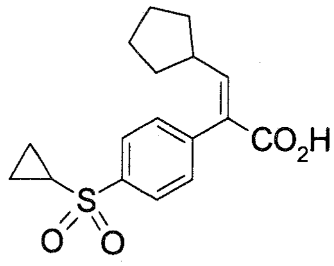 | MS(ESI+): 321 |

[Table 7]

| | | | |
|---|---|---|---|
| 53 | 19 | 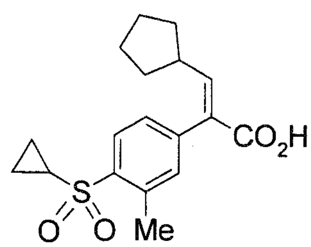 | MS(ESI+):335 |
| 54 | 19 | 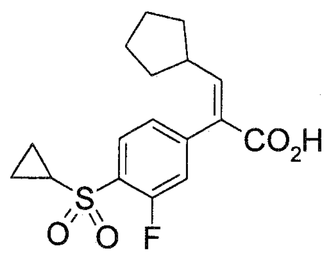 | MS (ESI+): 339 |

(continued)

| | | | |
|---|---|---|---|
| 20 | 20 | 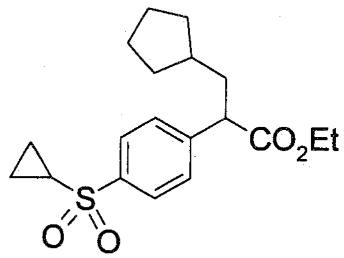 | MS(ESI+):351 |
| 21 | 21 | 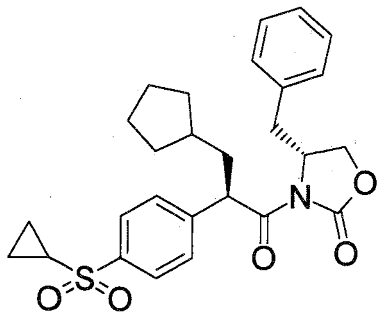 | NMR2: 0.99-1.07 (2H,m),1.10-1.25 (2H,m),1.32-1.40 (2H,m),1.45-1.56 (1H,m), 1.57-1.93 (7H,m), 2.15-2.24 (1H, m), 2.41-2.50 (1H, m), 2.75-2.86 (1H, m), 3.30-3.40 (1H, m), 4.05-4.19 (2H, m), 4.58-4.67 (1H, m), 5.19-5.27 (1H,m), 7.18-7.39 (5H, m), 7.59 (2H, d, J=6.3 Hz), 7.84 (2H,d, J=6.3 Hz) |
| 55 | 21 | 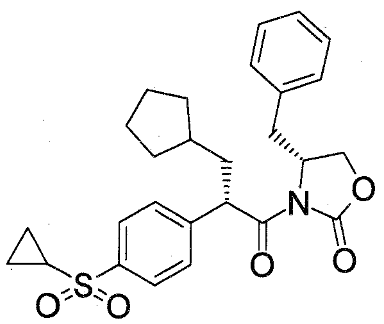 | NMR2:1.14-1.36 (4H,m),1.45-1.56 (2H,m), 1.56-2.08 (8H,m), 2.25-2.37 (1H,m),2.58-2.68 (1H,m),2.70-2.80 (1H,m), 3.18-3.26 (1H,m), 4.22-4.32 (1H,m), 4.35-4.44 (1H,m), 4.84-4.96 (1H,m), 5.28-5.38 (1H, m), 7.06-7.15 (2H,m), 7.30-7.39 (3H,m), 7.79 (2H, d, J = 6.2 Hz), 8.03 (2H,d,J = 6.2 Hz) |

[Table 8]

| | | | |
|---|---|---|---|
| 22 | 22 | 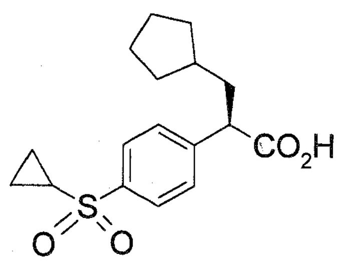 | MS(ESI+):323; $[\alpha]^{23}_D$=-49.1˚(c=1.01) |
| 56 | 22 | 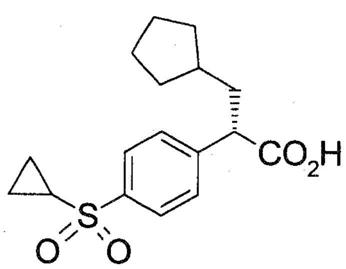 | MS(ESI+):323; $[\alpha]^{23}_D$= +48:5˚(c=1.02) |

(continued)

| | | | |
|---|---|---|---|
| 57 | 13 | 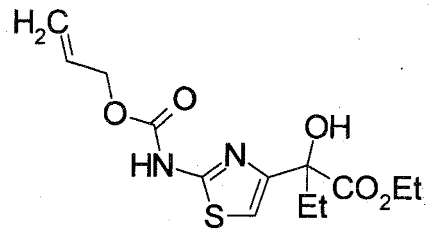 | MS(FAB+):315 |
| 58 | 7 | 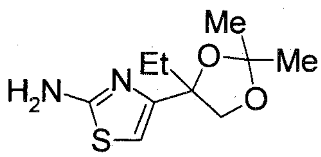 | MS(FAB+):229 |
| 23 | 23 | 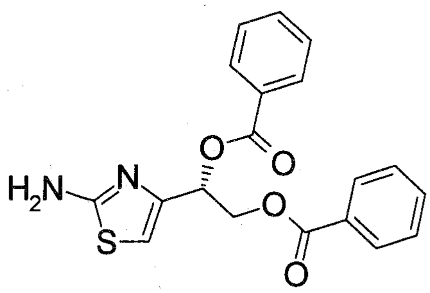 | MS(ESI+):369; $[\alpha]^{242}_{D}$= +31.5°(c=1.00) |
| 59 | 6 | 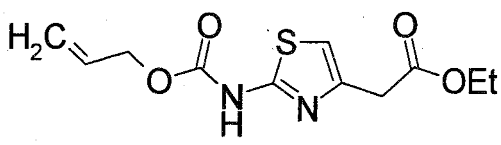 | MS(ESI+):271 |
| 24 | 24 | 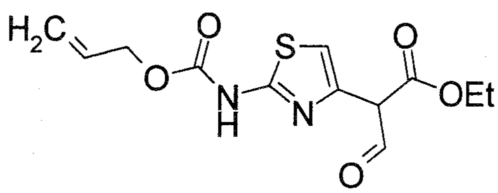 | MS(ESI+):299 |

[Table 9]

| | | | |
|---|---|---|---|
| 25 | 25 | 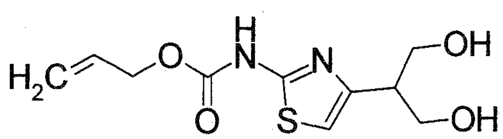 | NMR2: 3.07-3.15 (1H, m), 4.00 (4H, d, J = 5.2 Hz), 4.76(2H, d, J = 5.8 Hz), 5.27-5.46 (2H, m), 5.89-6.02 (1H, m), 6.76 (1H,s) |
| 26 | 26 | 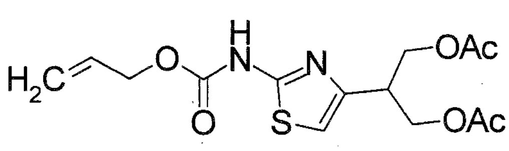 | NMR1:1.98 (6H, s), 3.29-3.40 (1H, m), 4.26 (4H, d, J = 6.5 Hz), 4.63-4.71 (2H,m), 5.21-5.41 (2H, m), 5.88-6.05 (1H, m), 6.98 (1H, s) |
| 27 | 27 | 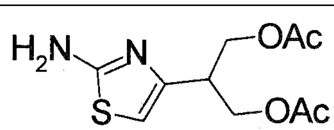 | MS(FAB+):259 |

(continued)

| | | | |
|---|---|---|---|
| 28 | 28 | 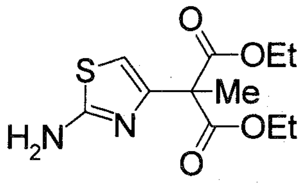 | MS(ESI+):273 |
| 60 | 6 | 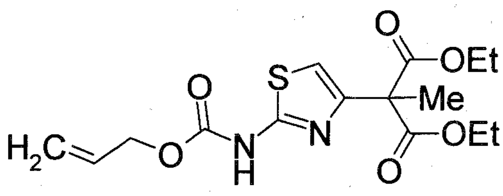 | NMR2:126 (6H, t, J = 7.2 Hz),1.82 (3H, s), 4.17-4.30 (4H, m), 4.73 (2H, d, J = 5.8 Hz), 5.26-5.42 (2H, m), 5.88-6.03 (1H, m), 7.01 (1H,s) |
| 61 | 25 | 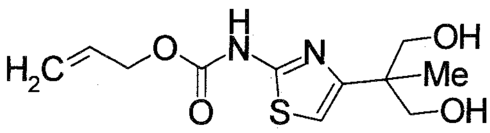 | MS(ESI+): 273 |
| 62 | 26 | 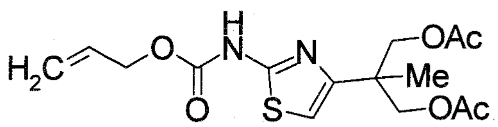 | MS(ESI+): 357 |
| 63 | 27 | 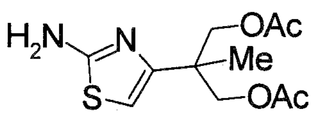 | MS(ESI+): 273 |
| 29 | 29 | 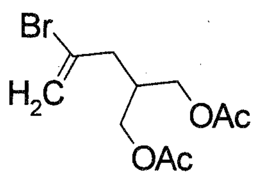 | MS (ESI+): 279 |

[Table 10]

| | | | |
|---|---|---|---|
| 30 | 30 | 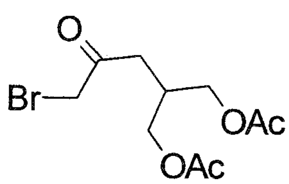 | MS (ESI+): :295 |
| 31 | 31 | 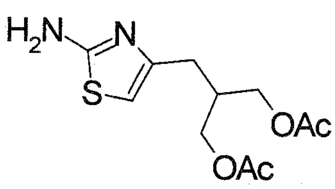 | MS (ESI+) :273 |
| 64 | 6 | 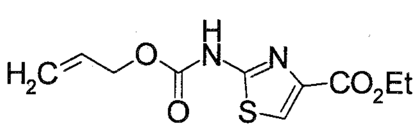 | NMR2:1.39 (3H, t, J = 7.5 Hz), 4.39 (2H, q, J = 6.6 Hz), 4.73-4.76 (2H, m), 525-5.42 (2H, m), 5.88-6.02 (1H, m), 7.82(1H.s) |

(continued)

| | | | |
|---|---|---|---|
| 65 | 16 | 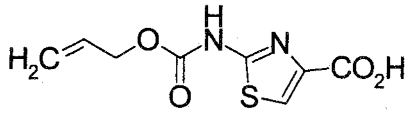 | NMR2: 4.82 (2H, d, J = 5.1 Hz), 5.33-5.48 (2H, m), 5.97-6.10 (1H, m), 7.92 (1H,s) |
| 32 | 32 | 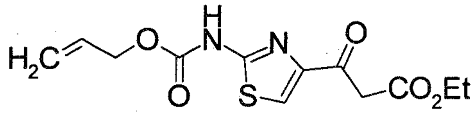 | MS (ESI+) :299 |
| 33 | 33 | 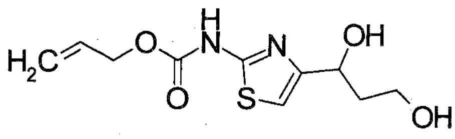 | MS (ESI-):257 |
| 34 | 34 | 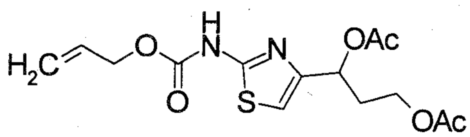 | MS(ESI+):343 |
| 35 | 35 | 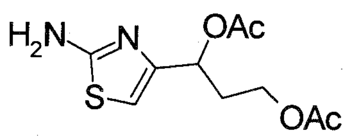 | NMR2:2.04 (3H, s), 2.09 (3H, s), 222-229 (2H, m), 4.00-4.20 (2H, m), 5.05 (2H, br), 5.80 (1H, t, J = 6.6 Hz), 6.46 (1H,s) |
| 36 | 36 | 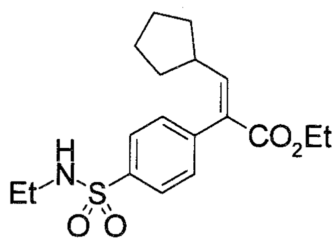 | MS(FAB+):352 |

[Table 11]

| | | | |
|---|---|---|---|
| 66 | 16 | 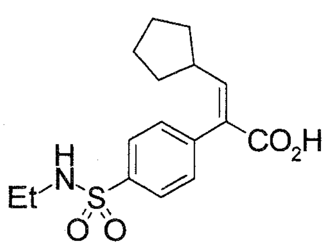 | MS(FAB+):324 |
| 37 | 37 | 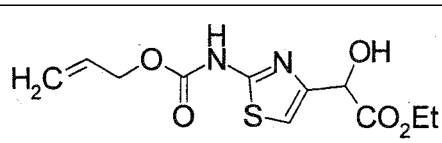 | MS(FAB+):287 |
| 38 | 38 | 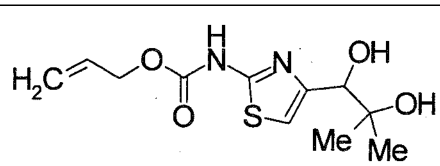 | MS(ESI-):271 |

(continued)

| | | | |
|---|---|---|---|
| 39 | 39 | 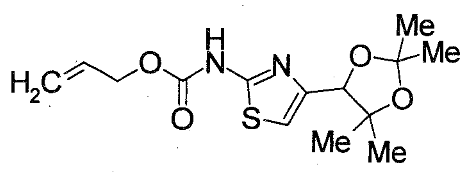 | NMR2:0.88 (3H, s),1.43 (3H, s), 1.46 (3H s),1.54 (3H, s), 4.75 (2H, d, J = 5.7 Hz), 4.89 (1H, s), 5: 29-5.42 (2H, m), 5.90-6.03 (1H, m), 6.94 (1H, s) |
| 67 | 27 | 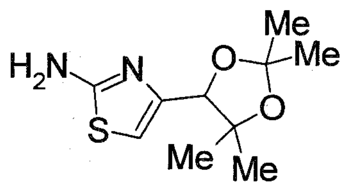 | MS(ESI-):227 |
| 40 | 40 | 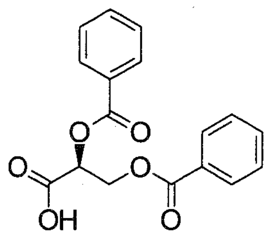 | NMR2:4.78-4.96 (2K m), 5.68-5.80 (1H, m), 7.35-7.69 (6H, m), 7.97-8.16 (4H,m) |
| 41 | 41 | 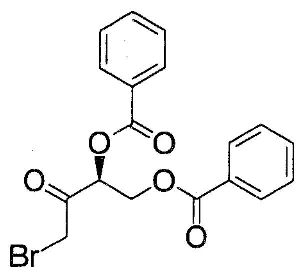 | NMR1: 4.73-4.77 (2H, m), 4.86-4.92 (2H, m), 5.95-6.00 (1H, m), 7.48-7.60 (4H,m), 7.63-7.75 (2H, m), 7.88-7.95 (2H,m), 7.98-8.05 (2H,m) |
| 42 | 42 | 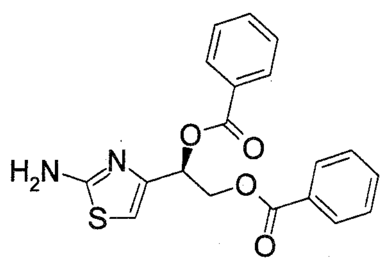 | NMR1:4.70-4.80 (2H, m), 6.20-6.26 (1H,m), 6.70 (1H, s), 7.12 (2H, s), 7.45-7.58 (4H,m), 7.59-7.70 (2H,m), 7.85-7.92 (2H, m), 7.94-8.02 (2H, m); $[\alpha]^{23.9}_{D}$=31.1 (c=1.00) |

[Table 12]

| Ex | Syn | Str | Dat |
|---|---|---|---|
| 1 | 1 | 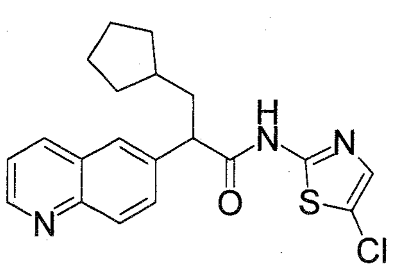 | NMR2:1.00-1.25 (2H,m),1.35-1.85 (7H,m) 1.95-2.10 (1H,m), 2.20-2.40 (1H,m), 3.77 (1H,t, J=7.5Hz),720-7.27 (1H,m), 7.42(1H,dd, J=4.2,8.3 Hz), 7.61 (1H,dd,J=2.0,8.6Hz), 7.65-7.75 (1H,m), 8.06 (1H,d,J = 8.6), 8.11 (1H, d,J = 8.3), 8.88-8.93 (1H,m), 10.00(1H,brs); MS (FAB+):386 |

(continued)

| Ex | Syn | Str | Dat |
|---|---|---|---|
| 16 | 2 | 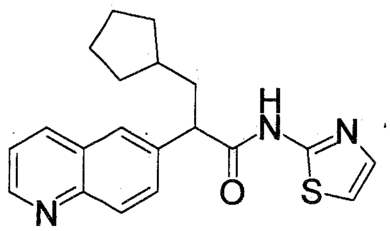 | NMR2:1.00-1.85 (9H,m),1.95-2.10 (1H, m) 2.25-2.40 (1H, m), 3.77 (1H, t, J = 7.6Hz), 7.03 (1H, d, J = 3.6Hz), 7.39 (1H,dd,J=42,8.3),7.50 (1H, d, J=3.6 Hz), 7.65-7.75 (2H, m), 8.02-8.12 (2H, m),8.88(1H,dd,J=1.7,4.2Hz); MS (FAB+): 352 |
| 17 | 1 | 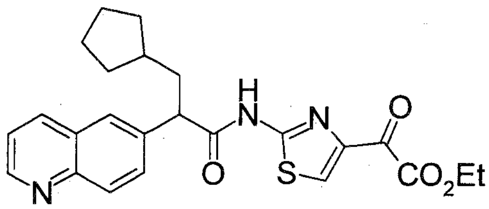 | NMR2:1.00-1.35 (12H,m), 2.00-2.12 (1H, m), 2.22-2.35 (1H, m), 3.80-3.90 (1H,t, J = 7.5Hz), 4.39 (2H, q, J= 7.2Hz), 7.45 (1H, dd, J=4.3,8.2 Hz), 7.61 (1H, dd, J=2.0, 8.7Hz), 7.71 (1H, d, J=1.9 Hz), 8.06-8.16 (2H,m), 8.33 (1H, s), 8.94 (1H, dd, J=1.7,4.2 Hz), 9.28(1H,brs); MS(ESI+): 452 |
| 18 | 3 | 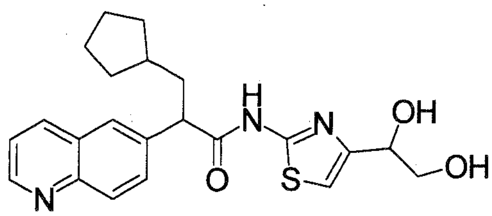 | NMR2:1.00-1.80(9H,m),1.85-2.00 (1H, m) 2.18-2.35 (1H,m), 3.37-3.77 (2H, m), 3.88 (1H,t, J=7.3Hz), 4.57-4.67 (1H, m), 6.59,6.61 (1H, s), 7.22-7.32 (1H, m), 7.58-7.78 (2H, m), 7.92-8.06 (2H, m), 8.69-8.81(2H, m); MS(FAB+):412 |

[Table 13]

| | | | |
|---|---|---|---|
| 19 | 1 | 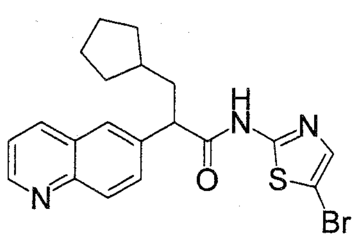 | NMR1 :1.04-1.25 (2H,m),1.35-1.80 (7H,m) 1.82-1:95 (1H,m),2.14-2.28 (1H,m), 4.12 (1H, t, J = 7.4Hz), 7.48-7.60 (2H, m), 7.75-7.83 (1H, m), 7.91-7.96 (1H, m), 7.99 (1H, d, J= 8.6), 8.39 (1H, d, J = 8.4), 8.85-8.91 (1H, m); MS (ESI+): 430, 432 |
| 2 | 2 | 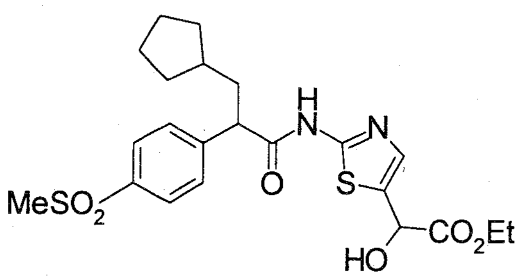 | NMR1:1.05-1.25 (SH,m),1.35-1.80 (8H, m) 2.08-220 (1H, m), 3.17 (3H, s), 4.00-4.13 (3H, m), 5.34-5.37 (1H,m), 6.32-6.36 (1H, m), 7.38 (1H, s), 7.62-7.67 (2H, m), 7.87-7.94 (2H, m); MS (FAB+):481 |

(continued)

| | | | |
|---|---|---|---|
| 3 | 3 | 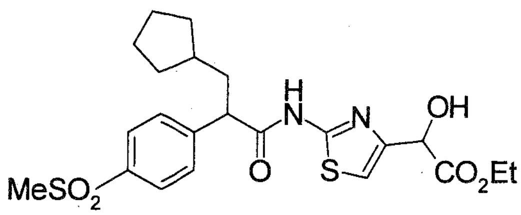 | NMR1:0.82-0.90(1H,m),1.10-120 (3H,m), 1.22-1.28 (1H,m),1.34-1.82 (8H,m), 2.04-2.21 (1H, m), 3.18 (3H, s), 3.98-4.16 (3H, m), 5.07-5.13 (1H, m), 5.98-6.03 (1H, m), 7.13 (1H, s), 7.65 (2H, d, J= 7.8 Hz), 7.91 (2H, d, J = 7.8 Hz); MS(FAB+):481 |
| 4 | 4 | 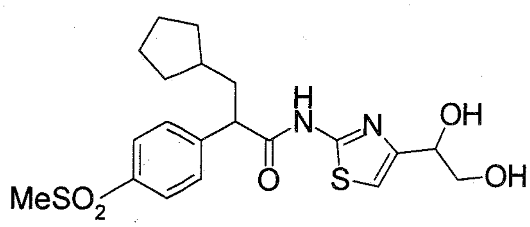 | NMR1:1.04-1.21 (2H,m),1.36-1.83 (8H,m), 2.08-2.22 (1H,m), 3.18 (3H,s), 3.40-3.70 (2H, m), 3.98-4.08 (1H,m), 4.46-4.68 (2H,m), 5.22-527 (1H,m), 6.94 (1H,s), 7.62-7.70 (2H, m), 7.87-7.94 (2H,m); MS(FAB+):439 |
| 5 | 5 | 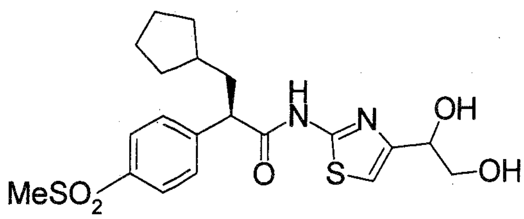 | MS(ESI+):439 |

[Table 14]

| | | | |
|---|---|---|---|
| 20 | 2 | 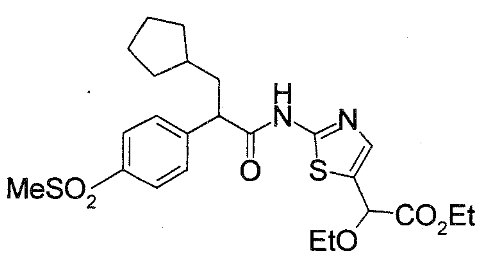 | NMR2 1.00-1.34 (8H, m),1.36-1.97 (8H, m), 2.18-2.32 (1H, m), 3.04 (3H, s), 3.54-3.69 (2H, m), 3.71-3.78 (1H, m), 4.18-4.32 (2H, m), 5.08-5.10 (1H, m), 7.43-7.46 (1H, m), 7.50-7.56 (2H, m), 7.85-7.91 (2H,m); MS (FAB+):509 |
| 21 | 3 | 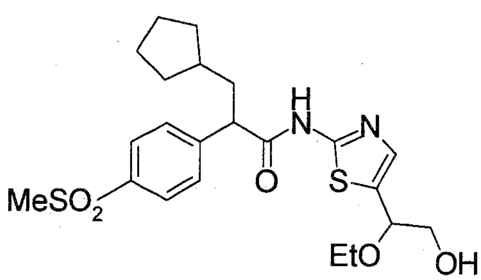 | NMR2:1.06-127 (5H,m), 1.40-1.98 (8H, m), 2.20-2.32 (1H, m), 3.04 (3H, s), 3.38-3.64 (2H, m), 3.70-3.80 (3H, m), 4.614.66 (1H, m), 7.35 (1H, s), 7.51-7.58 (2H, m), 7.86-7.93 (2H, m); MS (ESI+):467 |
| 22 | 3 | 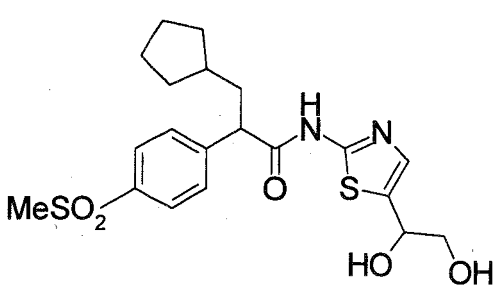 | NMR1:1.04-1.20 (2H,m),1.36-1.85 (8H, m), 2.07-2.20 (1H, m), 3.18 (3H, s), 3.36-3.55 (2H, m), 4.00-4.08 (1H, m), 4.66-4.75 (1H, m), 4.85-4.93 (1H, m), 5.54-5.59 (1H, m), 7.26 (1H, s), 7.61-7.67 (2H, m), 7.83-7.93 (2H, m); MS (ESI+):439 |

(continued)

| | | | |
|---|---|---|---|
| 23 | 3 | 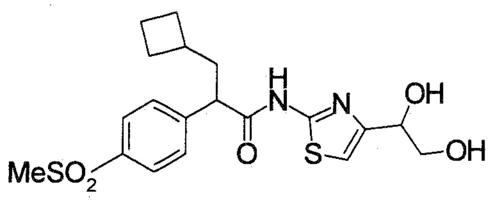 | NMR2:1.50-2.03 (9H, m), 2.09-237 (2H, m), 3.05 (3H, s), 3.62-3.81 (3H, m), 4.69 (1H, brs), 6.73-6.80 (1H, m), 7.55 (2H, d, J = 7.6 Hz), 7.89 (2H, d, J = 7.8 Hz),10.3(1H,brs) ; MS(ESI+):425 |
| 24 | 7 | 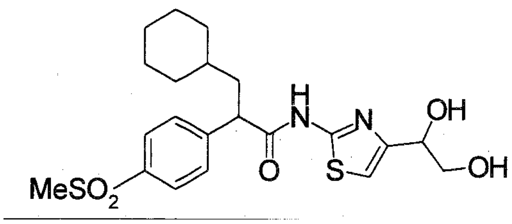 | MS(ESI+):453 |
| 6 | 6 | 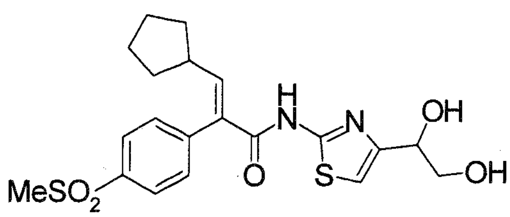 | NMR1:1.4-1.8 (8H,m), 2.3-2.5 (1H, m), 3.26 (3H, s), 3.4-3.5 (1H, m), 3.6-3.7 (1H, m), 4.5-4.7 (2H, m), 5.2-5.3 (1H, m), 6.84 (1H, d, J=10.4 Hz), 6.94 (1H, s), 7.4-7.5 (2H, m), 7.9-8.0 (2H, m), 12.24 (1H,s); MS(FAB+):437 |

[Table 15]

| | | | |
|---|---|---|---|
| 25 | 7 | 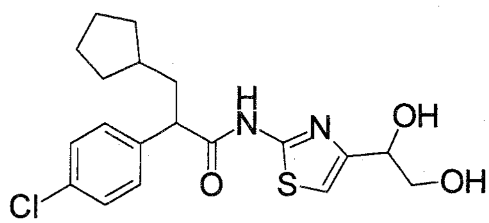 | MS(FAB+):395 |
| 26 | 7 | 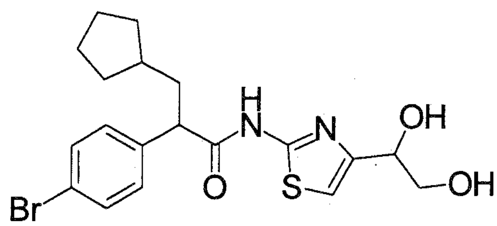 | MS(FAB+):439,441 |
| 27 | 7 | 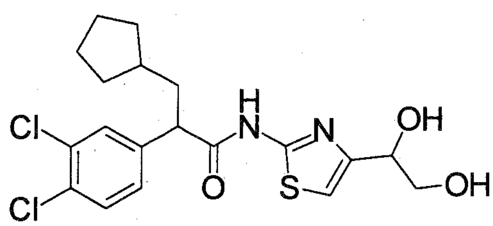 | MS(FAB+): 429 |

(continued)

| | | | |
|---|---|---|---|
| 28 | 7 | 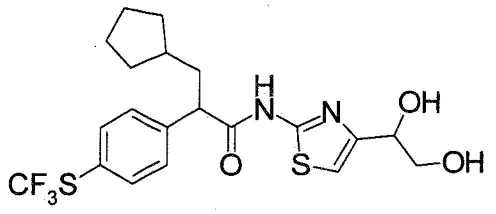 | MS(FAB+):461 |
| 7 | 7 | 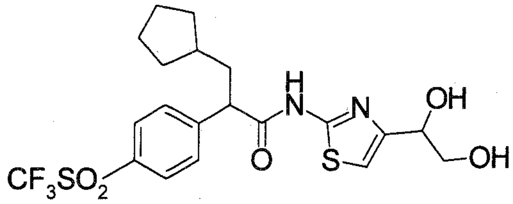 | NMR1:1.02-1.22 (2H,m),1.37-1.81 (8H,m), 2.15-2.23 (1H,m), 3.42-3.50 (1H,m), 3.60-3.70 (1H,m), 4.09-4.14 (1H,m), 4.51-4.60 (1H,m), 4.61-4.64 (1H,m), 5.22-5.24 (1H,m), 6.96 (1H, s), 7.85 (2H, d, J = 8.5 Hz), 8.15 (2H,d, J= 8.5 Hz),12.53 (1H,s); MS(FAB+): 493 |
| 29 | 7 | 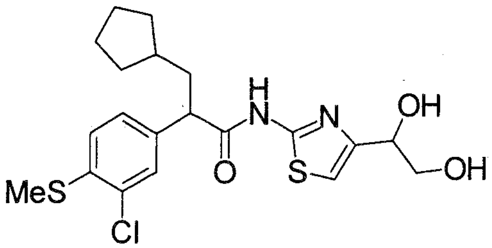 | MS(FAB+):441 |

[Table 16]

| | | | |
|---|---|---|---|
| 30 | 7 | 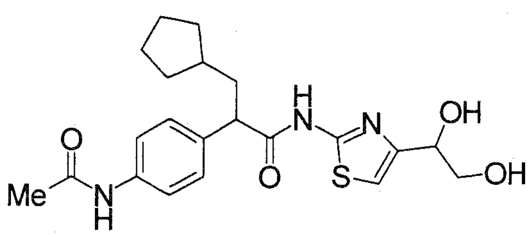 | MS(ESI+):418 |
| 31 | 7 | 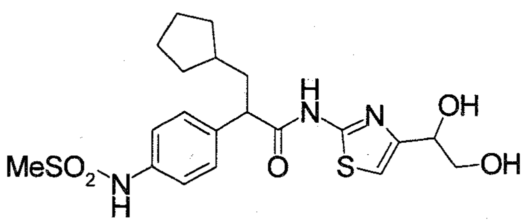 | NMR1:0.99-1.19 (2H,m),1.33-1.81 (8H,m), 1.99-2.11 (1H, m), 2.94 (3H, s), 3.16-3.18 (1H, m), 3.35-3.90 (4H,m), 4.47-4.68 (2H, m), 5.19-5.27 (1H, m), 6.91 (1H, s), 7.13 (2H, d, J = 8.3 Hz), 7.31 (2H,d,J=8.3Hz); MS(ESI-): 452 |
| 32 | 7 | 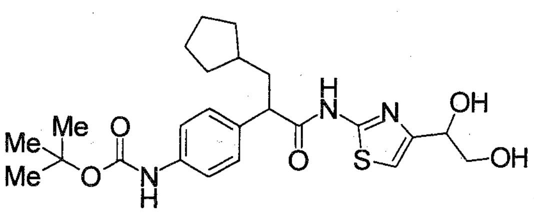 | MS(ESI+):476 |

(continued)

| | | | |
|---|---|---|---|
| 33 | 7 | 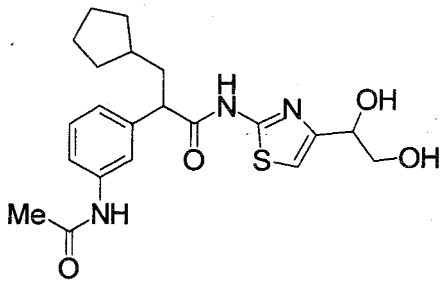 | NMR1:1.98-120 (2H,m),1.28-1.82 (9H,m), 1.95-2.17 (4H,m), 3.37-3.54 (1H, m), 3.55-3.72 (1H, m), 3.76-3.92 (1H, m), 4.44-4.58 (1H, m), 4.58-4.67 (1H, m), 5.17-5.29 (1H, m), 7.03 (1H, d, J= 7.8 Hz), 7.22 (1H, dd, J= 7.8,7.8 Hz), 7.51 (1H, d, J = 8.7 Hz), 7.57 (1H, s), 9.94 (1H,s),12.3 (1H, s); MS(ESI+): 418 |
| 34 | 7 | 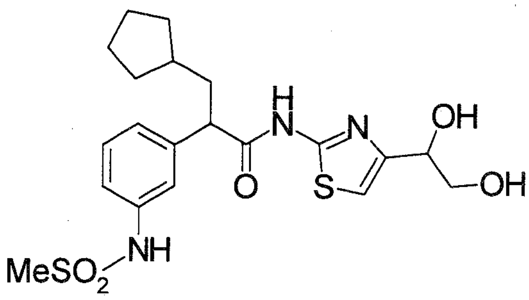 | MS(ESI+):454 |
| 35 | 7 | 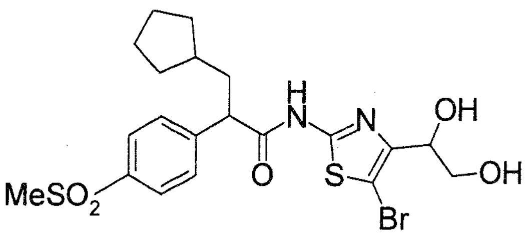 | MS(FAB+):517,519 |

[Table 17]

| | | | |
|---|---|---|---|
| 36 | 7 | 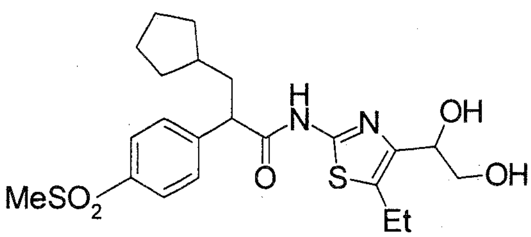 | NMR1:1.02-1.20(5H,m),1.33-1.79 (8H,m), 2.08-2.20 (1H,m), 2.70-2.82 (2H,m), 3.18 (3H, s), 3.43-3.65 (2H,m), 3.95-4.07 (1H,m), 4.46-4.62 (2H,m), 4.89-5.00 (1H,m), 7.64 (2H, d, J = 8.1 Hz), 7.90 (2H,d, J = 8.1 Hz), 12.32 (1H, s); MS(FAB+):467 |
| 37 | 7 | 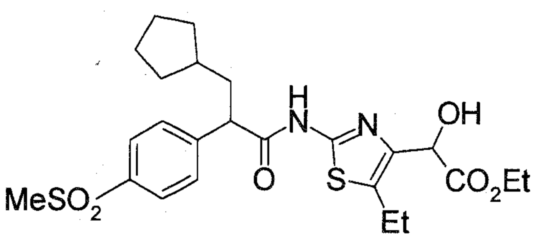 | MS(FAB+):509 |

(continued)

| | | | |
|---|---|---|---|
| 38 | 10 | 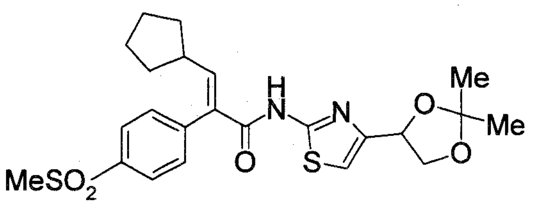 | NMR1:1.37 (3H,s),1.41 (3H,s), 1.42-1.80 (8H, m), 2.34-2.46 (1H,m), 3.26 (3H, s),3.86-3.91 (1H, m), 4.23-4.27 (1H, m), 5.08-5.12 (1H, m), 6.82-6.90 (1H, m), 7.11 (1H, s), 7.49 (2H, d, J = 4.3 Hz), 7.96 (2H, d, J = 4.3 Hz),12.34 (1H, s): MS(FAB+):477 |
| 39 | 7 | 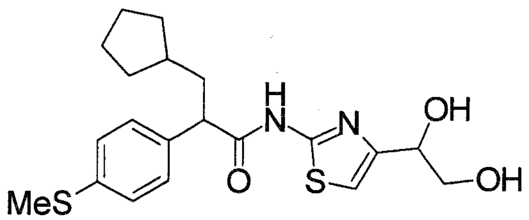 | MS(FAB+): 407 |
| 40 | 7 | 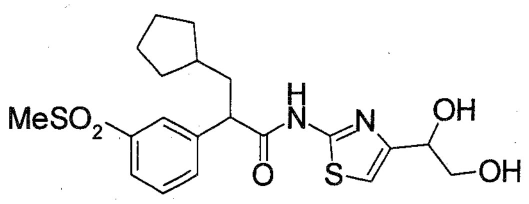 | MS(FAB+):439 |
| 41 | 7 | 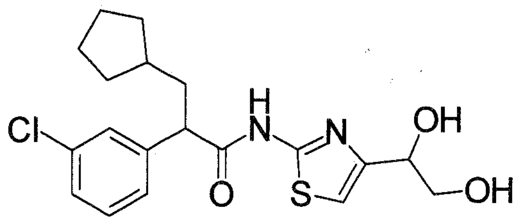 | MS(FAB+):395 |

[Table 14]

| | | | |
|---|---|---|---|
| 42 | 7 | 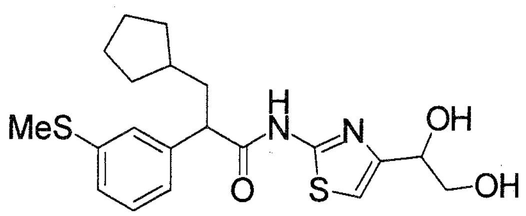 | MS(FAB+): 407 |
| 10 | 10 | 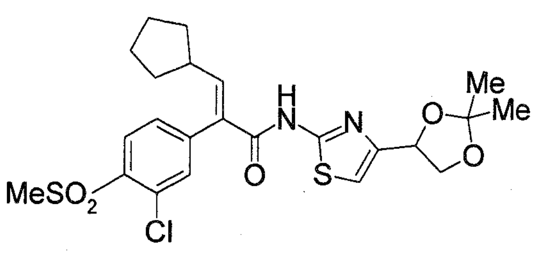 | NMR2:1.40-1.61 (4H,m),1.44 (3H,s), 1.49 (3H, s),1.70-1.80 (4H, m), 223-2.35 (1H,m), 3.38 (3H, s), 3.91-3.98 (1H, m), 4.25-4.32 (1H, m), 5.06-5.12 (1H,m), 6.93 (1H, s), 7.18 (1H, d, J= 10.6Hz), 7.37 (1H,dd, J=1.5,8.1 Hz); 7.47 (1H, d, J=1.5 Hz), 8.27 (1H, d, J = 8.1 Hz), 8.40 (1H, brs); MS(ESI-): 509 |

(continued)

| | | | |
|---|---|---|---|
| 43 | 6 | 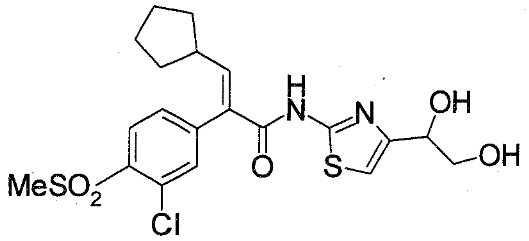 | MS(ESI-):469 |
| 44 | 9 | 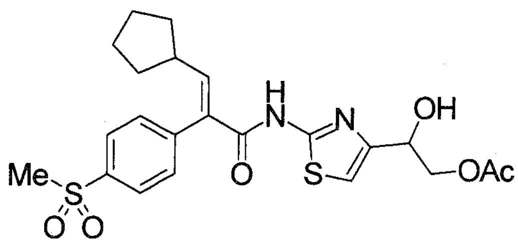 | MS (ESI+):479 |
| 45 | 6 | 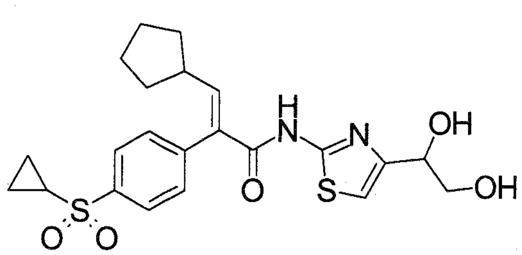 | NMR1: 1.02-1.22 (4H,m),1.37-1.60 (4H, m), 1.61-1.86 (4H, m), 2.35-2.55 (1H,m), 2.86-2.99 (1H, m), 3.41-3.53 (1H, m), 3.59-3.74 (1H, m), 4.50-4.62 (1H, m), 4.62-4.73 (1H, m), 5.21-5.34 (1H, m), 6.84 (1H, d, J=10.3 Hz), 6.95 (1H, s), 7.47 (2H, d, J = 8.3 Hz), 7.92 (2H, d, J = 8.3 Hz); MS (ESI+) :463 |

[Table 19]

| | | | |
|---|---|---|---|
| 8 | 8 | 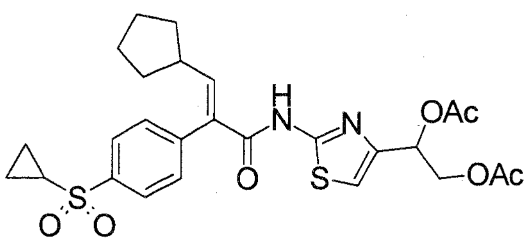 | NMR2:1.12-1.24(2H,m),1.34-1.82 (10H,m), 2.03 (3H,s), 2.10 (3H,s), 2.19-2.37 (1H, m), 2.55-2.68 (1H, m), 4.28-4.45 (2H,m), 6.03 (1H, dd,J=7.5, 4.8 Hz), 6.97(1H,s), 721 (1H,d, J=10.5 Hz), 7.47 (2H,d, J=8.4 Hz), 8.04 (2H,d, J=8.4 Hz), 8.50 (1H, brs) ; MS(ESI+): 547 |
| 9 | 9 | 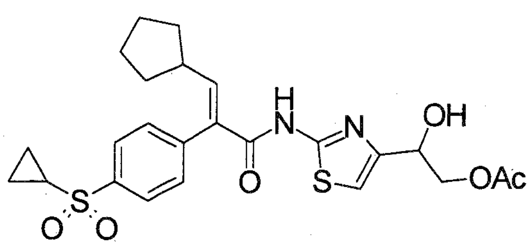 | NMR1:1.02-120(4H,m),1.37-1.82 (8H, m), 2.01 (3H, s), 2.35-2.49 (1H, m), 2.85-297 (1H, m), 4.07 (1H, dd, J = 11.1,7.8Hz),4.32(1H,dd, J=11.1,3.9 Hz), 4.74-4.85 (1H,m), 5.66 (1H,d, J= 5.1 Hz), 6.85 (1H, d, J=10.5 Hz), 7.05 (1H, s), 7.48 (2H, d, J = 8.1 Hz), 7.91 (2H, d, J = 8.1 Hz),12.34 (1H, s); MS(ESI+): 505 |

(continued)

| | | | |
|---|---|---|---|
| 46 | 6 | 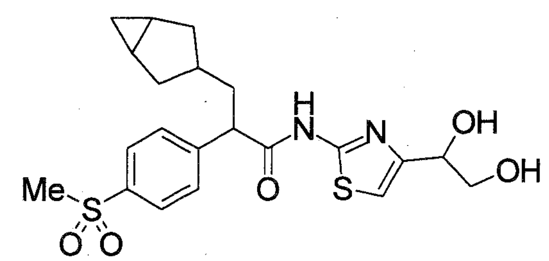 | NMR2: -0.03-0.05(1H, m), 0.19-0.27 (1H,m), 1.18-2.25 (9H, m), 3.06 (3H, s), 3.64-3.85 (3H, m), 4.73 (1H, m), 6.83 (1H, s), 7.56 (2H, d, J=8.1 Hz), 7.91 (2H, d; J= 8.1 Hz) ; MS (ESI+) : 451 |
| 47 | 6 | 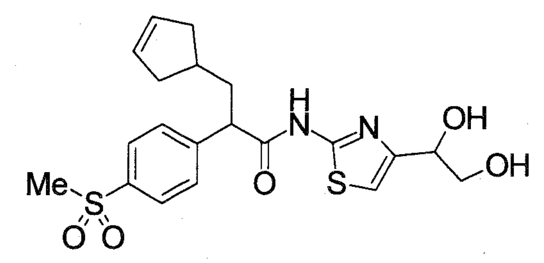 | NMR2 :1.94-2.53 (7H, m), 3.06 (3H, s), 3.67-3.86 (3H, m), 4.704.76 (1H, m), 5.63 (2H, s), 6.70 (1H, s), 6.78-6.83 (1H, m), 7.59 (2H, d, J = 8.4 Hz), 7.91 (2H, d, J=8.4 Hz) ; MS (ESI-): 435 |
| 48 | 7 | 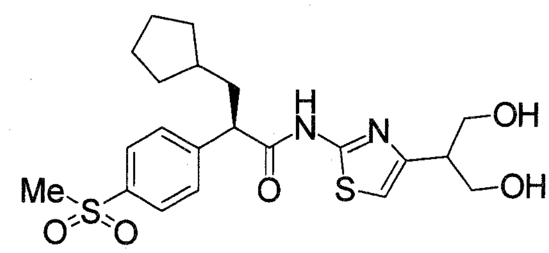 | NMR2:1.06-1.22 (2H,m),1.41-2.00 (8H,m), 2.19-2.31 (1H,m),3.01-3.14 (4H,m), 3.88-4.04 (5H,m), 6.79 (1H,s), 7.66 (2H,d, J = 6.2 Hz), 7.92 (2H,d, J= 6.2Hz); MS(ESI+):453 |

[Table 20]

| | | | |
|---|---|---|---|
| 49 | 6 | 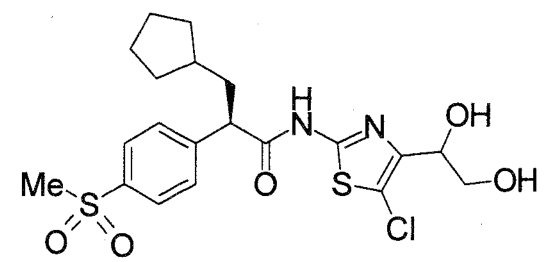 | NMR1:1.03-1.20 (2H, m),1.33-1.85 (8H, m), 2.18-2.22 (1H, m), 3.19 (3H, s), 3.44-3.68 (2H, m), 3.96-4.07 (1H, m), 4.57-4.74 (2H, m), 5.17-5.28 (1H, m), 7.64 (2H, d, J = 8.1Hz), 7.91 (2H,d,J= 8.1 Hz),12.74-12.82 (1H,m); MS (FAB+): 473 |
| 51 | 10 | 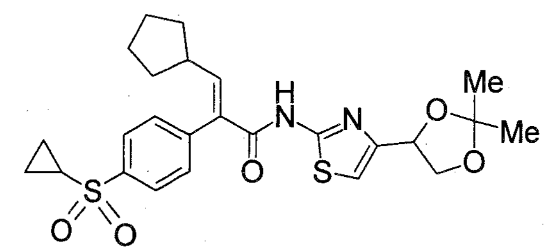 | MS(ESI+):503 |

(continued)

| | | | |
|---|---|---|---|
| 52 | 10 | 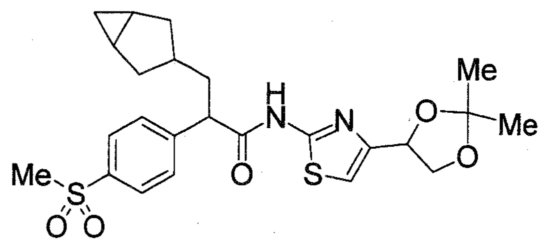 | MS(ESI-): 489 |
| 53 | 10 | 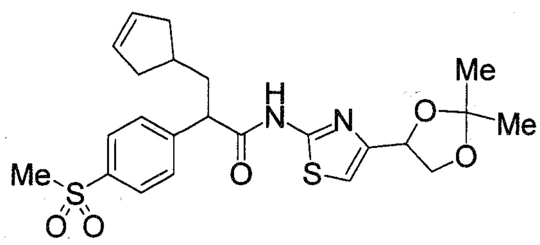 | MS(ESI-):475 |
| 54 | 10 | 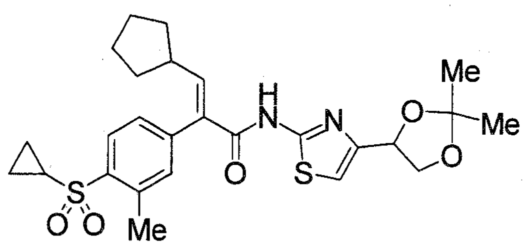 | MS(ESI-):515 |

[Table 21]

| | | | |
|---|---|---|---|
| 55 | 10 | 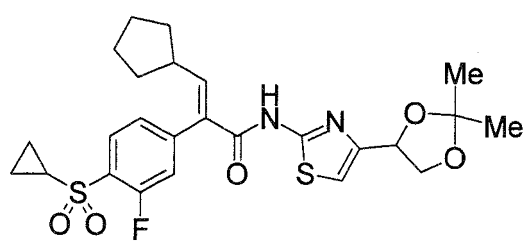 | MS(ESI-):519 |
| 56 | 10 | 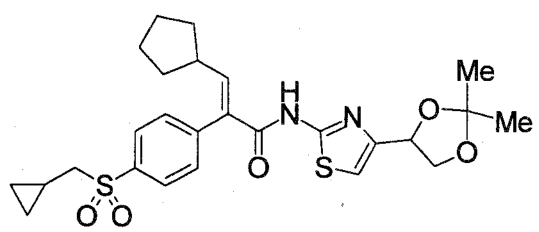 | NMR1:025-0.32 (2H,m), 0.64-0.71 (2H, m), 1.06-1.17 (1H, m),1.43-2.34 (15H,m),3.12 (2H,d,J=72Hz),3.90-3.95 (1H, m), 4.24-4.29 (1H, m), 5.06 (1H, t, J=6.9 Hz), 6.92 (1H, s), 7.19 (1H, d, J=11.4 Hz), 7.48 (2H, d, J=4.8 Hz), 8.06 (2H, d, J=4.8 Hz), 8.39 (1H, br). |

(continued)

| | | | |
|---|---|---|---|
| 57 | 10 | 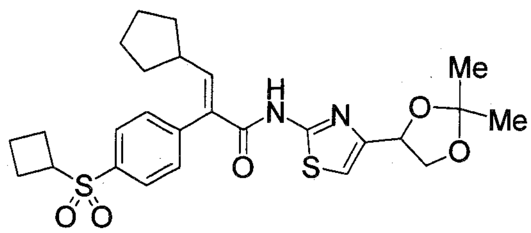 | MS(ESI-): 515 |
| 58 | 10 | 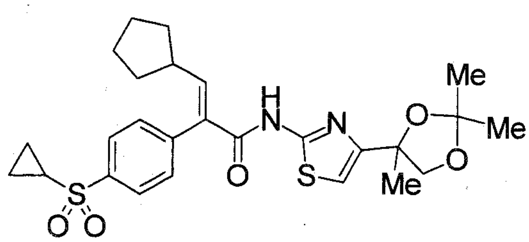 | MS(ESI+):517 |
| 59 | 6 | 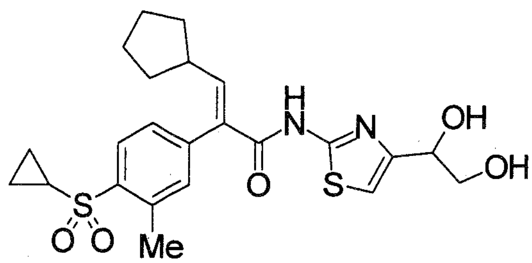 | MS(ESI-):475 |
| 60 | 6 | 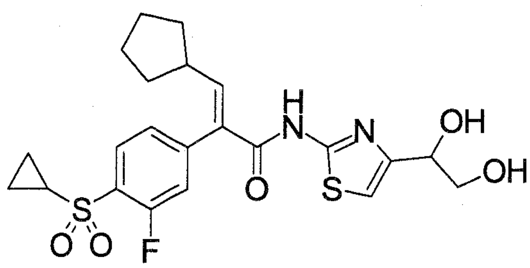 | NMR1:1.11-122 (2H,m),1.38-1.81 (10H,m), 228-2.39 (1H,m), 2.79-2.89 (1H,m), 3.71-3.85 (2H,m), 4.67-4.73 (1H,m), 6.90 (1H,s), 7.13-7.25 (3H,m), 7.98-8.05 (1H,m). MS (ESI-):479 |

[Table 22]

| | | | |
|---|---|---|---|
| 61 | 6 | 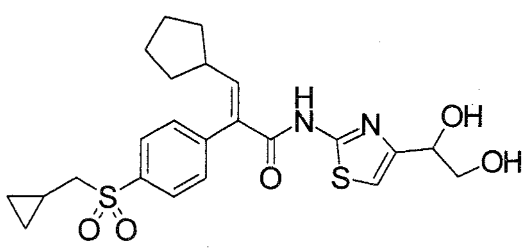 | MS(ESI-):475 |

(continued)

| | | | |
|---|---|---|---|
| 62 | 6 | 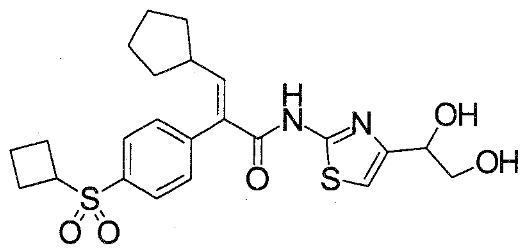 | MS(ESI-): 475 |
| 63 | 6 | 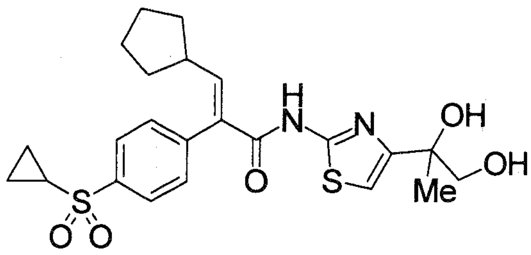 | NMR1 :1.02-123 (4H, m),1.37 (3H, s), 1.42-1.60 (4H,m), 1.61-1.85 (4H,m), 2.33-2.50 (1H, m), 2.83-3.00 (1H, m), 3.44-3.58 (2H, m), 4.58 (1H, dd, J = 5.8, 5.8 Hz), 4.90 (1H, s), 6.82 (1H, d, J = 10.3 Hz), 6.93 (1H, s), 7.48 (2H, d, J = 8.2 Hz), 7.92 (2H, d, J = 82 Hz),12.26 (1H, s); MS(ESI+):477 |
| 64 | 10 | 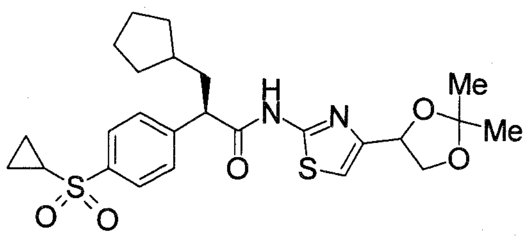 | MS (ESI+): 505 |
| 65 | 10 | 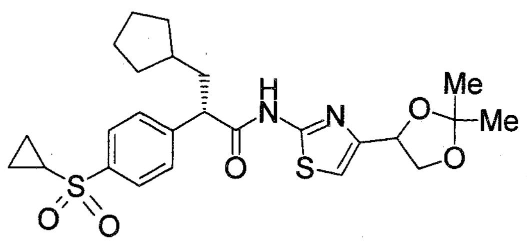 | MS (ESI+): 505 |
| 66 | 6 | 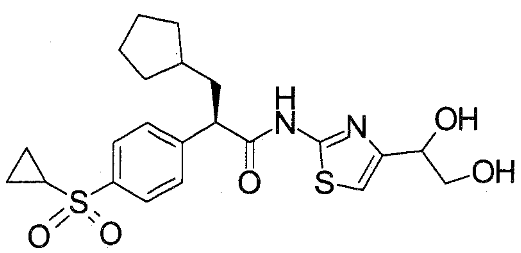 | NMR2:0.98-1.20(5H,m),1.27-1.39 (2H,m), 1.39-1.82(8H,m),1.84-1.97 (1H,m), 2.15-2.30 (1H,m), 2.41-2.52 (1H,m), 3.61-3.83 (2H,m), 3.87-3.98 (1H,m), 4.64-4.81 (1H,m), 6.67-6.78 (1H,m), 7.61 (2H,d, J=6.1Hz), 7.86 (2H,d,J=6.1 Hz); MS (ESI-):463 |

[Table 23]

| | | | |
|---|---|---|---|
| 67 | 6 | 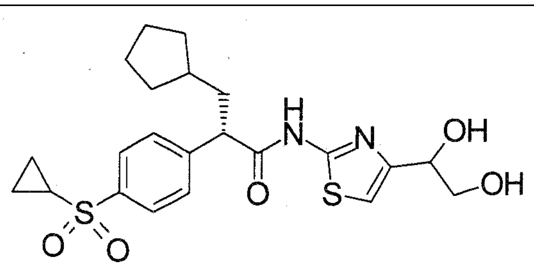 | NMR1: 0.97-1.21 (6H, m), 1.33-1.83 (8H, m), 2.05-224 (1H, m), 2.75-2.89 (1H, m), 3.37-3.53 (1H, m), 3.57-3.71 (1H, m), 3.964.11 (1H, m), 4.48-4.68 (2H,m), 5.19-5.30 (1H,m), 6.95 (1H, s), 7.64 (2H, d, J = 8.3 Hz), 7.87 (2H, d, J = 8.3 Hz), 12.5 (1H, s); MS (ESI-):463 |

(continued)

| | | | |
|---|---|---|---|
| 11 | 11 | 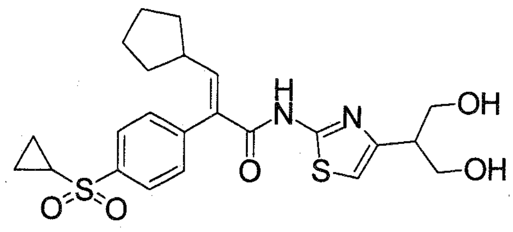 | NMR2:1.03-1.21 (2H, m), 1.35-1.85 (10H, m), 224-2.42 (1H, m), 2.52-2.64 (1H, m), 2.91 (2H, brs), 2.97-3.08 (1H, m), 3.89 (4H, d, J = 5.5 Hz), 6.74 (1H, s), 7.14 (1H, d, J = 10.6 Hz), 7.47 (2H, d, J = 6.6 Hz), 8.02 (2H, d, J = 6.6 Hz), 8.79 (1H,brs); MS(ESI+):477 |
| 12 | 12 | 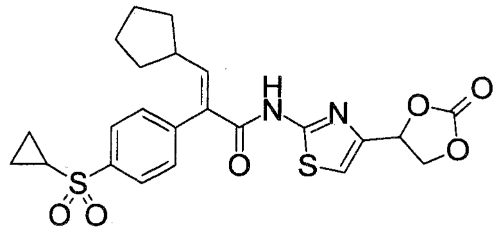 | NMR1:1.05-1.19 (4H,m),1.44-1.56 (4H,m), 1.65-1.80 (4H, m), 2.38-2.48 (1H, m), 2.88-2.97 (1H, m), 4.55 (1H, dd, J = 8.4,6.2 Hz), 4.83 (1H, dd, J = 8.4, 8.4 Hz), 5.87 (1H, dd, J = 8.4,62 Hz), 6.88 (1H, d, J = 10.3 Hz), 7.45-7.51 (3H, m), 7.91 (2H,d,J=82 Hz); MS(ESI+): 489 |
| 68 | 6 | 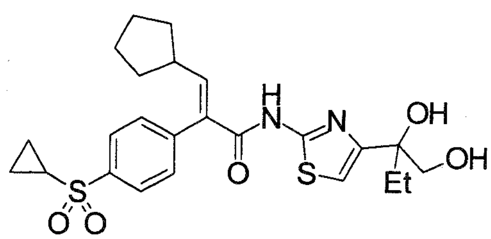 | NMR1 : 0.66 (3H, 1, J = 7.1 Hz), 1.00-1.33 (4H, m), 1.41-1.84(10H,m),2.33-2.48 (1H, m), 2.85-3.00 (1H, m), 3.48-3.63 (2H, m), 4.51 (1H, dd, J = 5.7,5.7 Hz), 4.67 (1H, s), 6.82 (1H, d, J=10.4 Hz), 6.92 (1H, s), 7.48 (2H, d, J = 8.3 Hz), 7.92 (2H, d, J = 8.3 Hz) ; MS (ESI+):491 |

[Table 24]

| | | | |
|---|---|---|---|
| 69 | 11 | 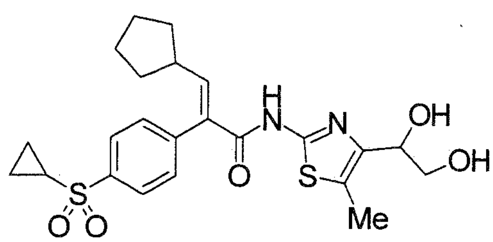 | MS(FAB+):477 |
| 70 | 11 | 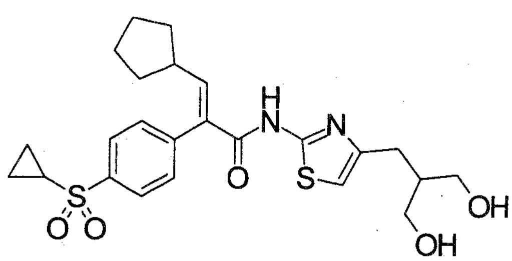 | NMR2:1.11-2.35 (14H,m), 2.54-2.67 (3H,m), 3.55-3.69 (4H,m), 6.62 (1H,s), 7.15 (1H,d, J=11.0 Hz), 7.47 (2H,d,J= 7.5 Hz),8.01 (2H,d, J = 7.5 Hz); MS (ESI+):491 |

(continued)

| | | | |
|---|---|---|---|
| 71 | 11 | 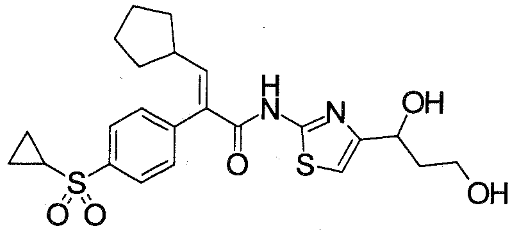 | NMR2: 0.86-2.03 (13H.m), 2.22-2.33 (2H,m), 2.55-2.63 (1H,m), 3.21-3.24 (1H,m), 3.81-3.85 (1H,m), 4.88-4.93 (1H,m), 6.85 (1H,s), 7.20 (1H,d, J = 10.5 Hz), 7.46 (2H,d, J = 8.7 Hz), 8.03 (2H,d,J=8.7Hz); MS(ESI+):477 |
| 72 | 11 | 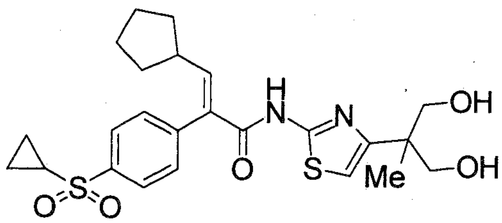 | MS(ESI+):491 |
| 73 | 6 | 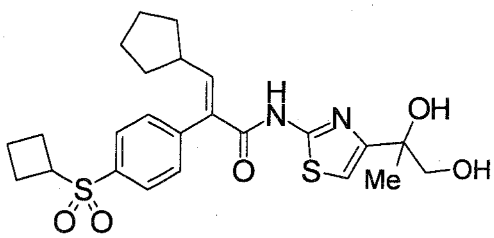 | NMR2 :1.18-3.01 (18H, m), 3.56-3.60 (1H, m), 3.79-3.82 (1H,m),3.89-3.98 (1H, m), 6.89 (1H, s), 7.17 (1H, d, J = 10.2 Hz), 7.46 (2H, d, J=7.8 Hz), 8.00 (2H, d, J=7.8 Hz); MS(ESI-):489 |
| 74 | 6 | 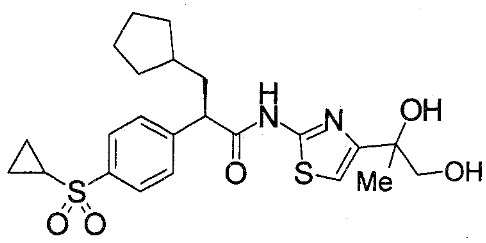 | NMR2:0.87-2.50 (19H, m), 3.54-3.59 (1H, m), 3.76-3.83 (2H, m), 6.79 (1H, s), 7.58 (2H, d, J=7.5 Hz), 7.85 (2H, d, J= 7.5Hz); MS(ESI-):477 |

[Table 25]

| | | | |
|---|---|---|---|
| 13 | 13 | 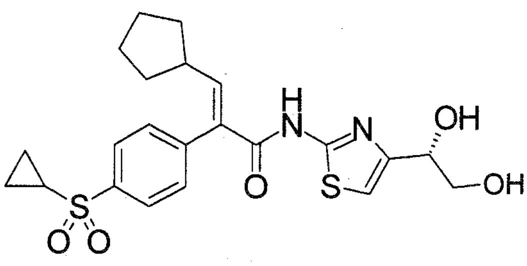 | NMR1:1.00-120(4H,m),1.40-1.83 (8H, m), 2.30-2.47 (1H, m), 2.86-2.98 (1H, m), 3.42-3.51 (1H, m), 3.61-3.72 (1H, m), 4.50-4.60 (1H, m), 4.65 (1H, t, J = 5.88 Hz), 5.25 (1H, d, J = 4.76 Hz), 6.84 (1H, d, J = 10.28 Hz), 6.94 (1H, s), 7.47 (2H, d, J = 8.24 Hz), 7.91 (2H, d, J = 8.24 Hz),12.27 (1H, s); MS(FAB+): 463 |

(continued)

| | | | |
|---|---|---|---|
| 75 | 6 | 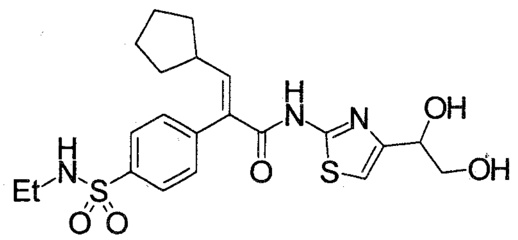 | NMR2:0.86-2.39 (12H, m), 3.05-3.14 (2H, m), 3.43-3.55 (2H, m), 4.54 (1H, br), 5.75-5.77 (1H, m), 6.81 (1H, s), 7.17 (1H, d, J = 10.2 Hz), 7.39 (2H, d, J = 8.1 Hz), 7.90 (2H, d, J = 8.1 Hz); MS (ESI+): 466 |
| 76 | 6 | 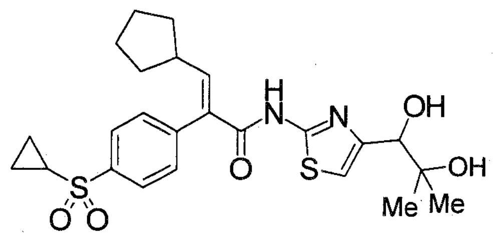 | NMR2:0.85-1.76 (18H, m), 2.26-2.36 (1H, m), 2.54-2.63 (1H, m), 4.29 (1H, s), 6.88 (1H, s), 7.20 (1H, d, J = 102 Hz), 7.45 (2H, d, J = 8.4 Hz), 8.02 (2H, d, J= 8.4 Hz); MS (ESI-): 489 |
| 77 | 11 | 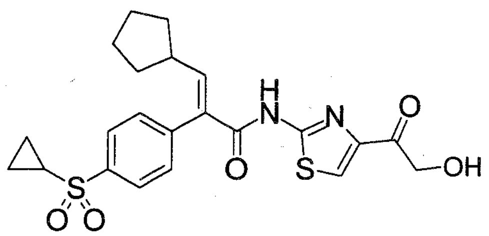 | NMR2:1.12-1.78 (13H, m), 2.26-2.38 (1H, m), 2.58-2.67 (1H, m), 4.80 (2H, s), 7.20 (1H, d, J=10.5 Hz), 7.48 (2H, d, J = 7.8 Hz), 8.05 (2H, d, J=7.8 Hz), 9.10 (1H, br); MS (ESI+):461 |
| 78 | 10 | 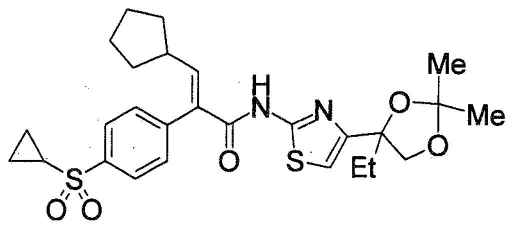 | MS (ESI+):531 |
| 79 | 10 | 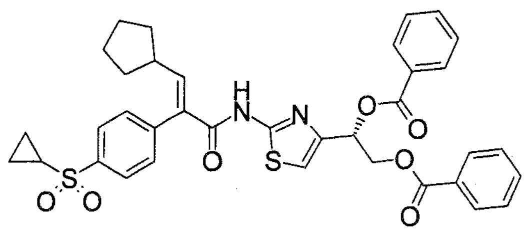 | MS(ESI+):671 |

[Table 26]

| | | | |
|---|---|---|---|
| 80 | 10 | 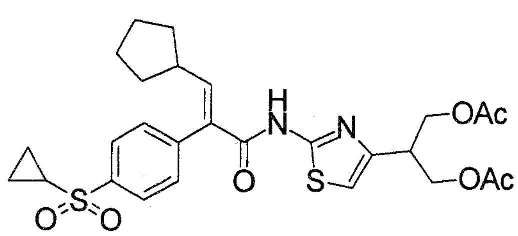 | MS(ESI+):561 |

(continued)

| | | | |
|---|---|---|---|
| 81 | 10 | 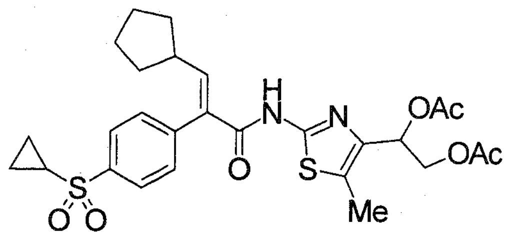 | MS(FAB+):561 |
| 82 | 10 | 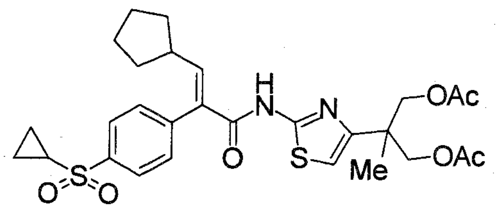 | MS(ESI+):575 |
| 83 | 10 | 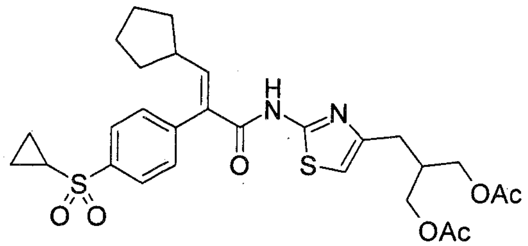 | MS (ESI+): 575 |
| 84 | 10 | 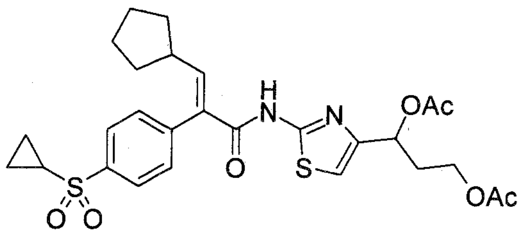 | NMR2 :1.16-2.31 (21H,m),2.57-2.65 (1H, m), 3.94-4.16 (2H, m), 5.87-5.91 (1H, m), 6.92 (1H, s), 7.20 (1H, d, J = 10.8 Hz), 7.46 (2H, d, J = 8.4 Hz), 8.03 (2H, d, J=8.4 Hz), 8.48 (1H, br) |
| 85 | 10 | 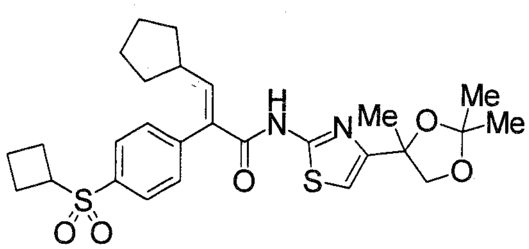 | MS (ESI+) : 531 |

[Table 27]

| | | | |
|---|---|---|---|
| 86 | 10 | 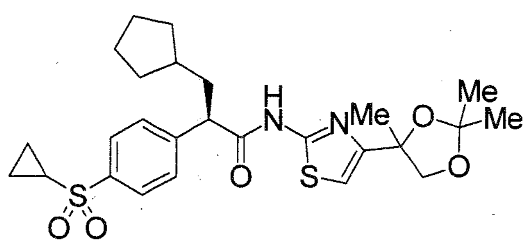 | NMR2:0.85-2.49 (25H, m), 3.71-3.76 (1H,m), 3.92-3.97 (1H,m), 4.10-4.22 (1H,m), 6.89 (1H, s), 7.51-7.57 (2H,m), 7.85-7.88 (2H,m), 9.46 (1H,br) |

(continued)

| | | | |
|---|---|---|---|
| 87 | 10 | 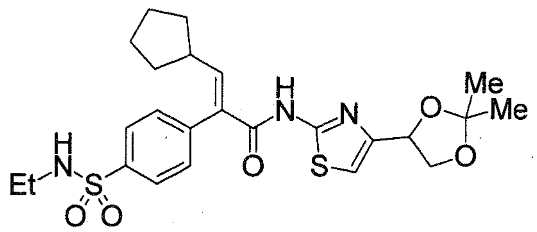 | MS (ESI+):506 |
| 14 | 14 | 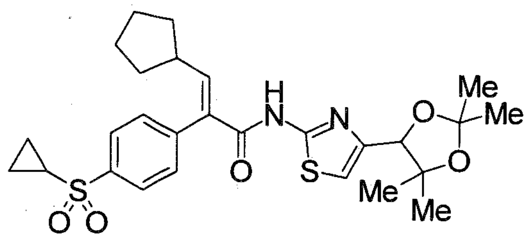 | MS(ESI-):529 |
| 15 | 15 | 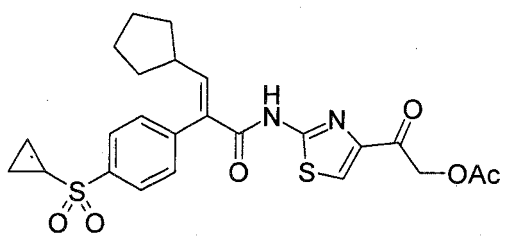 | MS (ESI+):503 |
| 88 | 13 | 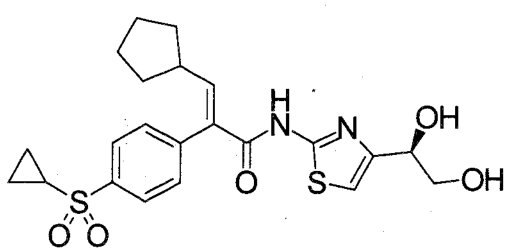 | NMR2 :1.03-1.20 (2H,m), 1.35-1.87 (10H, m), 2.20-2.40 (1H, m), 2.54-2.94 (2H,m), 3.58-3.87 (3H,m), 4.54-4.64 (1H,m), 6.87 (1H,s), 7.16 (1H,d,J= 10.7 Hz), 7.45 (2H, d, J = 8.28 Hz), 8.00 (2H, d, J = 8.28 Hz); MS (ESI+) : 463 ; $[\alpha]_D^{239}$=-16.9˚(c=0.75) |
| 89 | 10 | 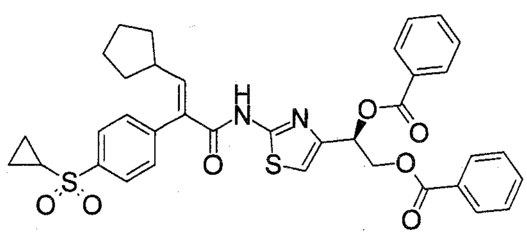 | NMR2:1.10-1.22 (2H,m),1.30-1.80 (10H,m), 2.20-2.40 (1H,m), 2.52-2.66 (1H,m), 4.74-4.82 (2H,m), 6.39-6.46 (1H,m), 7.09 (1H,s), 7.20 (1H,d, J = 10.7 Hz), 7.35-7.58 (8H,m), 7.92-8.10 (6H,m) |

[Table 28]

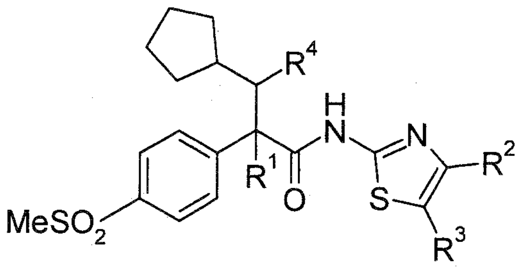

| No | $R^1$ | $R^2$ | $R^3$ | $R^4$ |
|---|---|---|---|---|
| 1 | F | H | H | H |
| 2 | F | 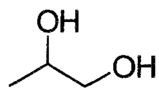 | H | H |
| 3 | F | H | Me | H |
| 4 | F | H | Cl | H |
| 5 | F | H | F | H |
| 6 | H | Me | 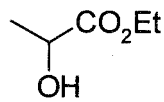 | F |

[Table 29]

| No | Str | No | Str |
|---|---|---|---|
| 7 | 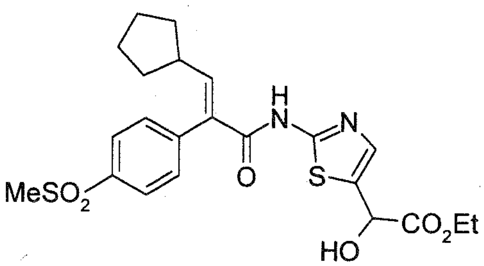 | 8 | 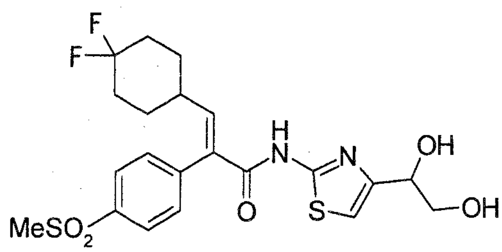 |
| 9 | 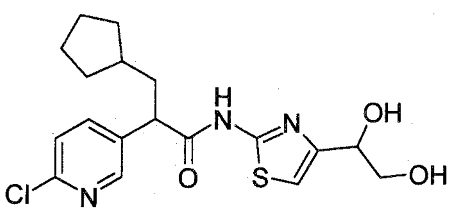 | 10 | 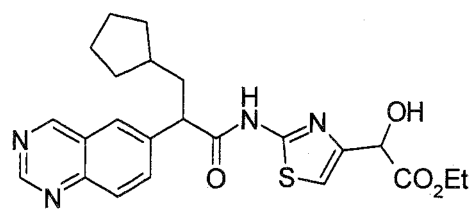 |

(continued)

| No | Str | No | Str |
|---|---|---|---|
| 11 | 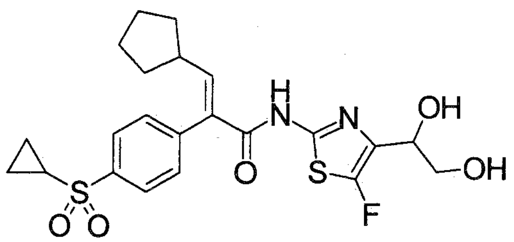 | 12 | 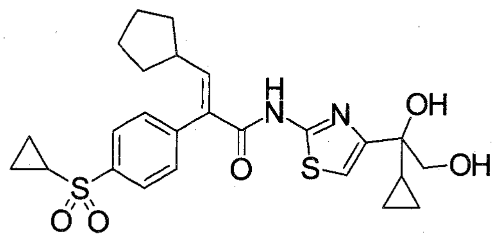 |
| 13 | 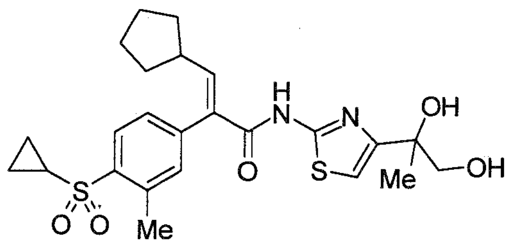 | 14 | 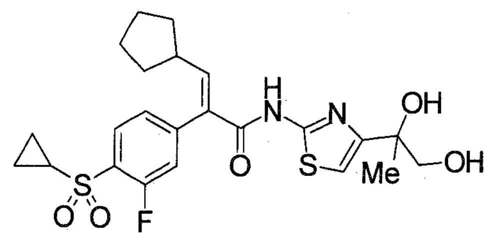 |
| 15 | 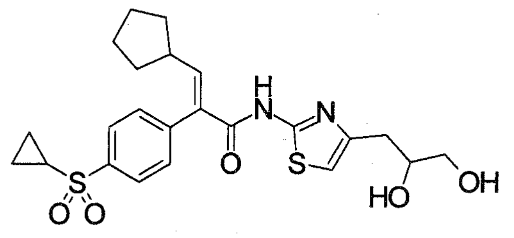 | 16 | 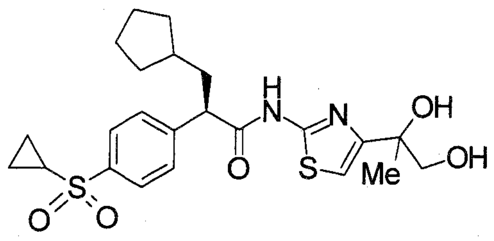 |
| 17 | 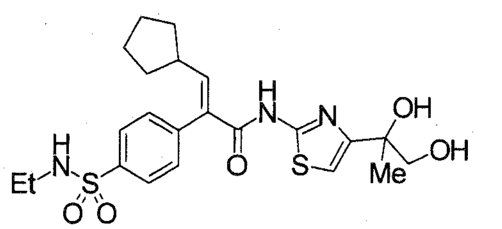 | 18 | 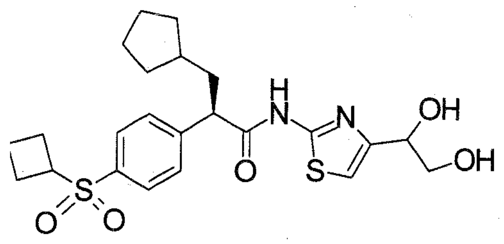 |
| 19 | 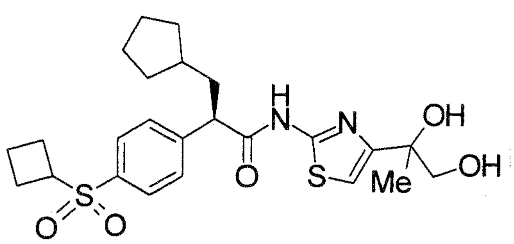 | | |

[Table 30]

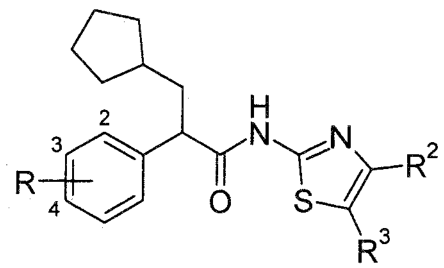

| No | R | $R^2$ | $R^3$ |
|---|---|---|---|
| 20 | 3-$MeSO_2$- | 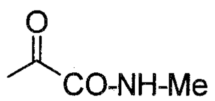 | H |
| 21 | 4-$MeSO_2$- | | H |
| 22 | 4-$MeSO_2$- | | Me |
| 23 | 3-$MeSO_2$- | 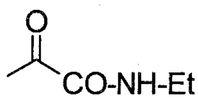 | H |
| 24 | 4-$MeSO_2$- | | H |
| 25 | 4-$MeSO_2$- | | Me |
| 26 | 3-$MeSO_2$- | 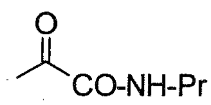 | H |
| 27 | 4-$MeSO_2$- | | H |
| 28 | 4-$MeSO_2$- | | -$CH_2OMe$ |
| 29 | 3-F-4-$MeSO_2$- | 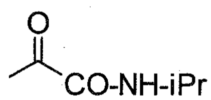 | H |
| 30 | 4-$MeSO_2$- | | H |
| 31 | 4-$MeSO_2$- | | Cl |
| 32 | 3-$MeSO_2$- | 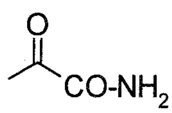 | H |
| 33 | 4-$MeSO_2$- | | H |
| 34 | 3-$CF_3$-4-$MeSO_2$- | | H |
| 35 | 3-$MeSO_2$- | 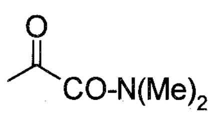 | H |
| 36 | 4-$MeSO_2$- | | H |
| 37 | 3-Cl-4-$MeSO_2$- | | H |
| 38 | 4-MeS- | 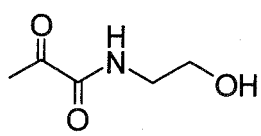 | H |
| 39 | 4-MeSO- | | H |
| 40 | 4-$MeSO_2$- | | H |
| 41 | 3-$NO_2$-4-$MeSO_2$- | 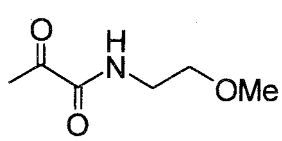 | H |
| 42 | 4-$MeSO_2$- | | H |
| 43 | 4-$CF_3SO_2$- | | H |
| 44 | 4-$MeSO_2$- | 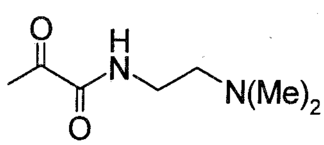 | H |
| 45 | 4-$EtSO_2$- | | H |
| 46 | 4-$iPrSO_2$- | | H |

[Table 31]

| | | | |
|---|---|---|---|
| 47 | 4-MeSO2-O- | 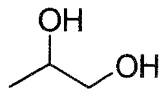 | H |
| 48 | 4-NC- | | H |
| 49 | 4-$F_3$C- | | H |
| 50 | 4-MeS- | | H |
| 51 | 4-$MeSO_2$-O- | 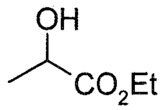 | H |
| 52 | 4-NC- | | H |
| 53 | 4-$CF_3$CO- | | H |
| 54 | 4-$MeSO_2$- | 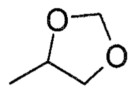 | H |
| 55 | 4-$MeSO_2$- | | Me |
| 56 | 4-$MeSO_2$- | 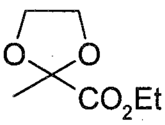 | H |
| 57 | 4-$MeSO_2$- | | Me |
| 58 | 4-$MeSO_2$- | 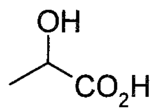 | H |
| 59 | 4-$MeSO_2$- | | Me |
| 60 | 4-$MeSO_2$- | 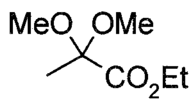 | H |
| 61 | 4-$MeSO_2$- | | Me |
| 62 | 4-$MeSO_2$- | 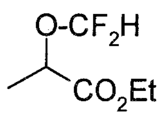 | H |
| 63 | 4-$MeSO_2$- | | Me |
| 64 | 4-$MeSO_2$- | 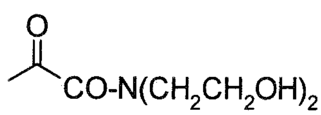 | H |
| 65 | 4-$MeSO_2$- | | Me |
| 66 | 4-$MeSO_2$- | H | 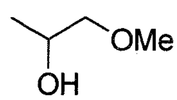 |
| 67 | 4-$MeSO_2$- | Me | |

INDUSTRIAL APPLICABILITY

Since the compound of the present invention has a GK activation action, it is useful as a therapeutic and preventive agent for diabetes, particularly type II diabetes. It is also useful as a therapeutic and preventive agent for complications of diabetes including nephropathy, retinopathy, neuropathy, disturbance of peripheral circulation, cerebrovascular accidents, ischemic heat disease and arteriosclerosis. In addition, it is also useful as a therapeutic and preventive agent for obesity and metabolic syndrome by suppressing overeating.

SEQUENCE LISTING FREE TEXT

Explanation of "Artificial Sequence" is described in the numerical index <223> of the following SEQUENCE LISTING. Illustratively, the nucleotide sequences represented by SEQ ID NOs:1 and 2 of the SEQUENCE LISTING are artificially synthesized primer sequences.

## Claims

1. A thiazole derivative represented by the following general formula (I) or a pharmaceutically acceptably salt thereof

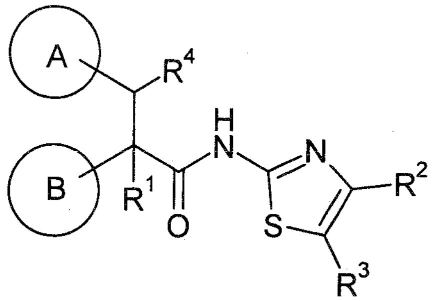

(I)

(symbols in the formula have the following meanings;

A: cycloalkyl or cycloalkenyl which may respectively be substituted,
B: a group selected from phenyl, pyridyl, quinolyl, isoquinolyl, quinoxalinyl, quinazolinyl and cinnolinyl, which may be substituted with 1 or 2 substituent groups,
$R^1$: -H, halogen or -$R^0$,
$R^4$: -H, -OH or halogen,
or $R^1$ and $R^4$ together form a bond,
$R^2$ and $R^3$: the same or different from each other, and each is a group selected from the following (i) or (ii),

(i): -CH($OR^A$)-$R^B$, -CO-CO-$NR^CR^D$, -CO-CO-$NR^C$-$OR^D$, -CO-lower alkylene-$OR^E$, -C($OR^E$)($OR^F$)-$R^B$, -C($OR^E$)($OR^F$)-$R^0$, -C($R^G$)($OR^E$)-CH($OR^F$)-$R^C$, -C($R^G$)($OR^E$)-C($R^0$)($OR^F$)-$R^C$, -CH($OR^E$)-CH($OR^F$)-$R^B$, -C($R^G$)($OR^E$)-lower alkylene-$OR^F$, -CH($CH_2OR^E$)-$CH_2OR^F$, -C($R^G$)($CH_2OR^E$)-$CH_2OR^F$, -lower alkylene-C($R^G$)($OR^E$)-CH($OR^F$)-$R^C$, -lower alkylene-C($R^G$)($OR^E$)-C($R^0$)($OR^F$)-$R^C$, -lower alkylene-CH($CH_2OR^E$)-$CH_2OR^F$ and/or lower alkylene-C($R^G$)($CH_2OR^E$)-$CH_2OR^F$,
(ii): -H, -halogen, -$NO_2$, -CN, -R°, -CO-$CO_2$H, -CO-CO-$OR^0$, -halogeno lower alkyl, -lower alkylene-$OR^A$ and/or -lower alkylene-$NR^CR^D$,

$R^A$: the same or different from each other and each represents -H, -$R^0$, -halogeno lower alkyl or -lower alkylene-aryl,
$R^B$: -$CO_2$H, -$CO_2R^0$, -CO-$NR^CR^D$, -CO-$NR^C$-$OR^D$, -lower alkylene-$NR^CR^D$, -lower alkylene-$OR^A$, -lower alkylene-$CO_2R^0$, -lower alkylene-CO-$NR^CR^D$ or -lower alkylene-CO-$NR^C$-$OR^D$,
$R^C$ and $R^D$: the same or different from each other and each represents -H, -$R^0$, -lower alkylene-N($R^A$)$_2$, -lower alkylene-$OR^A$, -lower alkylene-$CO_2$H, -lower alkylene-$CO_2R^0$ or -lower alkylene-CO-N($R^A$)$_2$,
$R^E$ and $R^F$: the same or different from each other and each represents a group described in $R^A$, -C(O)-$R^0$ or -C(O)-aryl, or $R^E$ and $R^F$ together form lower alkylene or -C(O)-,
$R^G$: H, -$R^0$ or cycloalkyl and
$R^0$: the same or different from each other and each represents lower alkyl, with the proviso that, when 1) B is phenyl or pyridyl which may be substituted and also 2)
$R^1$ is H or $R^1$ and $R^4$ together form a bond, at least one of $R^2$ and $R^3$ is a group selected from (i).

2. The compound described in claim 1, wherein $R^1$ and $R^4$ are both H, or $R^1$ and $R^4$ together form a bond.

3. The compound described in claim 2, wherein A is a $C_{5-6}$ cycloalkyl.

4. The compound described in claim 3, wherein B is phenyl substituted with 1 or 2 substituent groups selected from the group consisting of -$R^0$, halogeno lower alkyl, halogen, -$OR^0$, -CN, -$NO_2$ -CHO, -$CO_2$H, -$CO_2R^0$, -CO-$R^0$, -CO-hydrocarbon ring, -CO-hetero ring, -$SO_2R^0$, -$SO_2$-halogeno lower alkyl, -$SO_2$-hydrocarbon ring and -$SO_2$-hetero ring.

5. The compound described in claim 4, wherein one of $R^2$ and $R^3$ is H, lower alkyl or halogen and the other is -CH($OR^A$)-$R^B$, -C($OR^E$)($OR^F$)-$R^B$, -C($OR^E$)($OR^F$)-$R^0$, -C($R^G$)($OR^E$)-CH($OR^F$)-$R^C$, -lower alkylene-C($R^G$)($CH_2OR^E$)-$CH_2OR^F$ or -CO-lower alkylene-$OR^E$.

6. The compound described in claim 5, wherein B is phenyl which is substituted with one substituent group selected from the class consisting of -$SO_2R^0$, -$SO_2$-halogeno lower alkyl and -$SO_2$-cycloalkyl and which may be further substituted with one substituent group selected from the class consisting of -$R^0$ and halogen.

7. The compound described in claim 6, wherein one of $R^2$ and $R^3$ is H and the other is $-CH(OH)-CH_2OH$, $-C(R^0)(OH)-CH_2OH$, $-CH(OR^0)-CH_2OH$, $-CH(OR')-CH_2OR^0$, $-CH_2-CH(CH_2OH)-CH_2OH$ or $-CO-CH_2OH$.

8. The compound or a pharmaceutically acceptable salt thereof described in claim 1, which is selected from the group consisting of
(2E)-3-cyclopentyl-N-[4-(1,2-dihydroxyethyl)-1,3-thiazol-2-yl]-2-[4-(methylsulfonyl)phenylacrylamide,
(2E)-3-cyclopentyl-2-[4-(cyclopropylsulfonyl)phenyl]-N-[4-(1,2-dihydroxyethyl)-1,3-thiazol-2-yl]acrylamide,
(2R)-3-cyclopentyl-2-[4-(cyclopropylsulfonyl)phenyl]-N-[4-(1,2-dihydroxyethyl)-1,3-thiazol-2-yl]propanamide,
(2E)-3-cyclopentyl-2-[4-(cyclopropylsulfonyl)phenyl]-N-[4-(1,2-dihydroxy-1-methylethyl)-1,3-thiazol-2-yl]acrylamide,
(2E)-2-[4-(cyclobutylsulfonyl)phenyl]-3-cyclopentyl-N-[4-(1,2-dihydroxyethyl)-1,3-thiazol-2-yl]acrylamide,
(2E)-3-cyclopentyl-2-[4-(cyclopropylsulfonyl)phenyl]-N-{4-[3-hydroxy-2-(hydroxymethyl)propyl]-1,3-thiazol-2-yl}acrylamide,
(2E)-3-cyclopentyl-2-[4-(cyclopropylsulfonyl)phenyl]-N-[4-(1,2-dihydroxyethyl)-5-methyl-1,3-thiazol-2-yl]acrylamide,
(2E)-2-[4-(cyclobutylsulfonyl)phenyl]-3-cyclopentyl-N-[4-(1,2-dihydroxy-1-methylethyl)-1,3-thiazol-2-yl]acrylamide,
(2R)-3-cyclopentyl-2-[4-(cyclopropylsulfonyl)phenyl]-N-[4-(1,2-dihydroxy-1-methylethyl)-1,3-thiazol-2-yl]propanamide,
(2E)-3-cyclopentyl-2-[4-(cyclopropylsulfonyl)phenyl]-N-{4-[(1S)-1,2-dihydroxyethyl]-1,3-thiazol-2-yl}acrylamide, and
(2E)-3-cyclopentyl-2-[4-(cyclopropylsulfonyl)phenyl]-N-(4-glycoloyl-1,3-thiazol-2-yl)acrylamide.

9. A pharmaceutical composition which comprises the compound or a pharmaceutically acceptable salt thereof described in claim 1 and a pharmaceutically acceptable carrier.

10. The pharmaceutical composition described in claim 9, which is a GK activator.

11. The pharmaceutical composition described in claim 9, which is an agent for preventing and/or treating diabetes.

12. The pharmaceutical composition described in claim 11, which is an agent for preventing and/or treating type II diabetes.

13. The pharmaceutical composition described in claim 9, which is an agent for preventing and/or treating obesity.

14. The pharmaceutical composition described in claim 9, which is an agent for preventing and/or treating metabolic syndrome.

15. Use of the compound or a pharmaceutically acceptable salt thereof described in claim 1, for the manufacture of a GK activator or an agent for preventing and/or treating diabetes, obesity or metabolic syndrome.

16. A method for preventing and/or treating diabetes, obesity or metabolic syndrome, which comprises administering to a patient a therapeutically effective amount of the compound or a salt thereof described in claim 1.

**INTERNATIONAL SEARCH REPORT**

International application No.
PCT/JP2006/317102

A. CLASSIFICATION OF SUBJECT MATTER
*C07D277/20*(2006.01)i, *A61K31/426*(2006.01)i, *A61K31/4709*(2006.01)i, *A61K31/517*(2006.01)i, *A61P3/10*(2006.01)i, *A61P43/00*(2006.01)i, *C07D277/44*(2006.01)i, *C07D277/56*(2006.01)i, *C07D417/04*(2006.01)i,
According to International Patent Classification (IPC) or to both national classification and IPC

B. FIELDS SEARCHED
Minimum documentation searched (classification system followed by classification symbols)
C07D277/20, A61K31/426, A61K31/4709, A61K31/517, A61P3/10, A61P43/00, C07D277/44, C07D277/56, C07D417/04, C07D417/12

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)
CAplus(STN), REGISTRY(STN), WPI(DIALOG)

C. DOCUMENTS CONSIDERED TO BE RELEVANT

| Category* | Citation of document, with indication, where appropriate, of the relevant passages | Relevant to claim No. |
|---|---|---|
| Y | WO 2004/052869 A1 (F. HOFFMANN-LA ROCHE AG), 24 June, 2004 (24.06.04), Claims 1, 75, 76; Par. Nos. [0155] to [0160]; examples 54, 81<br>& US 2004/0147748 A1 & AU 2003292229 A1<br>& NO 200502555 A & EP 1572670 A1<br>& BR 200317268 A & TW 200426139 A<br>& MX 2005006250 A1 & ZA 200504474 A<br>& JP 2006-510650 A & CN 1726197 A | 1-15 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 07 November, 2006 (07.11.06) | 14 November, 2006 (14.11.06) |
| Name and mailing address of the ISA/<br>Japanese Patent Office<br>Facsimile No. | Authorized officer<br><br>Telephone No. |

Form PCT/ISA/210 (second sheet) (April 2005)

**INTERNATIONAL SEARCH REPORT**

International application No. PCT/JP2006/317102

C (Continuation). DOCUMENTS CONSIDERED TO BE RELEVANT

| Category* | Citation of document, with indication, where appropriate, of the relevant passages | Relevant to claim No. |
|---|---|---|
| Y | WO 2003/095438 A1 (F. HOFFMANN-LA ROCHE AG), 20 November, 2003 (20.11.03), Claim 8; Par. No. [0012]; examples & US 2003/0225283 A1 & AU 2003232204 A1 & EP 1501815 A1 & BR 200309546 A & NO 200404534 A & KR 2004104633 A & MX 2004010605 A1 & TW 200402418 A & CN 1649859 A & JP 2005-535589 A & US 2006/0178429 A1 & US 7105671 B2 | 1-15 |
| Y | US 2004/0186290 A1 (MATTHEW C. T. F.), 23 September, 2004 (23.09.04), Claim 11; Par. Nos. [0083] to [0091]; table 6 & WO 2004/072066 A1 & EP 1594863 A1 | 1-15 |
| A | JP 2004-521095 A (F. HOFFMANN-LA ROCHE AG), 15 July, 2004 (15.07.04), & WO 2002/048106 A2 & US 2002/0082260 A1 & AU 200238415 A & US 6482951 B2 & NO 200302674 A & EP 1349856 A2 & KR 2003064817 A & CZ 200301882 A3 & BR 200116169 A & SK 200300873 A3 & CN 1481382 A & HU 200400587 A2 & MX 2003005170 A1 & NZ 526236 A & RU 2249590 C2 & EP 1349856 B1 & DE 60111570 E & IN 200300924 P4 & ZA 200304265 A & ES 2243578 T3 & DE 60111570 T2 & MX 232748 B | 1-15 |
| A | WO 2004/063194 A1 (ELI LILLY AND CO.), 29 July, 2004 (29.07.04), & AU 2003294376 A1 | 1-15 |

Form PCT/ISA/210 (continuation of second sheet) (April 2005)

**INTERNATIONAL SEARCH REPORT**

International application No.
PCT/JP2006/317102

Continuation of A. CLASSIFICATION OF SUBJECT MATTER
(International Patent Classification (IPC))

*C07D417/12*(2006.01)i

(According to International Patent Classification (IPC) or to both national classification and IPC)

Form PCT/ISA/210 (extra sheet) (April 2005)

**INTERNATIONAL SEARCH REPORT**

International application No.
PCT/JP2006/317102

**Box No. II Observations where certain claims were found unsearchable (Continuation of item 2 of first sheet)**

This international search report has not been established in respect of certain claims under Article 17(2)(a) for the following reasons:

1. [X] Claims Nos.: 16
because they relate to subject matter not required to be searched by this Authority, namely:
The invention as set forth in claim 16 pertains to methods for treatment of the human body by therapy and thus relates to a subject matter which this International Searching Authority is not required to search (Article 17(2)(a)(i) of the PCT, Rule 39.1(iv) of the Regulations under the PCT).

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.
2. [ ] As all searchable claims could be searched without effort justifying an additional fee, this Authority did not invite payment of any additional fee.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** the
[ ] The additional search fees were accompanied by the applicant's protest and, where applicable, payment of a protest fee..
[ ] The additional search fees were accompanied by the applicant's protest but the applicable protest fee was not paid within the time limit specified in the invitation.
[ ] No protest accompanied the payment of additional search fees.

Form PCT/ISA/210 (continuation of first sheet (2)) (April 2005)

**INTERNATIONAL SEARCH REPORT**

International application No.
PCT/JP2006/317102

**Box No. I Nucleotide and/or amino acid sequence(s) (Continuation of item1.b of the first sheet)**

1. With regard to any nucleotide and/or amino acid sequence disclosed in the international application and necessary to the claimed invention, the international search was carried out on the basis of:

a. type of material
- [x] a sequence listing
- [ ] table(s) related to the sequence listing

b. format of material
- [ ] in written format
- [x] in computer readable form

c. time of filing/furnishing
- [x] contained in the international application as filed
- [ ] filed together with the international application in computer readable form
- [ ] furnished subsequently to this Authority for the purposes of search

2. [ ] In addition, in the case that more than one version or copy of a sequence listing and/or table relating thereto has been filed or furnished, the required statements that the information in the subsequent or additional copies is identical to that in the application as filed or does not go beyond the application as filed, as appropriate, were furnished.

3. Additional comments:

Although this international application includes a nucleotide sequence, the international search was carried out without retrieval for the sequence itself.

Form PCT/ISA/210 (continuation of first sheet (1)) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0058293 A
- WO 0183465 A
- WO 0185706 A
- WO 0185707 A
- WO 0208209 A
- WO 0214312 A
- WO 0395438 A
- WO 200472066 A
- WO 200450645 A
- WO 200658923 A


### Non-patent literature cited in the description


- *Diabetes,* 2005, vol. 54 (2), 2602-2611
- *Diabetes,* 2004, vol. 53, 2521-2528
- *Prog. Med.,* 1985, vol. 5, 2157-2161
- **HIROKAWA SHOTEN.** Iyakuhin no Kaihatsu. *Bunshi Sekkei,* 1990, vol. 7, 163-198
- **GREENE ; WUTS.** Protective Groups in Organic Synthesis. 1999
- *Science,* 2003, vol. 301, 370-373

SEQUENCE LISTING

<110> Astellas Pharma Inc.

<120> Thiazole derivative

<130> T64054

<140> EP 06 797 075.6
<141> 2006-08-30

<150> JP 2005-252464
<151> 2005-08-31

<150> JP 2006-177436
<151> 2006-06-28

<160> 2

<170> PatentIn version 3.3

<210> 1
<211> 28
<212> DNA
<213> Artificial

<220>
<223> Description of artificial sequence:primer

<400> 1
tagaattcat ggcgatggat gtcacaag 28

<210> 2
<211> 27
<212> DNA
<213> Artificial

<220>
<223> Description of artificial sequence:primer

<400> 2
atctcgagtc actggcccag catacag 27